# EUROPÄISCHE PATENTSCHRIFT

(11) **EP 2 253 302 B1**
(45) Veröffentlichungstag und Bekanntmachung des Hinweises auf die Patenterteilung: **18.11.2020**
(21) Anmeldenummer: 10162296.7
(22) Anmeldetag: 07.05.2010
(51) Int. Cl.: C08L 83/04

(54) **Härtbare Zusammensetzungen, daraus hergestellte gehärtete Produkte und deren Verwendung**
Hardening compounds, products derived from same and use thereof
Compositions durcissables, produits durcis fabriqués à partir de celles-ci, et leur utilisation

(30) Priorität: 09.05.2009 DE 102009021553
(43) Veröffentlichungstag der Anmeldung: 24.11.2010
(73) Patentinhaber: Kettenbach GmbH & CO. KG, 35713 Eschenburg (DE)
(72) Erfinder: Bublewitz, Dr. Alexander, 35745, Herborn (DE); Reber, Dr. Jens-Peter, 58540, Meinerzhagen (DE)
(74) Vertreter: Keil & Schaafhausen Patentanwälte PartGmbB

(56) Entgegenhaltungen:
- WO-A2-2007/080071
- DE-A1-102006 001 126

## Beschreibung

Beschrieben werden neue härtbare Zusammensetzungen, die sich besonders zum Einsatz als Dentalabformmassen eignen und die sich durch ein sehr gutes Benetzungsverhalten sowie durch eine sehr gute biologische Abbaubarkeit auszeichnen.

Dentalabformmassen sind an sich bekannt und werden bereits seit langem eingesetzt. Diese Massen müssen eine Vielzahl von Eigenschaften aufweisen, wie rasches Abbindeverhalten, ausgezeichnete Detailgenauigkeit und gleichzeitig gute Lagerfähigkeit der Vorprodukte. Üblicherweise werden Zweikomponenten-Mischungen eingesetzt, die unmittelbar vor dem Gebrauch miteinander vermischt werden und die sodann rasch verarbeitet werden müssen. Für das Erzielen einer möglichst guten Detailgenauigkeit ist es wichtig, dass die zwischen Zahn bzw. Zahnfleisch einerseits und dem sich ausbildenden Abdruck andererseits möglichst wenig Flüssigkeit, wie Speichel oder Blut, befindet und dass diese Flüssigkeit möglichst wenig in den sich ausbildenden Formkörper aufgenommen wird.

Dentalabformmassen enthalten üblicherweise hydrophobe Materialien wie Polyorganosiloxane. Zum Minimieren der Flüssigkeitsschicht zwischen Abdruck und Zahn bzw. Zahnfleisch hat man bereits den Einsatz von Tensiden in Dentalabdruckmassen vorgeschlagen. Diese bewirken ein Verteilen des Wasserfilms und daher wird die Ausbildung eines Flüssigkeitsfilms erschwert.

Aus der US-A-4,657,959 ist eine aushärtbare und als dentales Abformmaterial einsetzbare Siliconzusammensetzung bekannt, welche ein aushärtbares Siliconprepolymer und ein Tensid enthält. Bei dem einzusetzenden Tensid handelt es sich vorzugsweise um ein ethoxyliertes nichtionisches oberflächenaktives Agens, enthaltend eine oder mehrere Siloxan- oder Perfluoralkylgruppen. Das Tensid wird in einer Menge eingesetzt, dass die Zusammensetzung 3 Minuten nach Aufgabe des Wassertropfens auf die ausgehärtete Zusammensetzung besonders bevorzugt einen Kontaktwinkel < 10° aufweist. Diese Kontaktwinkeleigenschaften an einem bereits ausgehärteten Abformmaterial nach 3 Minuten entsprechen aber nicht den Praxisanforderungen. Weiterhin sind in den Beispielen dieser US-A-4,657,959 amphotere oder ionische Fluortenside aufgeführt und im Falle der nichtionischen Fluortenside sind die Perfluoralkylgruppen über eine SO₂NR-Gruppe mit den Polyethergruppen verknüpft. Diese SO₂NR-Gruppen sind für einen Einsatz in einem Platin-katalysierten additionsvernetzenden Silicon ungeeignet, da sie starke Metall-Chelat-Komplexe am Platinkatalysator ausbilden und diesen auf diese Weise inhibieren. Darüber hinaus ist in der US-A-4,657,959 weder der Zusatz eines nicht reaktiven Polyetherpolymers noch eines reaktiv einpolymerisierbaren Polyetherpolymers, z.B. mit Vinyl-, Allyl- oder SiH-Gruppen, offenbart.

In der EP-A-1 290 998 wird eine additionsvernetzende Silicon-Abformmassen-Zusammensetzung beschrieben, die ein Organopolysiloxan mit wenigstens 2 aliphatisch gesättigten Kohlenwasserstoffgruppen, einem Polyether mit wenigstens einer Alkylgruppe, ein Organohydrogensiloxan, einen löslichen Platinkatalysator, einen anorganischen Füllstoff sowie ein nichtionisches Tensid und/oder ein Polyether-modifiziertes Siliconöl enthält.

Die EP-A-0 847 745 beschreibt Dentalsiliconabdruckmaterialien, die neben Siliconpolymeren mit SiH-Gruppen und Siliconpolymeren mit Si-Alkenylgruppen, Katalysator und anorganischen Füllstoffen ein Polysiloxan-Polyester-Polymer mit mindestens einem auf Dimethicon basierenden Polyalkylenoxidsubstituenten und mit einem fluorierten Alkylsubstituenten enthalten. Gemäß dieser Offenbarung wird mit einem ausgehärteten, festen Probekörper ein Kontaktwinkel von weniger als 73° über die Wilhelmi-Methode bestimmt. Bei dem eingesetzten fluorhaltigen Polysiloxan-Polyester handelt es sich um ein hochmolekulares Polymer, an dessen Kettenenden jeweils fluorierte Kohlenwasserstoffe sitzen. Die damit erzielbaren Kontaktwinkel sind eher gering. Der fluorierte Polysiloxan-Polyester stellt aufgrund der polymeren Struktur, der hohen Molmasse der Polymere und der an beiden Kettenenden sitzenden Fluorkohlenwasserstoffgruppen kein klassisches niedermolekulares Tensid mit einem hydrophilen Kopf und einem hydrophoben Schwanz dar.

Die WO-A-2004/058196 beschreibt dentale Abdruckmaterialien umfassend ein Polyvinylsiloxan und ein Tensid, wobei das Tensid der Zusammensetzung eine derartige Benetzbarkeit verleiht, dass das Material 15 Minuten nach dem Aushärten nach ungefähr 15 Sekunden einen Oberflächenkontaktwinkel mit Wasser von weniger als ungefähr 10° aufweist. Insbesondere werden diese Kontaktwinkel durch Einsatz des Tensides PEG-8-Methicone erreicht.

Die EP-A-1 165 016 beschreibt ein additionsvernetzendes Abformmaterial auf Siliconbasis mit einem Polyalkylenoxid und/oder dessen Derivate mit einer Molmasse > 3000 g/mol mit einer Konzentration zwischen 0,001 und 1,0 Gew.-%. Der eingesetzte Polyether soll die Standfestigkeit des Abformmaterials verbessern. Gleichzeitig wurden die Kontaktwinkel mit > 80° bestimmt. Aus dieser Druckschrift ist zu entnehmen, dass nur durch einen Polyetherzusatz keine guten Kontaktwinkel und damit keine guten Hydrophilieeigenschaften zu erzielen sind.

EP-A-0 729 341 offenbart zur Hydrophilierung von Zahnabdruckmassen den Einsatz von Polyethercarbosilanen. Die Kontaktwinkel-/Randwinkelmessung erfolgt 30 Minuten nach Aushärtung des Abformmaterials und die Kontaktwinkel liegen bei minimal 42°.

Die EP-A-0 613 926 beschreibt Polyetherabformmaterialien, die wenigstens ein hydrophiles Mittel aus der Gruppe bestehend aus hydrophilen Silikonölen, fluorierten Kohlenwasserstoffen, Blockcopolymeren von Ethylenoxid/Propylenoxid, Fettalkoholderivaten, Alkylphenolderivaten, Fettaminen, Aminoxiden, Fettsäureglykol- und -glycerinderivaten, Fettsäuren und Fettsäuremonoestern enthalten. Die Kontaktwinkelmessung erfolgt 30 Minuten nach Aushärtung des Abformmaterials und die Kontaktwinkel liegen zwischen 18° und 65°.

Aus der WO-A-00/48553 ist eine additionsvernetzende Silicon-Abformmaterial-Zusammensetzung bekannt, die einen einpolymerisierbaren Siliconpolyether mit kammartigen Polyethergruppen enthält. In den Beispielen wurden Kombinationen dieses einpolymerisierbaren Siliconpolyethers mit Nonylphenylethoxylat-Tensiden beschrieben.

EP-A-0 231 420 offenbart ein additionsvernetzendes Silicon-Abformmaterial mit einem Siliconpolyether.

DE-A-40 10 281 offenbart additionsvernetzende Polyether-Abformzusammensetzungen, bei denen der Polyether endständige Vinyldimethylsiloxygruppen oder Allylgruppen enthält. Die über eine SiOC-Bindung an dem Polyether gebundenen ungesättigten Gruppen sind hydrolyseanfällig und nicht lagerstabil. Im anderen Fall sind die Polyether durch Pt-katalysierte Hydrosilylierung mit Vinyldimethylsiloxan-Gruppen verknüpft. Der hochaktive Katalysator kann nicht durch gebräuchliche ständige Reinigungsmethoden abgetrennt werden. Selbst eine Reinigung des Polymers über eine Hochvakuumdestillation ist aufgrund der hohen Molmassen des Polymers und Pt-Katalysators nicht möglich. Die auf diese Weise synthetisierten und mit Restplatin verunreinigten Polyetherpolymere sind nicht lagerstabil und somit für eine Dentalabformmassenzusammensetzung ungeeignet. Aus diesem Grund erfolgte bis zum heutigen Tag keine Kommerzialisierung dieser Produkte.

US-A-5,064,891 beschreibt eine additionsvernetzende Siliconzusammensetzung mit einem Silicontensid. Die Kontaktwinkelmessungen wurden an ausgehärteten Probekörpern und 3 Minuten nach Aufbringen der Wassertropfen durchgeführt. Die beschriebenen Kontaktwinkel liegen zwischen 60 und 65°.

EP-A-0 885 932 beschreibt additionsvernetzende Organopolysiloxan-Zusammensetzungen mit einem hydrophilen ungesättigten Polysiloxan-Polyether-Copolymer mit 2 bis 5 Siliziumatomen und mindestens einer aliphatischen ungesättigten Funktionalität und mindestens einer Polyether-Funktionalität.

Aus der US-A-5,907,002 ist ein additionsvernetzendes Abformsiliconmaterial beschrieben, das in der Kombination eines nichtionischen Tensids mit einem Methylphenylpolysiloxan formuliert wird. Das nichtionische Tensid kann neben der hydrophilen Gruppe eine lipophile Gruppe haben, wobei diese eine Alkylgruppe oder eine Fluorcarbon-Gruppe sein kann. Der Einsatz von Mischungen aus Silicontensid mit Fluortensid wird nicht offenbart. Die erreichten Kontaktwinkel liegen zwischen 28° und 60°.

Kombinationen von Fluortensiden und Silicontensiden sind aus der DE 699 17 384 T2 (entsprechend EP-B-0 974 626) bekannt. Dieses Dokument beschreibt wässrige pigmentierte Tintenstrahldrucktinten enthaltend solche Tensidgemische.

Aus der DE-A-199 22 929 schließlich sind härtbare dentale Abformmaterialien enthaltend Tenside oder Kombinationen von Tensiden bekannt. Diese Abformmaterialien sind für die Abdrucknahme der Mundschleimhaut bestimmt und fließen bei sehr geringem Druck; sie fließen jedoch nicht, wenn kein Druck angewendet wird. Das Abdruckmaterial soll auf die Mundschleimhaut nur eine sehr geringe Reizung ausüben. Der Einsatz von Gemischen aus Silicontensiden und Fluortensiden wird nicht offenbart.

US-A-6,861,457 beschreibt hydrophile dentale Abformmaterialien auf der Basis additionsvernetzender Polysiloxane. Diese enthalten neben einem Polyether mit ungesättigten Gruppen ein nichtionisches Tensid und/oder ein Polyether-modifiziertes Siliconöl. Kombinationen von Fluortensiden mit Silicontensiden werden nicht beschrieben.

In der DE-A-43 06 997 werden hydrophilierte Polyether offenbart. Diese können unterschiedlichste Tenside enthalten; als mögliche Substanzklassen werden neben anderen Tensiden hydrophile Silikonöle oder fluorierte Kohlenwasserstoffverbindungen offenbart. In den Beispielen werden härtbare Zusammensetzungen enthaltend mit Aziridinogruppen verkappte Polyether und ein nichtionisches Fluortensid (Fluorad FC430) beschrieben. Diese Verbindung enthält gemäß dem 3M Material Safety Data Sheet, Ausgabe 30.08.2002, Perfluoroctylsulfonatgruppen. Härtbare Zusammensetzungen enthaltend Gemische aus Silicontensiden und Fluortenside mit (Poly)alkylenoxidresten, Kohlenhydratresten oder aliphatischen Polyhydroxyresten werden in DE-A-43 06 997 nicht offenbart.

EP-B-0 244 478 beschreibt die Verwendung von hydrophilen Siliconen als Zahnabdruckmaterialien. In diesem Dokument wird unter anderem der Einsatz von Netzmitteln aus ethoxylierten nicht ionischen grenzflächenaktiven Stoffen mit solubilisierenden Siloxan- oder Perfluoralkylresten offenbart. Härtbare Zusammensetzungen enthaltend Gemische aus Silicontensiden und Fluortenside mit (Poly)alkylenoxidresten, Kohlenhydratresten oder aliphatischen Polyhydroxyresten werden auch in diesem Dokument nicht beschrieben.

WO-A-2005/016289 beschreibt Dentalabformmassen auf der Basis von Polyorganosiloxanen. Diese zeichnen sich durch die Anwesenheit eines Netzmittels aus, welches die Benetzbarkeit der Masse durch Wasser verbessert, so dass sich nach 3 Minuten ein Kontaktwinkel von weniger als 50° einstellt. Als Netzmittel werden beispielsweise ethoxylierte Nonylphenole oder PEG-8-Methicone vorgeschlagen.

In den genannten Patentdokumenten werden die Kontaktwinkelmessungen nicht auf die Praxisanforderungen bezogen ausgeführt, d. h. die Kontaktwinkel werden an ausgehärteten Probekörpern gemessen. Die Praxisanforderung aber sieht so aus, dass zum einen nicht die Kontaktwinkeleigenschaften des ausgehärteten Abformmaterials relevant, sondern ganz im Gegenteil die Kontaktwinkeleigenschaften im nicht ausgehärteten plastischen Zustand entscheidend sind und zum anderen die Kontaktwinkeleigenschaften nicht erst nach einer Zeitspanne von 3 Minuten relevant sind, sondern sofort bei der Abdrucknahme, d.h. zwischen > 0 Sekunden und < 10 Sekunden nach dem initialen Kontakt zwischen dem plastischen Abformmaterial und der Zahnsubstanz bzw. der Mundschleimhaut. Dies erklärt sich dadurch, dass das Abformmaterial bei der Zahnabdrucknahme während der Verarbeitungszeit, also im noch nicht ausgehärteten Zustand, in der plastischen Phase initial, d.h. zwischen > 0 Sekunden und < 10 Sekunden, die feuchte, mit Speichel benetzte Zahnsubstanz und Mundschleimhaut benetzen und unmittelbar anfließen muss. Alles was sich zeitlich danach an Kontaktwinkeleigenschaften einstellt, ist für eine detailgetreue Abdrucknahme somit nicht mehr relevant.

In der unter Praxisbedingungen für das Anfließen des Abformmaterials an den feuchten Zahn wichtigen Phase nach Mischen der beiden Komponenten, in der sogenannten Verarbeitungszeit, in der das Abdruckmaterial noch plastisch verformbar ist und mit feuchten Zähnen, Speichel und Blut in Kontakt kommt, weisen die im vorstehend beschriebenen Stand der Technik offenbarten Materialien keine guten Kontaktwinkel von ≤ 10° auf. Ein Spreiten des Wassertropfens auf der Materialoberfläche ist nicht zu beobachten. Die in diesen Dokumenten beschriebenen Kontaktwinkel sind lediglich beim Ausgießen der ausgehärteten Abformung außerhalb des Mundes mit flüssigem Gipsbrei zur Modellerstellung relevant, jedoch nicht bei der entscheidenden Abdrucknahme im Mund. Ferner beschreibt keines dieser Dokumente den Einsatz einer synergistisch wirkenden Tensidmischung oder einer Kombination dieser Mischung mit einem Polyether.

Eine sehr aktuelle und wissenschaftliche Untersuchung in Deutsche Zahnärztliche Zeitschrift 60 (2005) 10, S. 587-592 von Rupp et al. beschreibt die Anforderungen, die an ein Dentalabformmaterial hinsichtlich Benetzungsverhalten gestellt werden. Hier wurden Untersuchungen zur initialen Hydrophilie und zur Gleichgewichts-Hydrophilie durchgeführt. Als Praxisanforderung ergibt sich daraus, dass zu jedem Zeitpunkt während der plastischen Phase der Verarbeitungszeit das Abformmaterial eine niedrige initiale Hydrophilie und zusätzlich eine zu jedem Zeitpunkt gleich niedrige Gleichgewichts-Hydrophilie aufweisen soll.

Das Applizieren und Anfließen des Abformmaterials im Patientenmund erfolgt unter Praxisbedingungen zu verschiedenen Zeitpunkten, abhängig von der Abformtechnik und von der Anzahl der abzuformenden Zähne. So ist z.B. bei einer Einzelkronenabformung zum Applizieren und Anfließen eine relativ kurze Zeit, z.B. 40 Sekunden, erforderlich, während bei umfangreicheren Inlets mit 4 oder 5 abzuformenden Zähnen das Applizieren und Anfließen erst nach 2 Minuten erfolgt ist.

Nach der Untersuchung von Rupp et al. weisen die heute verwendeten Abformmaterialien, vor allem die Siliconabfommaterialien, eine starke Drift in hydrophober Richtung bei zunehmender Verarbeitungszeit auf, was gegen die o.g. Praxisbedürfnisse und Praxisanforderungen der Abformmaterials läuft.

Weiterhin sind die dort gemessenen initialen Hydrophilie-Kontaktwinkel sowohlbei den Polyether- bzw. Silicon-Abformmaterialien ebenfalls verbesserungswürdig.

EP 2 072 029 B1 und EP 2 231 102 B1 offenbaren eine Dentalmaterialzusammensetzung mit einem härtbaren Polysiloxan (A), einem Vernetzer (B), einem Katalysator (C), einem Tensid (D) und einem Fluortensid (E).

Dentalabformmassen mit deutlich verbessertem Benetzungsverhalten sind aus der WO 2007/080071 A2 bekannt. Dieses Dokument offenbart verschiedene vernetzbare Systeme enthaltend eine synergistisch wirkende Kombination ausgewählter Silicontenside und ausgewählter Fluortenside.

Ausgehend von diesem Stand der Technik war es Aufgabe der vorliegenden Erfindung, eine härtbare Zusammensetzung bereitzustellen, die sich vorzugsweise als Dentalabdruckmasse eignet, die einerseits zu jedem Zeitpunkt während der Verarbeitungszeit (sowohl am Anfang als auch am Ende) einen niedrigen initialen Kontaktwinkel und einen konstanten und niedrigen Gleichgewichts-Kontaktwinkel (Hydrophile) liefert, so dass unter Praxisbedingungen ein außerordentlich gutes Anfließen an den feuchten Zahn bzw. Zahngewebe erfolgt und dies wiederum zur Ausbildung eines äußerst detailgetreuen Abdrucks führt und die andererseits gut biologisch abbaubar ist.

Es wurde in überraschender Weise gefunden, dass der Einsatz ausgewählter Fluortenside in Kombination mit einem Silizium enthaltende Gruppen aufweisenden nichtionischen Tensid in ausgewählten vernetzbaren Systemen diese Aufgabe löst.

Die vorliegende Erfindung betrifft eine Zusammensetzung enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzenden Esterreste einer ethylenisch ungesättigten Carbonsäure enthaltenden Polyether oder der durch ringöffnende Metathesereaktion vernetzenden Polyether, Silicone oder Kautschuke, und enthaltend ferner mindestens ein nichtionisches Fluortensid ausgewählt aus der Gruppe
a) der Fluortenside, die mindestens eine teil- oder perforierte Alkylenoxid- oder Polyalkylenoxideinheit aufweisen,
   sowie enthaltend ferner mindestens ein Silizium enthaltende Gruppen aufweisendes nichtionisches Tensid mit einer Molmasse von weniger als 6.000 g/mol.
   Bevorzugte Zusammensetzungen können ferner mindestens ein ionisches Fluortensid enthalten, welches ausgewählt ist aus der Gruppe
b) der Fluortenside, die neben einer ionischen Gruppe einen teil- oder perfluorierten Alkylrest mit weniger als 5 Kohlenstoffatomen aufweisen,
c) der Fluortenside, die neben einer ionischen Gruppe einen teil- oder perfluorierten Alkoxyalkylenrest mit weniger als 5 Kohlenstoffatomen aufweisen,
d) der Fluortenside, die neben einer ionischen Gruppe einen teilfluorierten Alkylrest mit mindestens 5 Kohlenstoffatomen aufweisen, wobei als fluorierte Reste nur ω-Mοnο-, Di- oder Trifluormethylreste oder ω-Mοnο-, Di-, Tri-, Tetra- oder Pentafluorethylreste auftreten,
e) der Fluortenside, die neben einer ionischen Gruppe einen teilfluorierten Alkoxyalkylenrest mit mindestens 5 Kohlenstoffatomen aufweisen, wobei als fluorierte Reste nur ω-Mοnο-, Di- oder Trifluormethoxyreste oder ω-Mono-, Di-, Tri-, Tetra- oder Pentafluorethoxyreste auftreten,
f) der Fluortenside, die neben einer ionischen Gruppe einen teilfluorierten ω-(N,N-Di-per- oder teilfluormethyl- oder -ethylamino)-alkylenrest mit mindestens 7 Kohlenstoffatomen oder einen teilfluorierten ω-(N,N-Di-per- oder teilfluormethyl- oder -ethylamino)-alkylanoxyalkylenrest mit mindestens 7 Kohlenstoffatomen aufweisen, wobei als fluorierte Reste nur ω-(N,N-Di-per- oder teilfluormethyl- oder -ethylamino)-reste auftreten,
g) der Fluortenside, die neben einer ionischen Gruppe einen teilfluorierten ω-(Mono-oder Di-per- oder -teilfluormethyl- oder -ethylphenoxy)-alkylenrest mit mindestens 10 Kohlenstoffatomen aufweisen, wobei als fluorierte Reste nur ω-(Mοnο- oder Di-per- oder -teilfluormethyl- oder -ethylphenoxy)-reste auftreten, und der Fluortenside, die neben einer Polyalkylenglykolhauptkette mehrere teil- oder perfluorierte Alkoxyreste mit weniger als 5 Kohlenstoffatomen als Seitenketten aufweisen.

Bevorzugte Zusammensetzungen weisen 40 Sekunden nach Mischbeginn, vorzugsweise zu jedem Zeitpunkt während der Verarbeitungszeit zwischen > 0 und 3 Minuten, einen niedrigen initialen Wassertropfen-Kontaktwinkel von < 10° auf (gemessen bei 50 % Luftfeuchtigkeit in der Klimakammer) und bei einem Tropfenalter von 10 Sekunden.

Der dynamische Verlauf des Wassertropfen-Kontaktwinkels bevorzugter Zusammensetzung, gemessen 40 Sekunden nach Mischbeginn, nimmt vorzugsweise folgende Werte an: nach einem Tropfenalter von 0,25 Sekunden einen Wassertropfen-Kontaktwinkel von < 75°, vorzugsweise von < 40°; nach einem Tropfenalter von 0,5 Sekunden einen Wassertropfen-Kontaktwinkel von < 55°, vorzugsweise < 30°; nach einem Tropfenalter von 1 Sekunde einen Wassertropfen-Kontaktwinkel von < 35°, vorzugsweise < 25°; nach einem Tropfenalter von 2 Sekunden einen Wassertropfen-Kontaktwinkel von < 20°; und nach einem Tropfenalter von 3 Sekunden einen Wassertropfen-Kontaktwinkel von < 10°.

Auf der noch nicht ausgehärteten erfindungsgemäßen Zusammensetzung spreitet ein Wassertropfen und bildet nach kurzer Zeit, typischerweise nach spätestens 3 Sekunden, einen Tropfen mit einem sehr geringen Kontaktwinkel von <10° aus oder er verläuft vollständig unter Ausbildung eines Wasserfilms.

Die erfindungsgemäßen Zusammensetzungen sind durch die Anwesenheit ausgewählter nichtionischer Fluortenside gekennzeichnet, die in Kombination mit Silizium enthaltende Gruppen aufweisenden nichtionischen Tensiden verwendet werden.

Diese Kombinationen zeichnen sich durch ein besonders gutes Benetzungsverhalten aus.

Die erfindungsgemäß eingesetzten Fluortenside gehören der oben definierten Gruppe a) an.

Fluortenside der Gruppe a) enthalten mindestens eine teil- oder perfluorierte Alkylenoxideinheit oder eine teil- oder perfluorierte Polyalkylenoxideinheit (nachstehend teil- oder perfluorierte (Poly)alkylenoxideinheit genannt).

Der oder die teil- oder perfluorierten (Poly)alkylenoxideinheiten sind vorzugsweise über eine Brückengruppe mit einer hydrophilen (Poly)alkylenoxideinheit verbunden.

Die Fluortenside sind in Abhängigkeit vom vernetzbaren System zu wählen. In den durch Additionsreaktion vernetzenden Organopolysiloxanen oder den durch Additionsreaktion vernetzenden Alkenylreste enthaltenden Polyethern greifen Fluortenside mit Schwefel-, Stickstoff- und/oder Phosphoratomen in ungünstiger Weise in die Härtungsreaktion ein, so dass bei diesen Systemen Fluortenside enthaltend Schwefel-, Stickstoff- und/oder Phosphoratome vorzugsweise ausgeschlossen sind.

Bei den übrigen härtbaren Systemen lassen sich zusätzlich auch Fluortenside mit Stickstoff-, Schwefel- und/oder Phosphoratomen bzw. Gruppen enthaltend diese Atome einsetzen. Beispiele dafür sind Aminogruppen, Sulfonsäure-, Phosphorsäure- oder Phosphonsäureestergruppen; oder Carbonsäure-, Sulfonsäure-, Phosphorsäure- oder Phosphonsäureamidgruppen. Es können auch Fluortenside eingesetzt werden, welche mehrere dieser Gruppen bzw. Atome aufweisen, beispielsweise Amino- und Carbonsäureamidgruppen.

Bevorzugt werden nichtionische Fluortenside der Gruppe a) eingesetzt, die mindestens eine (Poly)alkylenoxideinheit und mindestens eine teil- oder perfluorierte (Poly)alkylenoxideinheit aufweisen, die über ein Sauerstoffatom oder eine Carbonsäureestergruppe miteinander verbunden sind.

Unter teil- oder perfluorierter (Poly)alkylenoxideinheit sind im Rahmen dieser Beschreibung Reste zu verstehen, die mindestens eine teil- oder perfluorierte Alkylenoxideinheit, vorzugsweise mehrere teil- oder perfluorierte Alkylenoxideinheiten aufweisen, wobei die Teil- oder Perfluoralkylengruppen in einem Rest unterschiedliche Anzahlen von Kohlenstoffatomen aufweisen können. Diese unterschiedlichen Teil- oder Perfluoralkylengruppen können im Rest statistisch oder in Form von Blöcken wiederkehrender Struktureinheiten auftreten.

Unter (Poly)alkylenoxideinheit sind im Rahmen dieser Beschreibung Reste zu verstehen, die mindestens eine Alkylenoxideinheit aufweisen, vorzugsweise mehrere Alkylenoxideinheiten, wobei die Alkylengruppen in einem Rest unterschiedliche Anzahlen von Kohlenstoffatomen aufweisen können. Diese unterschiedlichen Alkylengruppen können im Rest statistisch oder in Form von Blöcken wiederkehrender Struktureinheiten auftreten.

Erfindungsgemäß eingesetzte Fluortenside der Gruppe a) sind Blockcopolymere enthaltend Blöcke der Formel Ia

-[O-RF]ₐ-A'-[O-R_{H}]_{d}- (Ia),

worin
R_{F} ein teil- oder perfluorierter Alkylenrest mit 2 bis 12 Kohlenstoffatomen ist, wobei innerhalb eines Polyetherrestes die Anzahl der Kohlenstoffatome der teil- oder perfluorierten Alkylenreste im Rahmen der gegebenen Definitionen unterschiedlich sein kann,
R_{H} einen Alkylenrest mit 2 bis 12, vorzugsweise 2 bis 6 Kohlenstoffatomen bedeutet, wobei innerhalb eines Polyetherrestes die Anzahl der Kohlenstoffatome der Alkylenreste im Rahmen der gegebenen Definitionen unterschiedlich sein kann,
A' eine kovalente Bindung ist oder eine zweiwertige Brückengruppe bedeutet, die mit den Blöcken [O-R_{F}] und [O-R_{H}] über C-C- und/oder C-O-Bindungen verknüpft ist,
a eine ganze Zahl von 1 bis 200 ist, vorzugsweise von 1 bis 50, insbesondere 1 bis 20, und
d eine ganze Zahl von 1 bis 200 ist, vorzugsweise von 1 bis 50, insbesondere von 1 bis 10.

Zu den besonders bevorzugt eingesetzten Fluortensiden der Gruppe a) zählen die Verbindungen der Formel Ib

R¹-(O-R_{F})ₐ-A'-(O-R_{H})_{b}-A-(R'_{H}-O)_{c}-B (Ib),

worin R¹ Wasserstoff, ein teil- oder perfluorierter Alkylrest mit 1 bis 6 Kohlenstoffatomen oder ein Alkylrest mit 1 bis 6 Kohlenstoffatomen ist, vorzugsweise Wasserstoff, ein Alkylrest mit 1 bis 4 Kohlenstoffatomen oder ein teil- oder perfluorierter Alkylrest mit 1 bis 4 Kohlenstoffatomen,
R_{F} die oben definierte Bedeutung besitzt,
R_{H} und R'_{H} unabhängig voneinander Alkylenreste mit 2 bis 12, vorzugsweise 2 bis 6 Kohlenstoffatomen bedeuten, wobei innerhalb eines Polyetherrestes die Anzahl der Kohlenstoffatome der der Alkylenreste im Rahmen der gegebenen Definitionen unterschiedlich sein kann,
A und A' unabhängig voneinander eine kovalente Bindung oder eine zweiwertige Brückengruppe, die mit den Blöcken [O-R_{F}], [O-R_{H}] und [R'_{H}-O] über C-C- und/oder C-O-Bindungen verknüpft ist, bedeuten,
a eine ganze Zahl von 1 bis 200 ist, vorzugsweise von 2 bis 50,
b eine ganze Zahl von 0 bis 100 ist, vorzugsweise von 2 bis 50,
c eine ganze Zahl von 1 bis 100 ist, vorzugsweise von 2 bis 50, und
B Wasserstoff, Alkyl, Teil- oder Perfluoralkyl bedeutet.

Verbindungen der Formel Ib sind aus EP-B-0 864 643 bekannt.

Von den nichtionischen Fluortensiden der Formeln la und Ib werden solche besonders bevorzugt verwendet, bei denen
R_{F} ein Rest der Formel -Cₘ₁Fₙ₁Hₒ₁- ist oder ein Rest der Formel -CH₂-C(CH₃)R^{1a}-CH₂-, wobei innerhalb eines Polyetherrestes die Indizes m1, n1 und o1 im Rahmen der gegebenen Definitionen unterschiedlich sein können,
m1 eine ganze Zahl von 2 bis 4 bedeutet,
n1 eine ganze Zahl von 1 bis 8 bedeutet,
o1 eine ganze Zahl von 0 bis 7 bedeutet,
wobei die Summe von n1 und o1 dem Wert 2m1 entspricht,
R^{1a} ein teil- oder perfluorierter Alkylrest mit 1 bis 4 Kohlenstoffatomen ist,
R_{H} ein Rest der Formel -Cₚ₁H₂ₚ₁- ist, wobei innerhalb eines Polyetherrestes der Index p1 im Rahmen der gegebenen Definition unterschiedlich sein kann,
R'_{H} ein Rest der Formel -C_{q1}H_{2q1}- ist, wobei innerhalb eines Polyetherrestes der Index q1 im Rahmen der gegebenen Definition unterschiedlich sein kann,
p1 eine ganze Zahl von 2 bis 4 bedeutet,
q1 eine ganze Zahl von 2 bis 4, bedeutet,
A eine zweiwertige Brückengruppe ist, die ausgewählt wird aus der Gruppe Sauerstoffatom, Carbonsäureester oder -O-CH₂-CF₂-, mit der Maßgabe, dass die Brückengruppe mit den Blöcken [O-R_{H}] und [R'_{H}-O] eine C-O- und/oder eine C-C-Bindung ausbilden,
A' eine kovalente Bindung bedeutet,
a eine ganze Zahl von 2 bis 50 ist,
b eine ganze Zahl von 0 bis 25 ist,
c eine ganze Zahl von 1 bis 25 ist,
d eine ganze Zahl von 1 bis 25 ist, und
B Wasserstoff, Alkyl, Teil- oder Perfluoralkyl bedeutet,

Von den nichtionischen Fluortensiden der Gruppe a) werden solche der Formeln Ic oder Id besonders bevorzugt eingesetzt

B-[O-R_{H}]ₑ-A-[O-R_{F}]ₐ-A'-[O-R'_{H}]_{d}-B' (Ic),

B-[O-R_{F}]ₑ-A-[O-R_{H}]ₐ-A'-[O-R'_{F}]_{d}-B' (Id),

worin
A und A' unabhängig voneinander eine kovalente Bindung oder eine zweiwertige Brückengruppe, die mit den Blöcken [O-R_{F}], [O-R_{H}] und [O-R'_{H}] über C-C- und/oder C-O-Bindungen verknüpft ist, bedeuten,
B und B' unabhängig voneinander Wasserstoff, ein teil- oder perfluorierter Alkylrest mit 1 bis 6 Kohlenstoffatomen oder ein Alkylrest mit 1 bis 6 Kohlenstoffatomen ist,
R_{F} ein Rest der Formel -CₘFₙHₒ- ist, wobei innerhalb eines Polyetherrestes die Indizes m, n und o im Rahmen der gegebenen Definitionen unterschiedlich sein können,
R'_{F} ein Rest der Formel -C_{m'}F_{n'}H_{o'}- ist, wobei innerhalb eines Polyetherrestes die Indizes m, n und o im Rahmen der gegebenen Definitionen unterschiedlich sein können,
m und m' unabhängig voneinander ganze Zahlen von 2 bis 12 bedeuten,
n und n' unabhängig voneinander ganze Zahlen von 1 bis 24 bedeuten,
o und o' unabhängig voneinander ganze Zahlen von 0 bis 23 bedeuten,
wobei die Summe von n und o dem Wert 2m entspricht,
R_{H} ein Rest der Formel -CₚH₂ₚ- ist, wobei innerhalb eines Polyetherrestes der Index p im Rahmen der gegebenen Definition unterschiedlich sein kann,
R'_{H} ein Rest der Formel -C_{q}H_{2q}- ist, wobei innerhalb eines Polyetherrestes der Index q im Rahmen der gegebenen Definition unterschiedlich sein kann,
p eine ganze Zahl von 2 bis 12, vorzugsweise von 2 bis 4, bedeutet,
q eine ganze Zahl von 2 bis 12, vorzugsweise von 2 bis 4, bedeutet,
a eine ganze Zahl von 1 bis 100 ist, vorzugsweise 2 bis 50,
d eine ganze Zahl von 1 bis 100 ist, vorzugsweise 2 bis 50, und
e eine ganze Zahl von 1 bis 100 ist vorzugsweise 2 bis 50.

Verbindungen der Formel Ic sind aus der US 7,230,140 B2 bekannt.

Gruppen A oder A' in den Verbindungen der Formeln Ia, Ib, Ic und Id sind bevorzugt kovalente Bindungen oder zweiwertige Brückengruppen, die ausgewählt sind aus der Gruppe Sauerstoffatom, Carbonsäurereste oder -O-CH₂-CF₂-. Bei der Auswahl der jeweiligen Brückengruppen ist darauf zu achten, dass diese mit den entsprechenden Blöcken C-C- und/oder C-O-Bindungen ausbilden.

Ganz besonders bevorzugt werden nichtionische Fluortenside der Gruppe a) mit der Formel Ica eingesetzt

HO-(CH₂CH₂O)ₙₐ-CH₂CF₂O-(CF₂CF₂O)ₚₐ-(CF₂O)_{qa}-CF₂CH₂-(OCH₂CH₂)ₙₐ-OH (Ica),

worin na eine ganze Zahl von 1 bis 20 ist,
pa eine ganze Zahl von 0 bis 12 und
qa eine ganze Zahl von 0 bis 20 bedeutet,
mit der Maßgabe, dass die Summe von pa und qa mindestens 1 sein muss.

Nichtionische Tenside dieses Typs sind unter der Bezeichnung Fluorolink® D10-H (Solvay Solexis) im Handel.

Weiterhin bevorzugt werden nichtionische Fluortenside der Gruppe a) mit der Formel Iaa eingesetzt

HO-(CH₂CH₂O)ₙₐ-CH₂CF₂O-(CF₂CF₂O)ₚₐ-(CF₂O)_{qa}-R_{F} (Iaa),

worin
der Rest R_{F} ein teil- oder perfluorierter Alkylrest mit 2 bis 12 Kohlenstoffatomen ist, wobei innerhalb eines Polyetherrestes die Anzahl der Kohlenstoffatome der teil- oder perfluorierten Alkylreste im Rahmen der gegebenen Definitionen unterschiedlich sein kann, und
die Indices na, pa und qa den Erläuterungen zur Stuktur Ica entsprechen.

Bei den Fluortensiden der Gruppe b) handelt es sich typischerweise um Verbindungen der Formel Ie 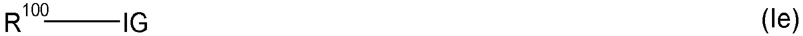
worin R¹⁰⁰ ein teil- oder perfluorierter Alkylrest mit weniger als 5 Kohlenstoffatomen ist, und
IG eine ionische Gruppe darstellt.

Beispiele für bevorzugte Gruppen IG sind Carboxylat-, Sulfonat-, Sulfat-, Phosphat-, Phosphonat- oder Ammoniumgruppen oder von diesen Gruppen abgeleitete Salze. Beispiele für Salze von Carboxylat-, Sulfonat-, Sulfat-, Phosphat-Phosphorsäure- oder Phosphonsäureestergruppen; oder Carbonsäure-, Sulfonsäure-, Phosphorsäure- oder Phosphonsäureamidgruppen. Es können auch Fluortenside eingesetzt werden, welche mehrere dieser Gruppen bzw. Atome aufweisen, beispielsweise Amino- und Carbonsäureamidgruppen.

Bevorzugt werden nichtionische Fluortenside der Gruppe a) eingesetzt, die mindestens eine (Poly)alkylenoxideinheit und mindestens eine teil- oder perfluorierte (Poly)alkylenoxideinheit aufweisen, die über ein Sauerstoffatom oder eine Carbonsäureestergruppe miteinander verbunden sind.

Unter teil- oder perfluorierter (Poly)alkylenoxideinheit sind im Rahmen dieser Beschreibung Reste zu verstehen, die mindestens eine teil- oder perfluorierte Alkylenoxideinheit, vorzugsweise mehrere teil- oder perfluorierte Alkylenoxideinheiten aufweisen, wobei die Teil- oder Perfluoralkylengruppen in einem Rest unterschiedliche Anzahlen von Kohlenstoffatomen aufweisen können. Diese unterschiedlichen Teil- oder Perfluoralkylengruppen können im Rest statistisch oder in Form von Blöcken wiederkehrender Struktureinheiten auftreten.

Unter (Poly)alkylenoxideinheit sind im Rahmen dieser Beschreibung Reste zu verstehen, die mindestens eine Alkylenoxideinheit aufweisen, vorzugsweise mehrere Alkylenoxideinheiten, wobei die Alkylengruppen in einem Rest unterschiedliche Anzahlen von Kohlenstoffatomen aufweisen können. Diese unterschiedlichen Alkylengruppen können im Rest statistisch oder in Form von Blöcken wiederkehrender Struktureinheiten auftreten.

Grundsätzlich können Fluortenside der Gruppe a) eingesetzt werden. Diese sind Blockcopolymere enthaltend Blöcke der Formel Ia

-[O-R_{F}]ₐ-A'-[O-R_{H}]_{d}- (la),

worin
R_{F} ein teil- oder perfluorierter Alkylenrest mit 2 bis 12 Kohlenstoffatomen ist, wobei innerhalb eines Polyetherrestes die Anzahl der Kohlenstoffatome der teil- oder perfluorierten Alkylenreste im Rahmen der gegebenen Definitionen unterschiedlich sein kann,
R_{H} einen Alkylenrest mit 2 bis 12, vorzugsweise 2 bis 6 Kohlenstoffatomen bedeutet, wobei innerhalb eines Polyetherrestes die Anzahl der Kohlenstoffatome der Alkylenreste im Rahmen der gegebenen Definitionen unterschiedlich sein kann,
A' eine kovalente Bindung ist oder eine zweiwertige Brückengruppe bedeutet, die mit den Blöcken [O-R_{F}] und [O-R_{H}] über C-C- und/oder C-O-Bindungen verknüpft ist,
a eine ganze Zahl von 1 bis 200 ist, vorzugsweise von 1 bis 50, insbesondere 1 bis 20, und
d eine ganze Zahl von 1 bis 200 ist, vorzugsweise von 1 bis 50, insbesondere von 1 bis 10.

Zu den eingesetzten Fluortensiden der Gruppe a) zählen die Verbindungen der Formel Ib

R¹-(O-R_{F})ₐ-A'-(O-R_{H})_{b}-A-(R'_{H}-O)_{c}-B (Ib),

worin R¹ Wasserstoff, ein teil- oder perfluorierter Alkylrest mit 1 bis 6 Kohlenstoffatomen oder ein Alkylrest mit 1 bis 6 Kohlenstoffatomen ist, vorzugsweise Wasserstoff, ein Alkylrest mit 1 bis 4 Kohlenstoffatomen oder ein teil- oder perfluorierter Alkylrest mit 1 bis 4 Kohlenstoffatomen,
R_{F} die oben definierte Bedeutung besitzt,
R_{H} und R'_{H} unabhängig voneinander Alkylenreste mit 2 bis 12, vorzugsweise 2 bis 6 Kohlenstoffatomen bedeuten, wobei innerhalb eines Polyetherrestes die Beispiele für Reste R¹⁰³ sind weiter oben aufgeführt.

Bei den Fluortensiden der Gruppe f) handelt es sich typischerweise um Verbindungen der Formel Ii 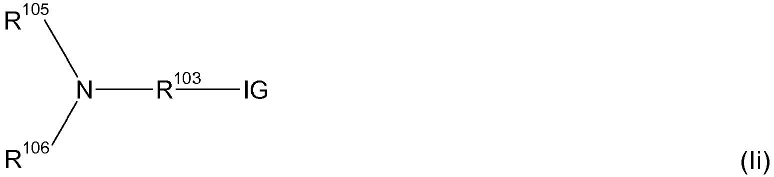
worin R¹⁰⁵ und R¹⁰⁶ unabhängig voneinander Teil- oder Perfluormethyl- oder -ethylreste bedeuten,
R¹⁰³ die oben definierte Bedeutung besitzt, wobei die Summe der Kohlenstoffatome von R¹⁰⁵, R¹⁰⁶ und R¹⁰³ mindestens 7 ist, und
IG eine ionische Gruppe darstellt, vorzugsweise eine Carboxylat-, Sulfonat-, Sulfat-, Phosphat-, Phosphonat- oder Ammoniumgruppe oder eines von dieser Gruppe abgeleiteten Salzes.

Beispiele für Reste R¹⁰⁵ und R¹⁰⁶ sind Mono-, Di- oder Trifluormethyl oder Mono-, Di-, Tri-, Tetra- oder Pentafluorethyl. Diese Reste können geradkettig oder verzweigt sein.

Beispiele für Reste R¹⁰³ sind weiter oben aufgeführt.

Bei den Fluortensiden der Gruppe g) handelt es sich typischerweise um Verbindungen der Formel Ij
die Brückengruppe mit den Blöcken [O-R_{H}] und [R'_{H}-O] eine C-O- und/oder eine C-C-Bindung ausbilden,
A' eine kovalente Bindung bedeutet,
a eine ganze Zahl von 2 bis 50 ist,
b eine ganze Zahl von 0 bis 25 ist,
c eine ganze Zahl von 1 bis 25 ist,
d eine ganze Zahl von 1 bis 25 ist, und
B Wasserstoff, Alkyl, Teil- oder Perfluoralkyl bedeutet,

Erfindungsgemäß werden von den nichtionischen Fluortensiden der Gruppe a) solche der Formeln Ic eingesetzt

B-[O-R_{H}]ₑ-A-[O-R_{F}]ₐ-A'-[O-R'_{H}]_{d}-B' (Ic),

worin
A und A' unabhängig voneinander eine kovalente Bindung oder eine zweiwertige Brückengruppe, die mit den Blöcken [O-R_{F}], [O-R_{H}] und [O-R'_{H}] über C-C- und/oder C-O-Bindungen verknüpft ist, bedeuten,
B und B' unabhängig voneinander Wasserstoff, ein teil- oder perfluorierter Alkylrest mit 1 bis 6 Kohlenstoffatomen oder ein Alkylrest mit 1 bis 6 Kohlenstoffatomen ist,
R_{F} ein Rest der Formel -CₘFₙHₒ- ist, wobei innerhalb eines Polyetherrestes die Indizes m, n und o im Rahmen der gegebenen Definitionen unterschiedlich sein können,
R'_{F} ein Rest der Formel -C_{m'}f_{n'}H_{o'}- ist, wobei innerhalb eines Polyetherrestes die Indizes m, n und o im Rahmen der gegebenen Definitionen unterschiedlich sein können,
m und m' unabhängig voneinander ganze Zahlen von 2 bis 12 bedeuten, 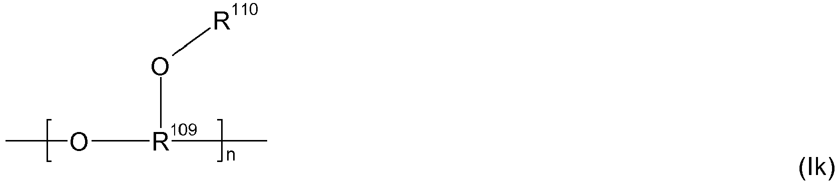
worin R¹⁰⁹ ein dreiwertiger aliphatischer Kohlenwasserstoffrest ist,
R¹¹⁰ und R¹¹¹ unabhängig voneinander Teil- oder Perfluoralkylreste mit 1 bis 4 Kohlenstoffatomen bedeuten,
R¹¹² ein vierwertiger aliphatischer Kohlenwasserstoffrest ist, und
n eine ganze Zahl von mindestens 1, vorzugsweise 1 bis 20, ist.

Beispiele für Reste R¹⁰⁹ sind Reste abgeleitet von Trimethylolpropan.

Beispiele für Reste R¹¹² sind Reste abgeleitet von Pentaerythrit.

Beispiele für Reste R¹¹⁰ und R¹¹¹ sind Mono-, Di- oder Trifluormethyl, Mono-, Di-, Tri-, Tetra- oder Pentafluorethyl, Mono-, Di-, Tri-, Tetra-, Penta-, Hexa- oder Heptafluorpropyl, oder Mono-, Di-, Tri-, Tetra-, Penta-, Hexa-, Hepta-, Octa- oder Nonafluorbutyl. Diese Reste können geradkettig oder verzweigt sein.

Neben den Struktureinheiten der Formel Ik und/oder II enthalten Fluortenside der Gruppe h) vorzugsweise Struktureinheiten der Formel Im 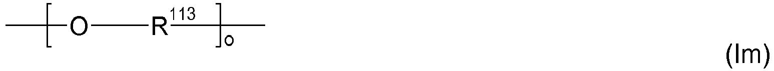 worin R¹¹³ ein Alkylenrest mit mindestens 2 Kohlenstoffatomen bedeutet, und o eine ganze Zahl von mindestens 1 ist, vorzugsweise von 2 bis 50.

Besonders bevorzugte erfindungsgemäß eingesetzte Fluortenside sind sehr gut biologisch abbaubar und weisen beim Einsatz in Dental-Abformmassen keine oder akzeptable Befunde gemäß ISO 10993-1 auf.

In einer bevorzugten Ausführungsform werden die Fluortenside der Gruppen a) bis h) in Kombination mit anderen ionischen und/oder nichtionischen und/oder amphoteren Fluortensiden eingesetzt.

Besonders bevorzugt werden Kombinationen von Fluortensiden der Gruppen a) bis h) mit zusätzlichen nichtionischen Tensiden eingesetzt.

Zu den besonders bevorzugt eingesetzten nichtionischen Tensiden zählen Fettalkoholethoxylate, insbesondere Verbindungen der allgemeinen Formel R¹¹⁴-O-(CH₂-CH₂-O)ₙ₁₀-R¹¹⁵, worin R¹¹⁴ langkettiges Alkyl, insbesondere C₁₀-C₁₈-Alkyl ist, R¹¹⁵ Wasserstoff oder Alkyl mit bis zu 4 Kohlenstoffatomen, und n10 eine ganze Zahl von 1 bis 30 bedeuten; oder
Alkylphenylethoxylate, insbesondere Verbindungen der allgemeinen Formel R¹¹⁶-C₆H₄-O-(CH₂-CH₂-O)ₙ₁₁-R¹¹⁷, worin R¹¹⁶ Alkyl, insbesondere C₆-C₁₂-Alkyl ist, R¹¹⁷ Wasserstoff oder Alkyl mit bis zu 4 Kohlenstoffatomen, und n11 eine ganze Zahl von 1 bis 30 bedeuten; oder
Polysorbate, insbesondere Verbindungen der Formel In 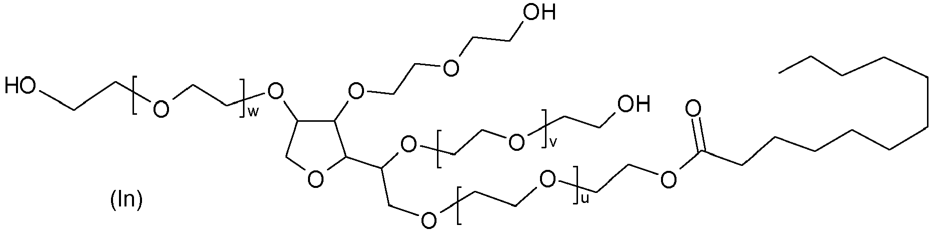 worin u, v und w unabhängig voneinander ganze Zahlen von 2 bis 20 sind; oder

Alkylpolyglucoside, insbesondere Verbindungen der Formel Io 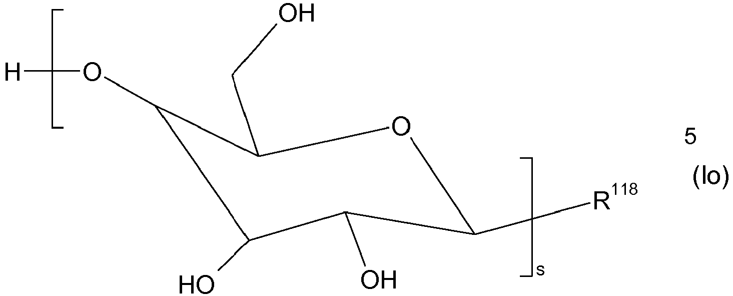 worin R¹¹⁸ langkettiges Alkyl, insbesondere C₆-C₁₆-Alkyl ist, und s eine ganze Zahl von 1 bis 10 bedeutet.

Ebenfalls besonders bevorzugt werden Kombinationen von Fluortensiden der Gruppen a) bis h) mit zusätzlichen anionischen Tensiden eingesetzt.

Zu den besonders bevorzugt eingesetzten anionischen Tensiden zählen Alkyl- oder Alkenylcarboxylate oder deren Salze, insbesondere Verbindungen der allgemeinen Formel (R¹¹⁹-COO⁻)ₜ M^{t+}, worin R¹¹⁹ langkettiges Alkyl oder langkettiges Alkenyl, insbesondere C₆-C₁₈-Alkyl oder C₆-C₁₈-Alkenyl ist, M Wasserstoff oder ein t-wertiges Metallion ist und t 1, 2 oder 3 bedeutet; oder Alkylbenzolsulfonate, insbesondere Verbindungen der allgemeinen Formel (R¹²⁰-C₆H₄-SO₃⁻)ₜ M^{t+}, worin R¹²⁰ Alkyl, insbesondere C₄-C₁₈-Alkyl ist, M Wasserstoff oder ein t-wertiges Metallion ist und t 1, 2 oder 3 bedeutet; oder Alkylsulfonate, insbesondere Verbindungen der allgemeinen Formel (R¹²¹-SO₃⁻)ₜ M^{t+}, worin R¹²¹ Alkyl, insbesondere C₄-C₁₈-Alkyl ist, M Wasserstoff oder ein t-wertiges Metallion ist und t 1, 2 oder 3 bedeutet; oder
Fettalkoholsulfonate, insbesondere Verbindungen der allgemeinen Formel (R¹²¹-OSO₃⁻)ₜ M^{t+}, worin R¹²¹ Alkyl, insbesondere C₄-C₁₈-Alkyl ist, M Wasserstoff oder ein t-wertiges Metallion ist und t 1, 2 oder 3 bedeutet; oder Dialkylsulfosuccinate, insbesondere Verbindungen der allgemeinen Formel (R¹²²-O-CO-CH₂-CH(SO₃⁻)-CO-O-R¹²³)ₜ M^{t+}, worin R¹²² und R¹²³ unabhängig voneinander Alkyl bedeuten, insbesondere C₄-C₁₈-Alkyl, M Wasserstoff oder ein t-wertiges Metallion ist und t 1, 2 oder 3 bedeutet.

Ebenfalls besonders bevorzugt werden Kombinationen von Fluortensiden der Gruppen a) bis h) mit zusätzlichen kationischen Tensiden eingesetzt.

Zu den besonders bevorzugt eingesetzten kationischen Tensiden zählen quaternäre Ammoniumverbindungen, insbesondere Verbindungen der allgemeinen Formel (R¹²⁴R¹²⁵R¹²⁶R¹²⁷N)ₜ⁺ An^{t-}, worin R¹²⁴, R¹²⁵, R¹²⁶ und R¹²⁷ unabhängig voneinander Wasserstoff, Alkyl oder Aryl bedeuten, wovon mindestens einer dieser Reste ein langkettiges Alkyl ist, insbesondere C₆-C₁₈-Alkyl, An ein t-wertiges Anion, vorzugsweise ein Halogenidanion ist, und t 1, 2 oder 3 bedeutet; oder
quaternäre Ammoniumesterverbindungen sind, insbesondere Verbindungen der allgemeinen Formel (R¹²⁸R¹²⁹N(R¹³⁰-COOR¹³¹)₂)ₜ⁺ An^{t-}, worin R¹²⁸ und R¹²⁹ unabhängig voneinander Wasserstoff, Alkyl oder Aryl bedeuten, wovon mindestens einer dieser Reste ein langkettiges Alkyl ist, insbesondere C₆-C₁₈-Alkyl, R¹³⁰ einen Alkylenrest darstellt, insbesondere einen Alkylenrest mit 2 bis 4 Kohlenstoffatomen, R¹³¹ Alkyl oder Aryl bedeutet, vorzugsweise C₁-C₆-Alkyl, An ein t-wertiges Anion, vorzugsweise ein Halogenidanion ist, und t 1, 2 oder 3 bedeutet.

Ebenfalls besonders bevorzugt werden Kombinationen von Fluortensiden der Gruppen a) bis h) mit zusätzlichen amphoteren Tensiden eingesetzt.

Zu den besonders bevorzugt eingesetzten amphoteren Tensiden zählen Betaine, insbesondere Verbindungen der allgemeinen Formel (R¹³²CO-NH-R¹³³-N⁺R¹³⁴R¹³⁵-R¹³⁶-COO-), worin R¹³², R¹³⁴ und R¹³⁵ unabhängig voneinander Alkyl oder Aryl bedeuten, vorzugsweise C₁-C₆-Alkyl, R¹³³ und R¹³⁶ Alkylen sind, von denen eines gegebenenfalls mit einer Hydroxylgruppe substituiert ist, vorzugsweise C₁-C₄-Alkylen; oder
Sultaine, insbesondere Verbindungen der allgemeinen Formel (R¹³⁷CO-NH-R¹³⁸-N⁺R¹³⁹R¹⁴⁰⁻R¹⁴¹-SO₃⁻), worin R¹³⁷, R¹³⁹ und R¹⁴⁰ unabhängig voneinander Alkyl oder Aryl bedeuten, vorzugsweise C₁-C₆-Alkyl, R¹³⁸ und R¹⁴¹ Alkylen sind, von denen eines gegebenenfalls mit einer Hydroxylgruppe substituiert ist, vorzugsweise C₁-C₄-Alkylen.

Das erfindungsgemäß eingesetzte Silizium aufweisende Tensid enthält vorzugsweise mindestens einen (Poly)alkylenoxidrest und besitzt eine Molmasse von weniger als 6.000 g/mol, vorzugsweise von weniger als 4.000 g/mol, insbesondere von 350 bis 2000 g/mol.

Das erfindungsgemäß eingesetzte Silizium aufweisende Tensid enthält neben dem mindestens einen (Poly)alkylenoxidrest mindestens einen Rest, der Organosiloxanreste oder der Organosilanreste enthält. Die organischen Gruppen sind Kohlenwasserstoffreste, die gegebenenfalls teil- oder perfluoriert sind.

Derartige Organosiloxantenside oder Organocarbonsilantenside sind an sich bekannt.

Bevorzugt werden Silizium aufweisende Tenside eingesetzt, die Organosiloxantenside der Formeln II oder III sind oder die Organocarbonsilantenside der Formel IV, V oder VI sind

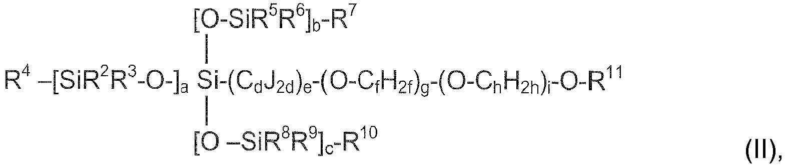

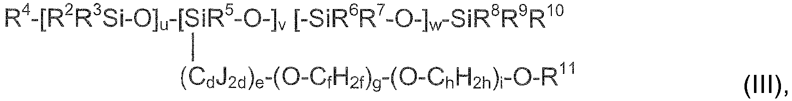

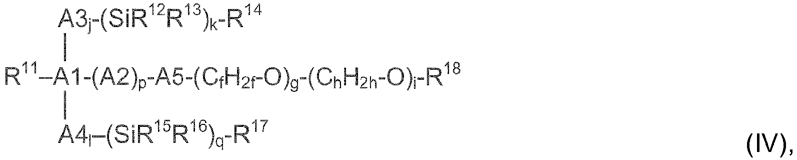

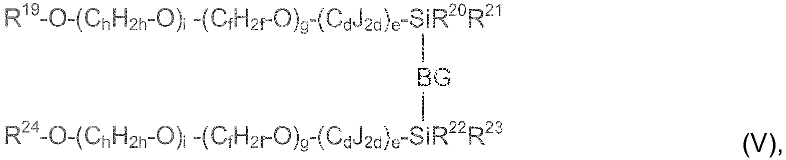

R¹⁹-O-(CₕH₂ₕ-O)ᵢ-(C_{f}H_{2f}-O)_{g}-(C_{d}J_{2d})ₑ-SiR²⁰R²¹R²² (VI),

worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
a, b, c und w unabhängig voneinander ganze Zahlen von 0 bis 100 bedeuten, vorzugsweise 0 bis 75, insbesondere 0 bis 35 und ganz besonders bevorzugt 0 bis 15,
v eine ganze Zahl von 1 bis 100 bedeutet, vorzugsweise 1 bis 15 und ganz besonders bevorzugt 1 bis 6,
wobei die Summe von a, b und c zwischen 1 und 300 beträgt, vorzugsweise 1 bis 50, insbesondere 1 bis 10 und ganz besonders bevorzugt 1 bis 3,
und die Summe von v und w zwischen 1 und 200 beträgt, vorzugsweise 2 bis 90,
u 0 oder 1 ist,
d eine ganze Zahl von 1 bis 10 ist, vorzugsweise 1 bis 6 und insbesondere 1 bis 3,
J Wasserstoff oder Fluor bedeutet, vorzugsweise Wasserstoff,
e 0 oder 1 ist,
f und h unabhängig voneinander ganze Zahlen von 2 bis 6 bedeuten,
g und i unabhängig voneinander ganze Zahlen von 0 bis 30 sind, vorzugsweise 0 bis 15, wobei die Summe von g und i 1 bis 60 bedeutet, vorzugsweise 2 bis 30, insbesondere 2 bis 15,
R¹¹ Wasserstoff, Alkyl, Alkenyl oder Aryl ist, das gegebenenfalls teilweise oder vollständig fluoriert ist, vorzugsweise Wasserstoff oder Methyl,
R¹², R¹³, R¹⁴, R¹⁵, R¹⁶ und R¹⁷ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
k und q unabhängig voneinander 0 oder 1 bedeuten,
A1 Kohlenstoff oder Silizium bedeutet,
A2, A3 und A4 unabhängig voneinander eine Gruppe C_{d}J_{2d} ist, worin J und d die oben definierten Bedeutungen aufweisen,
j, p und I unabhängig voneinander 0 oder 1 sind,
A5 eine zweiwertige Brückengruppe, insbesondere -O-, -CO-O- oder -CO- bedeutet,
R¹⁸ Wasserstoff, Alkyl, Alkenyl oder Aryl ist, das gegebenenfalls teilweise oder vollständig fluoriert ist, vorzugsweise Wasserstoff oder Methyl,
R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl, BG eine zweiwertige Brückengruppe ist, und
R¹⁹ und R²⁴ unabhängig voneinander Wasserstoff, Alkyl, Alkenyl oder Aryl sind, die gegebenenfalls teilweise oder vollständig fluoriert sind, vorzugsweise Wasserstoff oder Methyl,
mit der Maßgabe, dass von den Resten R², R³ und R⁴ und/oder den Resten R⁵, R⁶ und R⁷ und/oder den Resten R⁸, R⁹ und R¹⁰ und/oder den Resten R¹⁵, R¹⁶ und R¹⁷ und/oder den Resten R²⁰ und R²¹ und/oder den Resten R²² und R²³ und/oder den Resten R²⁰, R²¹ und R²² nur einer Wasserstoff sein kann,
wobei f und h innerhalb eines Moleküls im Rahmen der gegebenen Definition unterschiedliche Werte annehmen können.

Unterschiedliche Indizes f und h bedeuten, dass Alkylenoxideinheiten unterschiedlicher Kohlenstoffanzahl vorliegen können, die in statistischer Verteilung oder in Form von Blöcken auftreten können.

Besonders bevorzugt werden Organosiloxantenside der Formel IIIa eingesetzt 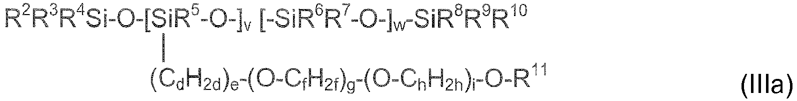
worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
v eine ganze Zahl von 1 bis 100 bedeutet,
w eine ganze Zahl von 0 bis 100 bedeutet,
d eine ganze Zahl von 1 bis 10 ist,
e 0 oder 1 ist,
f und h unabhängig voneinander ganze Zahlen von 2 bis 6 bedeutet,
g und i unabhängig voneinander ganze Zahlen von 0 bis 30 sind, wobei die Summe von g und i 1 bis 60 bedeutet, und
R¹¹ Wasserstoff, Alkyl, Alkenyl oder Aryl ist.

Weitere besonders bevorzugte Organosiloxantenside sind Verbindungen der Formel IIIb oder IIIc 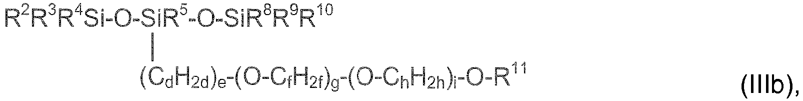 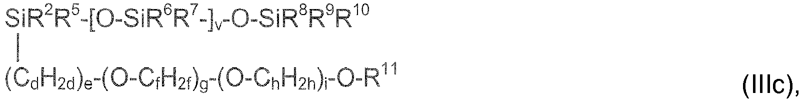
worin R², R³, R⁴, R⁵, R⁶, R⁷, R⁸, R⁹ und R¹⁰ unabhängig voneinander Wasserstoff, Alkyl oder Alkenyl sind, vorzugsweise Wasserstoff, Methyl, insbesondere Butyloxy und Methoxy, Vinyl oder Allyl,
v eine ganze Zahl von 1 bis 100 ist,
d, e, f, g, h und i die weiter oben definierten Bedeutungen besitzen, und
R¹¹ Wasserstoff, Alkyl, Alkenyl oder Aryl ist.

Ganz besonders bevorzugte Organosiloxantenside sind Verbindungen der Formel IIId 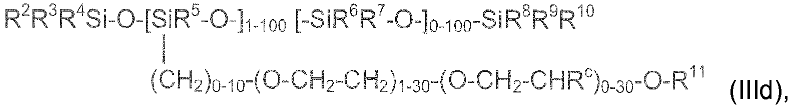
worin R², R³, R⁴, R⁵, R⁶, R⁷, R⁸, R⁹ und R¹⁰ unabhängig voneinander Alkyl, Alkenyl, Alkinyl, Aryl, Aryloxy, Aralkyl, Aralkyloxy, Alkylaryl oder Alkylaryloxy bedeuten, vorzugsweise Methyl,
R^{c} C₁-C₆ Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Weitere ganz besonders bevorzugte Organosiloxantenside sind Verbindungen der Formel IIIe 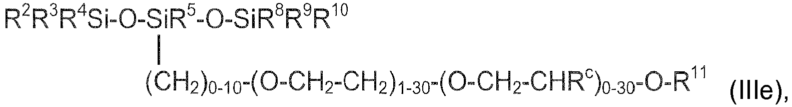
worin R², R³, R⁴, R⁵, R⁸, R⁹ und R¹⁰ unabhängig voneinander Alkyl, Alkenyl, Alkinyl, Aryl, Aryloxy, Aralkyl, Aralkyloxy, Alkylaryl oder Alkylaryloxy bedeuten, vorzugsweise Methyl,
R^{c} C₁-C₆-Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Weitere ganz besonders bevorzugte Organosiloxantenside sind Verbindungen der Formel IIIf 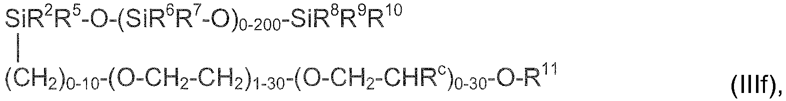
worin R², R⁵, R⁶, R⁷, R⁸, R⁹ und R¹⁰ unabhängig voneinander Alkyl, Alkenyl, Alkinyl, Aryl, Aralkyl oder Alkylaryl bedeuten, vorzugsweise Methyl,
R^{c} C₁-C₆-Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Ganz besonders bevorzugt werden Organosiloxantenside der Formel IIIg eingesetzt

[R²R³R⁴Si-O]₂-SiR⁵-(CH₂)₁₋₁₀-(O-CH₂-CH₂)₁₋₃₀-O-R¹¹ (IIIg),

worin R², R³, R⁴ und R⁵ unabhängig voneinander Alkyl und/oder Alkenyl bedeuten, vorzugsweise Methyl,
R¹¹ Wasserstoff, Alkyl, Alkenyl oder Aryl ist, vorzugsweise Wasserstoff oder Methyl.

Ganz besonders bevorzugte Silizium aufweisende Tenside sind Verbindungen der Formeln VII, VIII, IX und X 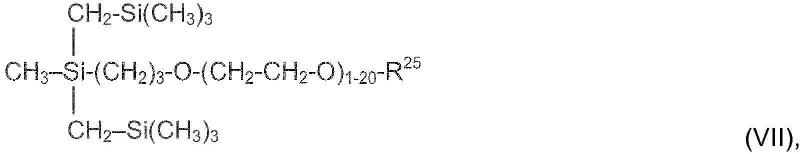 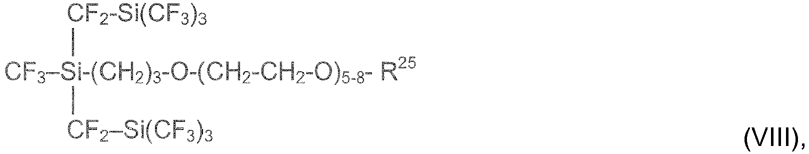 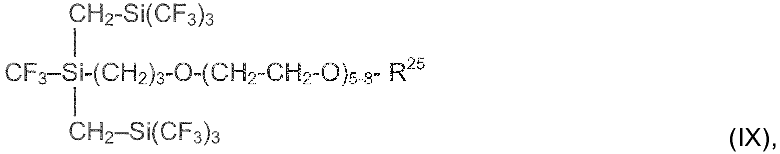 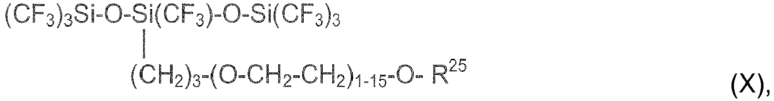 worin R²⁵ Wasserstoff, Methyl, Ethyl, Propyl oder Butyl ist, vorzugsweise Wasserstoff oder Methyl.

Diese bevorzugten Silicontenside oder Carbonsilantenside sind z.B. unter der Bezeichnung Masil SF 19 (Fa. Lubrizol), Silwet L77 (Fa. GE-Bayer-Silicones) kommerziell erhältlich.

Besonders bevorzugt werden Carbosilantenside der Formel IVa eingesetzt 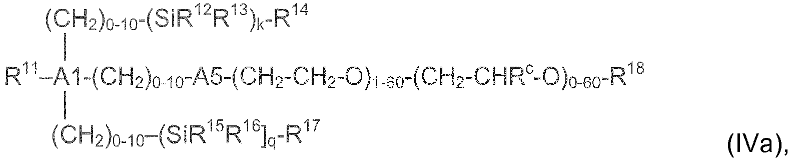
worin R¹¹, R¹², R¹³, R¹⁴, R¹⁵, R¹⁶ und R¹⁷ unabhängig voneinander Wasserstoff, Alkyl, Alkenyl, Alkinyl, Aryl, Aralkyl oder Alkylaryl, sind,
die Summe von k und q 1 oder 2 ist,
A5 ein Rest -O-, -CO-, -CO-O-, -S-, -NR¹⁹-, -CO-NR¹⁹-, -SO₂- oder -SO₂-NR¹⁹- ist,
R¹⁹ Wasserstoff, Alkyl, Alkenyl oder Aryl bedeutet,
R^{c} C₁-C₆-Alkyl, vorzugsweise Methyl oder Ethyl, oder Phenyl ist, und
R¹⁸ Wasserstoff, Alkyl, Alkenyl oder Aryl bedeutet.

Ebenfalls besonders bevorzugt werden Carbosilantenside der Formel Va eingesetzt 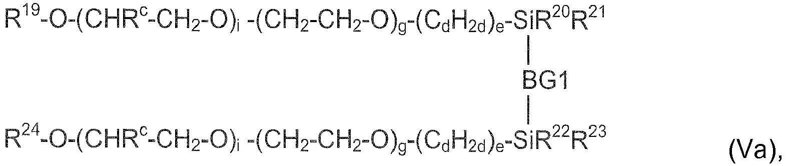
worin R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet,
BG1 eine Brückengruppe ist, die ausgewählt wird aus den Gruppen der Formeln -O-, Alkylen, Polyoxyalkylen, Phenylen, Cycloalkylen, Bicycloalkadiendiyl und -C₆H₅-K-C₆H₅-,
K eine direkte C-C-Bindung, -O-, -SO₂-, Alkylen oder Haloalkylen ist, und
R¹⁹ und R²⁴ unabhängig voneinander Wasserstoff, Alkyl, Alkenyl oder Aryl bedeuten.

Ganz besonders bevorzugt werden Verbindungen der Formel Va eingesetzt, worin BG1 eine Brückengruppe ist, die ausgewählt wird aus der Gruppe bestehend aus Formeln -O-, C₁-C₁₀-Alkylen, -(O-CH₂-CHR^{c})₂₋₆₀-, -(CH₂)₁₋₁₀-(O-CH₂-CHR^{c})₂₋₆₀-(CH₂)₁₋₁₀-, Phenylen, Cyclohexylen, Cyclopentylen, Norbonen-diyl, Bis-cyclopentadienyl-diyl, -C₆H₅-O-C₆H₅-, -C₆H₅-SO₂-C₆H₅-, -C₆H₅-C(CH₃)₂-C₆H₅- und -C₆H₅-C(CF₃)₂-C₆H₅-.

Ebenfalls besonders bevorzugt werden Carbosilantenside der Formel VIa eingesetzt

R¹⁹-O-(CHR^{c}-CH₂-O)ᵢ-(CH₂-CH₂-O)_{g}-(C_{d}H_{2d})ₑ-SiR²⁰R²¹R²² (VIa),

worin R²⁰, R²¹ und R²² unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, vorzugsweise Wasserstoff, Alkyl, Alkyloxy oder Alkenyl,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet, und
R¹⁹ und R²⁴ unabhängig voneinander Wasserstoff, Alkyl, Alkenyl oder Aryl bedeuten.

Besonders bevorzugt werden Fluor-Carbosilantenside der Formel IVb eingesetzt 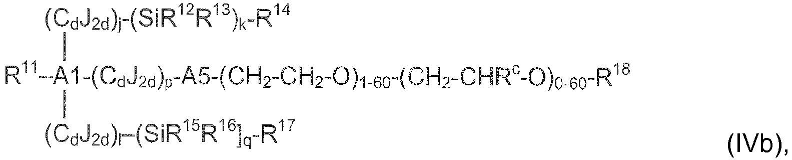
worin R¹¹, R¹², R¹³, R¹⁴, R¹⁵, R¹⁶ und R¹⁷ unabhängig voneinander Wasserstoff, Fluor, Alkyl, Alkyloxy, Alkenyl, Alkenyloxy, Alkinyl, Alkinyloxy, Aryl, Aryloxy, Aralkyl, Alkylaryl, Fluoralkyl, Fluoralkyloxy, Fluoralkenyl, Fluoralkenyloxy, Fluoralkinyl, Fluoralkinyloxy, Fluoraryl, Fluoraryloxy, Fluoraralkyl oder Fluoralkylaryl sind, vorzugsweise Wasserstoff, Fluor, Alkyl, Fluoralkyl, Alkoxy, Fluoralkoxy, Alkenyl und Fluoralkenyl, wobei mindestens einer dieser Reste Fluor enthält und die Fluor enthaltenden Reste teil- oder perfluoriert sein können,
d eine ganze Zahl von 1 bis 10 bedeutet,
j, p und I 0 oder 1 sind,
die Summe von k und q 1 oder 2 ist,
A5 ein Rest -O-, -CO-O-, -S-, -NR¹⁹-, -CO-NR¹⁹-, -SO₂- oder -SO₂-NR¹⁹- ist,
R¹⁹ Wasserstoff, Alkyl, Alkenyl oder Aryl bedeutet,
R^{c} C₁-C₆-Alkyl, vorzugsweise Methyl oder Ethyl, oder Phenyl ist, und
R¹⁸ Wasserstoff, Alkyl, Alkenyl oder Aryl bedeutet.

Ganz besonders bevorzugt werden Fluor-Silicontenside der Formel IVc eingesetzt, 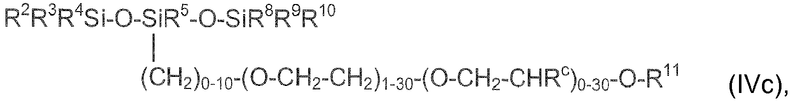
worin R², R³, R⁴, R⁵, R⁸, R⁹ und R¹⁰ unabhängig voneinander Alkyl, Alkyloxy, Alkenyl, Alkenyloxy, Alkinyl, Alkinyloxy, Aryl, Aryloxy, Aralkyl, Aralkyloxy, Alkylaryl oder Alkylaryloxy, Fluoralkyl, Fluoralkyloxy, Fluoralkenyl, Fluoralkenyloxy, Fluoralkinyl, Fluoralkinyloxy, Fluoraryl, Fluoraryloxy, Fluoraralkyl, Fluoraralkyloxy, Fluoralkylaryl oder Fluoralkylaryloxy bedeuten, vorzugsweise Methyl, Trifluormethyl, C₁-C₄-Alkoxy, C₁-C₄-Fluoralkoxy, insbesondere Methoxy oder Trifluormethyl, und/oder Vinyl und/oder Allyl, wobei mindestens einer dieser Reste Fluor enthält und die Fluor enthaltenden Reste teil- oder perfluoriert sein können,
R^{c} C₁-C₆-Alkyl, vorzugsweise Methyl oder Ethyl, oder Phenyl bedeutet, und
R¹¹ Wasserstoff, Alkyl, Alkenyl oder Aryl ist, vorzugsweise Wasserstoff oder Methyl.

Ebenfalls besonders bevorzugt werden Fluor-Carbosilantenside der Formel Vc eingesetzt 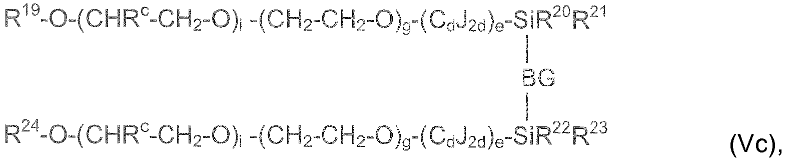
worin R²⁰, R²¹, R²² und R²³ unabhängig voneinander Wasserstoff, Fluor, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy, Fluoralkyl, Fluoralkyloxy, Fluoralkenyl, Fluoralkenyloxy, Fluoralkinyl, Fluoralkinyloxy, Fluoraryl, Fluoraryloxy, Fluoraralkyl, Fluoraralkyloxy, Fluoralkylaryl und/oder Fluoralkylaryloxy bedeuten, vorzugsweise Wasserstoff, Fluor, Alkyl, Fluoralkyl, Alkyloxy, Fluoralkyloxy, Alkenyl oder Fluoralkenyl, wobei mindestens einer dieser Reste Fluor enthält und die Fluor enthaltenden Reste teil- oder perfluoriert sein können,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet,
BG eine Brückengruppe ist, die ausgewählt wird aus den Gruppen der Formeln -O-, Alkylen, Polyoxyalkylen, Phenylen, Cycloalkylen, Bicycloalkadiendiyl und -C₆H₅-K-C₆H₅-,
K eine direkte C-C-Bindung, -O-, -SO₂-, Alkylen oder Haloalkylen ist, und
R¹⁹ und R²⁴ unabhängig voneinander Wasserstoff, Alkyl, Alkenyl oder Aryl bedeuten.

Ganz besonders bevorzugt werden Verbindungen der Formel Vc eingesetzt, worin BG eine Brückengruppe ist, die ausgewählt wird aus der Gruppe bestehend aus Formeln -O-, C₁-C₁₀-Alkylen, -(O-CH₂-CHR^{c})₂₋₆₀-, -(CH₂)₁₋₁₀-(O-CH₂-CHR^{c})₂₋₆₀-(CH₂)₁₋₁₀-, Phenylen, Cyclohexylen, Cyclopentylen.

Ebenfalls besonders bevorzugt werden Fluor-Carbosilantenside der Formel VIb eingesetzt

R¹⁹-O-(CHR^{c}-CH₂-O)ᵢ-(CH₂-CH₂-O)_{g}-(C_{d}H_{2d})ₑ-SiR²⁰R²¹R²² (VIb),

worin R²⁰, R²¹ und R²² unabhängig voneinander Wasserstoff, Fluor, Alkyl, Alkenyl, Alkinyl, Aryl, Aralkyl und/oder Alkylaryl, Fluoralkyl, Fluoralkenyl, Fluoralkinyl, Fluoraryl, Fluoraralkyl und/oder Fluoralkylaryl bedeuten, vorzugsweise Wasserstoff, Fluor, Alkyl, Fluoralkyl, Alkenyl oder Fluoralkenyl, wobei mindestens einer dieser Reste Fluor enthält und die Fluor enthaltenden Reste teil- oder perfluoriert sein können,
d eine ganze Zahl von 1 bis 10 ist,
e 0 oder 1 ist,
g und i unabhängig voneinander ganze Zahlen von 0 bis 30 sind, wobei die Summe von g und i 1 bis 60 bedeutet, und
R¹⁹ und R²⁴ unabhängig voneinander Wasserstoff, Alkyl, Alkenyl oder Aryl bedeuten.

Die nichtionischen Silicontenside oder nichtionischen Carbonsilantenside der Formeln II bis X werden einzeln oder in Kombination zusammen mit den Fluortensiden der Gruppen a) bis h), insbesondere mit solchen der Formeln Ia bis II, oder zusammen mit Gemischen von zwei oder mehreren dieser Fluortenside eingesetzt.

Der Anteil an Fluortensid der Gruppen a) an der erfindungsgemäßen Zusammensetzung beträgt üblicherweise 0,001 bis 5,0 Gew.-%, vorzugsweise 0,01 bis 5,0 Gew.-%, bezogen auf die Zusammensetzung.

Werden in den Zusammensetzungen neben dem Fluortensid der Gruppen a) bis h) weitere nichtionische, anionische, kationische und/oder amphotere Fluortenside eingesetzt, so beträgt deren Anteil an der erfindungsgemäßen Zusammensetzung üblicherweise ebenfalls 0,001 bis 5,0 Gew.-%, vorzugsweise 0,01 bis 5,0 Gew.-%, bezogen auf die Zusammensetzung.

Der Anteil an Silizium aufweisendem nichtionischem Tensid an der erfindungsgemäßen Zusammensetzung beträgt üblicherweise 0,0 bis 5,0 Gew. %, vorzugsweise 0,05 bis 5,0 Gew.-%, besonders bevorzugt 0,1 bis 5,0 Gew.-%, bezogen auf die Zusammensetzung.

Kommen in den Zusammensetzungen Kombinationen von Fluortensid der Gruppen a) bis h) mit anderem Fluortensid zum Einsatz, so beträgt das Gewichtsverhältnis von Fluortensiden der Gruppen a) bis h) zu anderen Fluortensiden 100 : 1 bis 1 : 100, bevorzugt 50 : 1 bis 1 : 50, besonders bevorzugt 10 : 1 bis 1 : 10 und insbesondere bevorzugt 4 : 1 bis 1 : 5.

In den erfindungsgemäßen Kombinationen von Fluortensid mit Silicontensid beträgt das Gewichtsverhältnis von Silizium aufweisendem nichtionischem Tensid zu nichtionischem Fluortensid 100 : 1 bis 1 : 100, bevorzugt 50 : 1 bis 1 : 50, besonders bevorzugt 10 : 1 bis 1 : 10 und insbesondere bevorzugt 4: 1 bis 1 : 5.

Die Zusammensetzungen enthalten vorzugsweise neben dem Fluortensid der Gruppen a) bis h), dem Silicontensid und gegebenenfalls weiterem Fluortensid als weitere Komponente einen Alkenylreste und/oder Alkinylreste enthaltenden Polyether und/oder einen hydroxyl- und/oder aryloxy- und/oder arylalkyloxy- und/oder alkoxy-terminierten Polyether.

Diese zusätzliche Komponente bewirkt eine weitere Verbesserung der Kontakt-Diese zusätzliche Komponente bewirkt eine weitere Verbesserung der Kontaktwinkeleigenschaften des nicht ausgehärteten plastischen Materials und somit eine weitere Verbesserung der Formtreue des entstehenden Abdrucks.

Beispiele für bevorzugte Zusätze dieses Typs sind die Verbindungen der Formel XI

R³⁰-A-(R³⁰)ₙ₁ (XI),

worin R³⁰ ein ethylenisch ungesättigter Kohlenwasserstoffrest, vorzugsweise Allyl, Styryl, Acryloyl oder Methacryloly, oder Wasserstoff oder Alkyl bedeutet, und mehrere Reste R³⁰ eines Moleküls im Rahmen der gegebenen Definition unterschiedlich sein können,
n1 eine ganze Zahl von 1 bis 5 bedeutet, vorzugsweise 1, 2 oder 3, insbesondere 1,
A ein Rest der Formel Z-(O-(Cₙ₂H₂ₙ₂-O)ₘ₁-)ₙ₁₊₁ bedeutet,
Z ein n1-wertiger Kohlenwasserstoffrest ist, vorzugsweise ein von einem zwei-, drei-, vier-, fünf- oder sechswertigen aliphatischen Alkohol abgeleiteter Rest, insbesondere ein von Alkylenglykol, Trimethylolpropan, Pentaerythrit oder Sorbit abgeleiteter Rest,
n2 eine ganze Zahl von 2 bis 8, vorzugsweise von 2 bis 4 bedeutet, und
m1 eine ganze Zahl von 1 bis 35.000, vorzugsweise von 50 bis 1.500 ist, wobei n2 und m1 innerhalb eines Moleküls im Rahmen der gegebenen Definition unterschiedlich sein können.

Die Indizes n2 und/oder m1 können innerhalb eines Moleküls sowie innerhalb einer Alkylenoxidkette im Rahmen der gegebenen Definition unterschiedliche Werte annehmen. Unterschiedliche Indizes n2 und/oder m1 bedeuten, dass Alkylenoxideinheiten unterschiedlicher Kohlenstoffanzahl vorliegen können, die in statistischer Verteilung oder in Form von Blöcken auftreten können bzw. dass die einzelnen Blöcke innerhalb eines Moleküls unterschiedliche Längen aufweisen können.

Ganz besonders bevorzugt eingesetzte Polyether sind Verbindungen der Formeln XII und XIII

CH₂=CH-CH₂-O-(Cₙ₂H₂ₙ₂-O)ₘ₉-CH₂-CH=CH₂ (XII),

R³¹-O-(Cₙ₂H₂ₙ₂-O)ₘ₉-R³² (XIII),

worin n2 eine ganze Zahl von 2 bis 8, vorzugsweise von 2 bis 4 bedeutet,
m9 eine ganze Zahl von 3 bis 70.000, vorzugsweise von 10 bis 2.500 ist, wobei n2 und m9 innerhalb eines Moleküls im Rahmen der gegebenen Definition unterschiedlich sein können, und
R³¹ und R³² unabhängig voneinander Wasserstoff oder C₁-C₆-Alkyl, insbesondere Wasserstoff und/oder Methyl, Ethyl oder Propyl bedeuten.

Beispiele für besonders bevorzugte Alkylenoxideinheiten sind -CH₂-CH₂-O-, -CH₂-CH₂-CH₂-O-, -CH₂-CH(CH₃)-O- und -CH₂-CH₂-CH₂-CH₂-O-.

Besonders bevorzugt werden Polyether der Formeln XII und XIII, insbesondere solche, die Ethylenoxideinheiten oder Ethylenoxideinheiten und Propylenoxideinheiten oder Ethylenoxideinheiten und Butylenoxideinheiten aufweisen.

Diese Polyether kommen in der Regel als Gemische von Polymeren unterschiedlicher Kettenlänge zum Einsatz. Typische Molekulargewichte (Zahlenmittel) bewegen sich im Bereich von 150 bis 3.000.000, vorzugsweise von 250 bis 100.000 und besonders bevorzugt von 250 bis 50.000.

Der Anteil dieser Polyether an der erfindungsgemäßen Zusammensetzung beträgt üblicherweise 0,1 bis 25,0 Gew.-%, vorzugsweise 0,1 bis 10,0 Gew.-%, und besonders bevorzugt 0,5 bis 2,5 Gew.-%, bezogen auf die Zusammensetzung.

Die Zusammensetzungen enthalten vorzugsweise neben dem Fluortensid der Gruppen a) bis h), dem Silicontensid und gegebenenfalls weiterem Fluortensid als weitere Komponente ein Polyol.

Diese zusätzliche Komponente bewirkt eine weitere Verbesserung der Kontaktwinkeleigenschaften des nicht ausgehärteten plastischen Materials und somit eine weitere Verbesserung der Detailgenauigkeit des entstehenden Abdrucks.

Bei dieser zusätzlichen Komponente kann es sich um monomere, oligomere oder polymere Polyole handeln, Diese. können primäre, sekundäre und/oder tertiäre Hydroxylgruppen aufweisen. Die Hydroxylgruppen können an aromatische Reste gebunden sein, bevorzugt jedoch an aliphatische Reste.

Der Anteil dieser Polyole an der erfindungsgemäßen Zusammensetzung beträgt üblicherweise 0,1 bis 25,0 Gew.-%, vorzugsweise 0,1 bis 10,0 Gew.-%, und besonders bevorzugt 0,5 bis 2,5 Gew.-%, bezogen auf die Zusammensetzung.

Bevorzugte Polyole werden ausgewählt aus der Gruppe der Kohlehydrate, der Polyvinylalkohole, der aliphatischen Di-, Tri-, Tetra-, Penta- und/oder Hexaole und Mischungen von zwei oder mehreren dieser Polyole.

Besonders bevorzugte Polyole werden ausgewählt aus der Gruppe der Polyvinylalkohole, der Polysaccharide, Trimethylolpropan, Pentaerythritol, Dipentaerythritol, Glycerin, Allyloxy-1,2-propandiol, 2-Methyl-2,4-pentandiol, Trimethylolpropanallylether, Decandiol, Nonandiol, Octandiol, Heptandiol, Hexandiol, Pentandiol, Butandiol, Propandiol, Ethandiol, Fructose, Glucose und Mischungen von zwei oder mehreren dieser Polyole, insbesondere Glycerin.

In einer weiteren bevorzugten Ausführungsform enthält die Zusammensetzung neben dem Polyol als weitere Komponente einen Alkenylreste und/oder Alkinylreste enthaltenden Polyether und/oder einen aryloxy- und/oder arylalkyloxy- und/oder hydroxyl- und/oder alkoxy-terminierten Polyether.

Durch die Anwesenheit dieser beiden Komponenten lassen sich die Kontaktwinkeleigenschaften des nicht ausgehärteten plastischen Materials verbessern.

Bei den härtbaren Polymersystemen können unterschiedliche Typen eingesetzt werden. In Abhängigkeit vom jeweiligen Polymersystem können die erfindungsgemäßen Zusammensetzungen als Einkomponentensysteme oder als Mehrkomponentensysteme, vorzugsweise als Zweikomponentensysteme, vorliegen. Die härtbaren Polymersysteme und deren zusätzliche Komponenten, wie Katalysatoren und/oder Initiatoren, sind dem Fachmann an sich bekannt.

Der Anteil der härtbaren Polymeren an der erfindungsgemäßen Zusammensetzung beträgt üblicherweise 5 bis 80 Gew.-%, vorzugsweise 20 bis 70 Gew.-%, bezogen auf die Zusammensetzung.

Der Anteil der Katalysatoren und/oder der Photoinitiatoren an der erfindungsgemäßen Zusammensetzung beträgt üblicherweise 0,00005 bis 10 Gew.-%, vorzugsweise 0,0001 bis 5 Gew.-%, bezogen auf die Zusammensetzung.

Bevorzugt werden Zusammensetzungen enthaltend einen vernetzbaren Polyether, der Alkoxysilylreste, Aziridinoreste, von einer ethylenisch ungesättigten Carbonsäure abgeleitete Reste, Alkenylreste als vernetzbare Gruppen oder ten Carbonsäure abgeleitete Reste, Alkenylreste als vernetzbare Gruppen oder über ringöffnende Metathesereaktion vernetzbare Gruppen aufweist, einen Vernetzungskatalysator und/oder Photoinitiator sowie ein Fluortensid der Gruppen a) bis h), ein Silizium enthaltende Gruppen aufweisendes nichtionisches Tensid mit einer Molmasse von weniger als 6.000 g/mol.und gegebenenfalls ein weiteres Fluortensid.

Besonders bevorzugt werden Zusammensetzungen enthaltend Organopolysiloxane als härtbare Polymersysteme eingesetzt. Derartige Zusammensetzungen sind z.B. aus DE 34 10 646 A1 bekannt. Bekanntermaßen unterscheidet man zwischen durch Additionsreaktion vernetzenden Organopolysiloxanen, durch Kondensationsreaktion vernetzenden Organopolysiloxanen, und durch ringöffnende Metathesereaktion vernetzenden Organopolysiloxanen. Erfindungsgemäß können alle diese Polymersysteme eingesetzt werden.

Bevorzugt werden durch Additionsreaktion vernetzende Organopolysiloxane. Durch Additionsreaktion härtbare Organopolysiloxane sind beispielsweise aus DE 34 10 646 A1, DE 100 17 154 A1 bekannt.

Diese kommen üblicherweise in Form einer Mehrkomponenten Dentalabformmasse enthaltend Komponenten A und B zum Einsatz. Darin enthält
a) Komponente A ein Organopolysiloxan mit mindestens 2 ethylenisch ungesättigten Gruppen und einen Hydrosilylierungskatalysator,
b) Komponente B enthält ein Organohydrogenpolysiloxan, und
c) mindestens eine der Komponenten A und/oder B enthält das Fluortensid der Gruppen a) bis h), das Silizium aufweisende nichtionische Tensid und gegebenenfalls ein weiteres Fluortensid.

Als Organopolysiloxan mit mindestens 2 ethylenisch ungesättigten Gruppen kommen üblicherweise Organopolysiloxane zum Einsatz, die mindestens 2 an Si-Atome gebundene Allyl- oder insbesondere Vinylgruppen aufweisen.

Typischerweise handelt es sich dabei um Verbindungen der folgenden Formeln XIV oder XV

CH₂=CH-SiR₂-B-SiR₂-CH=CH₂ (XIV),

CH₂=CH-CH₂-SiR₂-B-SiR₂-CH₂-CH=CH₂ (XV),

worin B ein Rest der Formel -O-(SiR₂-O)ₘ₂- bedeutet,
die einzelnen R innerhalb der Polymerkette unabhängig voneinander Alkyl, Alkenyl, Alkinyl, Cycloalkyl, Aryl und/oder Aralkyl bedeuten, die gegebenenfalls substituiert sind, und m2 eine ganze Zahl von 10 bis 6.000 ist, vorzugsweise von 20 bis 2.000.

Organopolysiloxane mit mindestens 2 ethylenisch ungesättigten Gruppen kommen in der Regel als Gemische von Polymeren unterschiedlicher Kettenlänge zum Einsatz. Typische Molekulargewichte (Zahlenmittel) bewegen sich im Bereich von 900 bis 500.000, vorzugsweise von 1.500 bis 150.000.

Beispiele für Alkylgruppen sind geradkettige oder verzweigte Alkylgruppen mit 1 bis 6 Kohlenstoffatomen, wie Methyl, Ethyl, n-Propyl, Isopropyl oder n-Butyl.

Beispiele für substituierte Alkylgruppen sind geradkettige oder verzweigte Alkylgruppen mit 1 bis 6 Kohlenstoffatomen, die mit einem oder mehreren Halogenatomen substituiert sind, z.B. Trifluormethyl.

Beispiele für Cycloalkylgruppen sind Reste mit 5 bis 6 Ringkohlenstoffatomen, wie Cyclohexyl.

Beispiele für Arylgruppen sind ein- der zweikernige aromatische Kohlenwasserstoffreste wie Phenyl oder Naphthyl.

Beispiele für substituierte Arylgruppen sind mit Alkyl- oder Halogen substituierte Phenyle wie Tolyl, Xylyl oder Chlorphenyl.

Ein Beispiel für eine Aralkylgruppe ist Benzyl.

Besonders bevorzugt werden Organopolysiloxane der Formeln I und/oder II, worin R Methyl ist.

Als Organohydrogenpolysiloxane kommen die üblicherweise als Vernetzer eingesetzten Verbindungen zum Einsatz. Dabei kann es sich um Polyalkyl-, Polyaryl-, Polyalkylaryl-, Polyhalogenalkyl-, Polyhalogenaryl- und Polyhalogenalkylaryl-Siloxane handeln, welche im Molekül mindesten 2 an Siliziumatome gebundene Wasserstoffatome aufweisen.

Ein typisches Beispiel sind Verbindungen der Formel RₐH_{b}SiO_{(4-(a+b)/2)}, worin R die oben für die Verbindungen der Formel XIV oder XV definierte Bedeutung besitzt, a eine reelle Zahl mit 1 < a < 2, b eine reelle Zahl mit 0 < b ≤ 1 bedeutet, mit der Maßgabe, dass 1 < a+b < 2,7 ist und dass die Verbindungen mindestens zwei, vorzugsweise mindestens 3 Si-H-Bindungen aufweisen.

Bevorzugte Beispiele für Organohydrogenpolysiloxane sind die Verbindungen der Formel XVIa, XVIb, XVIc, XVId und XVIe

H-SiR₂-O-(SiR₂-O)_{c}-(SiHR-O)_{d}-SiR₂H (XVIa),

R₃Si-O-(SiR₂-O)_{c}-(SiHRO)_{d}-SiR₃ (XVIb),

Rₙ₃Si-(O-SiR₂-H)₄₋ₙ₃ (XVIc),

(SiO_{4/2})_{q}(R₂HSiO_{1/2})ₚ (XVId),

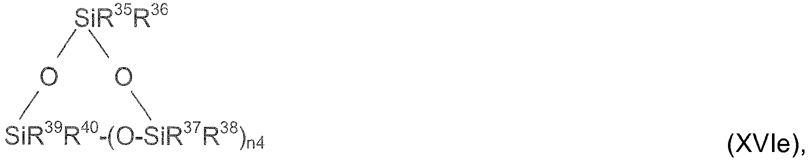

worin R die für Verbindungen der Formeln XIV oder XV definierten Bedeutungen aufweisen, und innerhalb eines Moleküls unterschiedliche Reste R im Rahmen der gegebenen Definition unterschiedliche Bedeutungen annehmen können, insbesondere Alkylreste, vorzugsweise Methyl bedeuten,
R³⁵ bis R⁴⁰ unabhängig voneinander Wasserstoff, Alkyl, Cycloalkyl, Aryl oder Aralkyl bedeuten, wobei mindestens 2 dieser Reste Wasserstoff bedeuten und von den Resten R³⁵ und R³⁶, oder R³⁷ und R³⁸ oder R³⁹ und R⁴⁰ nur jeweils einer Wasserstoff ist,
c, d und e unabhängig voneinander ganze Zahlen von 0 bis 100 sind, wobei die Summe von c, d und e 2 bis 300 beträgt,
n3 eine ganze Zahl von 0 bis 3 ist,
q und p unabhängig voneinander reelle Zahlen größer als 0 sind, mit der Maßgabe, dass die Summe von q und p 1 ist und
n4 eine ganze Zahl von 1 bis 5 ist, vorzugsweise 1 oder 2.

Bevorzugt werden Organohydrogenpolysiloxane eingesetzt, die einen SiH-Gehalt von 0,01 bis 15 mmol/g, vorzugsweise von 0,1 bis 10 mmol/g, aufweisen.

Weitere bevorzugt eingesetzte Organohydrogenpolysiloxane sind Methylhydrogen-Polysiloxane.

Als Katalysatoren für die Hydrosilylierung werden üblicherweise Salze, Komplexe oder kolloidal vorliegende Formen der Übergangsmetalle der 8. Nebengruppe eingesetzt. Vorzugsweise kommen Platin, Palladium oder Rhodium in Form von Metallen oder als Komplexe zum Einsatz. Besonders bevorzugt werden Platinkomplexe eingesetzt, die z.B. aus Hexachloroplatinsäure oder aus Platinsalzen hergestellt worden sind, z.B. Tris(divinyltetramethyldisiloxane)di-platinum(o)-Komplex, Platin-Divinyltetramethyldisiloxan-Komplex.

Besonders bevorzugte Zusammensetzungen enthalten in Komponente A ein Polydialkylsiloxan mit mindestens 2 Vinylgruppen und eine Platinverbindung als Hydrosilylierungskatalysator.

Weitere besonders bevorzugte Zusammensetzungen enthalten in Komponente B das Fluortensid der Gruppen a) bis h) in Kombination mit einem Silizium aufweisenden nichtionischen Tensid und gegebenenfalls in Kombination mit einem weiteren Fluortensid.

Weitere bevorzugte Zusammensetzungen enthalten durch Kondensationsreaktion vernetzende Organopolysiloxane.

Durch Kondensationsreaktion härtbare Organopolysiloxane sind beispielsweise aus DE 41 37 698 A1 bekannt.

Bevorzugt werden Zusammensetzungen, deren Komponente C ein Polydialkylsiloxan mit mindestens 2 Hydroxylgruppen enthält und deren Komponente D ein Polydialkylsiloxan und/oder ein Silan mit mindestens 2 Di- oder Trialkoxysilylgruppen sowie einen Kondensationskatalystor, vorzugsweise eine Zinnverbindung, enthält.

Ebenfalls bevorzugt werden Zusammensetzungen, deren Komponente D das Fluortensid der Gruppen a) bis h) in Kombination mit einem Silizium aufweisenden nichtionischen Tensid und gegebenenfalls in Kombination mit einem weiteren Fluortensid enthält.

Diese kommen üblicherweise in Form einer Mehrkomponenten Dentalabformmasse enthaltend Komponenten C und D zum Einsatz. Darin enthält
d) Komponente C ein Organopolysiloxan mit mindestens 2 Hydroxylgruppen,
e) Komponente D ein Kieselsäureester, Polykieselsäureester und/oder ein Organopolysiloxan mit mindestens 2 Alkoxygruppen sowie einen Kondensationskatalysator, und
f) mindestens eine der Komponenten C und/oder D enthält das Fluortensid der Gruppen a) bis h), ein Silizium aufweisendes nichtionisches Tensid und gegebenenfalls ein weiteres Fluortensid.

Als Organopolysiloxan mit mindestens 2 Hydroxylgruppen kommen üblicherweise Organopolysiloxane zum Einsatz, die mindestens 2 an Si-Atome gebundene Hydroxylgruppen aufweisen.

Typischerweise handelt es sich dabei um Verbindungen der folgenden Formel XVII

HO-SiR₂-B-SiR₂-OH (XVII),

worin B die für die Verbindungen der Formeln XIV und XV definierte Bedeutung aufweist.

Besonders bevorzugt werden Organopolysiloxane mit Hydroxyl-Endgruppen, worin R Methyl ist.

Organopolysiloxane mit mindestens 2 Hydroxylgruppen kommen in der Regel als Gemische von Polymeren unterschiedlicher Kettenlänge zum Einsatz. Typische Molekulargewichte (Zahlenmittel) bewegen sich im Bereich von 900 bis 500.000, vorzugsweise von 1.500 bis 150.000.

Als Silane, Kieselsäureester, Polykieselsäureester und/oder Organopolysiloxane mit mindestens 2 Alkoxygruppen kommen üblicherweise Organopolysiloxane oder Organooxysiliziumverbindungen zum Einsatz, die mindestens 2, vorzugsweise 3 oder 4 an Si-Atome gebundene Alkoxygruppen aufweisen.

Typischerweise handelt es sich dabei um Verbindungen der folgenden Formel XVIII und/oder XIX und/oder XX

(RO)ₘ-SiR₃₋ₘ-B-SiR₃₋ₘ-(OR)ₘ (XVIII),

SiR_{z}(OR')_{4-z} (XIX),

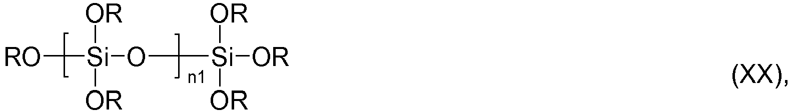

worin B die für Verbindungen der Formel XIV und XV definierte Bedeutung besitzt,
die einzelnen R innerhalb der Polymerkette der Formel XVIII oder XIX oder innerhalb der Verbindung der Formel XX unabhängig voneinander Alkyl, Cycloalkyl, Aryl und/oder Aralkyl bedeuten, die gegebenenfalls substituiert sind,
die einzelnen R' innerhalb der Verbindung der Formel XIX unabhängig voneinander eine der für R definierten Bedeutungen aufweisen, vorzugsweise Alkyl sind,
z eine ganze Zahl von 0 bis 2 ist,
n1 eine ganze Zahl von 1 bis 100 ist, vorzugsweise von 50 bis 70,
und m eine ganze Zahl von 1 bis 3 ist.

Beispiele für Reste R sind bei der Beschreibung der vinyl- bzw. allyl-terminierten Organopolysiloxane der Formeln XIV und XV aufgeführt.

Besonders bevorzugt werden Alkoxysiliziumverbindungen der Formel XIX, worin R und R' Methyl sind und z 0, 1 oder 2 bedeutet.

Organopolysiloxane mit mindestens 2 Alkoxygruppen kommen in der Regel als Gemische von Polymeren unterschiedlicher Kettenlänge zum Einsatz. Typische Molekulargewichte (Zahlenmittel) bewegen sich im Bereich von 400 bis 10.000, vorzugsweise von 250 bis 5.000.

Als Katalysatoren für die Kondensationsreaktion werden üblicherweise Organozinnverbindungen, Titanate, Zirkonate oder Wismuthate eingesetzt, beispielsweise Tetraethyltitanat, Tetraisopropyltitanat, Tetra-n-propyltitanat, Tetra-n-butyltitanat, n-Butyl-polytitanat, Tetra-2-ethylhexyl-titanat, Tetraisooctyltitanat, Octylenglykoltitanat, Tetra-n-propylzirkonat, Tetra-n-butylzirkonat, Zinn-II-isooctoat, Dioctylzinndicarboxylat, Dioctylzinndilaurat, Dibutylzinndilaurat, Organozinncarboxylat, Dibutylzinncarboxylat, Dibutylzinnacetylacetonat, Dibutylzinndiacetat und Wismuth-II-ethylhexanoat.

Weitere bevorzugte Zusammensetzungen enthalten Alkenylreste enthaltende Polyether.

Härtbare Systeme dieses Typs sind beispielsweise aus DE-A-40 10 281 bekannt.

Bevorzugt werden Zusammensetzungen, deren Komponente E ein Polyalkylenether mit mindestens 2 Alkylenresten und eine Platinverbindung als Hydrosilylierungskatalysator enthält.

Ebenfalls bevorzugt werden Zusammensetzungen, deren Komponente F das Fluortensid der Gruppen a) bis h) und gegebenenfalls ein weiteres Fluortensid und/oder gegebenenfalls ein Silizium aufweisendes nichtionisches Tensid enthält.

Diese kommen üblicherweise in Form einer Mehrkomponenten Dentalabformmasse enthaltend Komponenten E und F zum Einsatz. Darin enthält
g) Komponente E einen Vernetzungskatalysator,
h) Komponente F einen vernetzbaren und Alkenylreste aufweisenden Polyether sowie ein Organohydrogenpolysiloxan und/oder SiH-Polyether, und
i) mindestens eine der Komponenten E und/oder F enthält das Fluortensid der Gruppen a) bis h), ein Silizium aufweisendes nichtionisches Tensid und gegebenenfalls ein weiteres Fluortensid.

Als Alkenylreste aufweisende Polyether kommen üblicherweise von Polyalkylenglykolen abgeleitete und ethylenisch ungesättigten Gruppen, z.B. mit Allyletherresten terminierte Polymere in Frage.

Typischerweise handelt es sich dabei um Verbindungen der folgenden Formel XXI

X-A-(X)ₙ₁ (XXI),

worin A und n1 die für Verbindungen der Formel XI definierte Bedeutung besitzen und X eine Alkenylgruppe mit endständiger Doppelbindung ist.

Beispiele für Alkenylgruppen mit endständiger Doppelbindung X sind -CH₂-CH=CH₂, -SiR₂-CH=CH₂, -CR₂-CH=CH₂ und -C₆H₄-CH=CH₂, worin R Alkylgruppen bedeuten.

Besonders bevorzugt werden Polyether der Formel XXI, die Ethylenoxid- und/oder Propylenoxideinheiten aufweisen, insbesondere solche mit endständigen Allylgruppen.

Alkenylreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3.000.000, vorzugsweise von 250 bis 100.000.

Als Organohydrogenpolysiloxane kommen die bereits weiter oben bei der Beschreibung der durch Addionsreaktion vernetzenden Organopolysiloxane beschriebenen Verbindungen in Frage.

Auch in diesem vernetzbaren Polymersystem werden bevorzugt Organohydrogenpolysiloxane eingesetzt, die einen SiH-Gehalt von 0,01 bis 15 mmol/g, vorzugsweise von 0,1 bis 10 mmol/g, aufweisen.

Weitere bevorzugt eingesetzte Organohydrogenpolysiloxane sind Methylhydrogen-Polysiloxane.

Als SiH-Polyether kommen Verbindungen der Formel XXII in Frage

Y-A-(Y)ₙ₁ (XXII),

worin A und n1 die für Verbindungen der Formel XI definierte Bedeutung besitzen und Y eine Gruppe enthaltend einen Siliziumwasserstoffrest ist.

Beispiele für Reste Y sind Gruppen der Formel -R'-SiR₂H oder der Formel XVIf 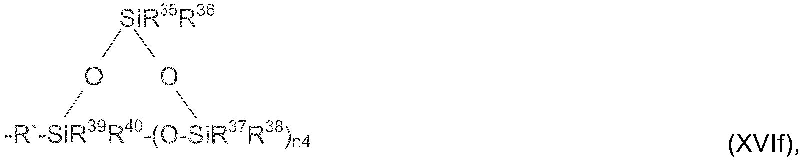
worin R' für einen Alkylenrest, vorzugsweise mit 1 bis 6 Kohlenstoffatomen, steht,
R die für Verbindungen der Formeln XIV oder XV definierten Bedeutungen aufweist, und innerhalb eines Moleküls unterschiedliche Reste R im Rahmen der gegebenen Definition unterschiedliche Bedeutungen annehmen können, insbesondere Alkylreste, vorzugsweise Methyl bedeuten,
und R³⁵ bis R⁴⁰ sowie n4 die weiter oben für Reste der Formel XVIe definierte Bedeutung besitzt.

Als Katalysatoren werden auch in diesem härtbaren System die weiter oben beschriebenen Salze, Komplexe oder kolloidal vorliegende Formen der Übergangsmetalle der 8. Nebengruppe eingesetzt. Vorzugsweise kommen Platin, Palladium oder Rhodium in Form von Metallen oder als Komplexe zum Einsatz.

Besonders bevorzugt werden Platinkomplexe eingesetzt, die z.B. aus Hexachloroplatinsäure oder aus Platinsalzen hergestellt worden sind, z.B. Tris(divinyltetra-methyldisiloxan)di-platin(o)-komplex, Platin-Divinyltetramethyldisiloxan-Komplex.

Weitere bevorzugte Zusammensetzungen enthalten Alkoxysilylreste enthaltende Polyether.

Härtbare Systeme dieses Typs sind beispielsweise aus PCT/EP2005/001470 und aus EP-A-1 226 808 bekannt.

Bevorzugt werden Zusammensetzungen, die einen Polyalkylenether mit mindestens 2 Alkoxysilylresten und eine Zinnverbindung und/oder organische Säuren und/oder Basen und/oder deren Salze als Kondensationskatalysator enthalten.

Härtbare Systeme dieses Typs kommen bevorzugt in Form einer Mehrkomponenten Dentalabformmasse enthaltend Komponenten G und H zum Einsatz. Darin enthält
j) Komponente G einen vernetzbaren und Alkoxysilylreste aufweisenden Polyether,
k) Komponente H enthält Wasser,
I) mindestens eine der Komponenten G und/oder H enthalten einen Katalysator sowie das Fluortensid der Gruppen a) bis h), ein Silizium aufweisendes nichtionisches Tensid und gegebenenfalls ein weiteres Fluortensid.

Als Alkoxysilylreste aufweisende Polyether kommen üblicherweise von Polyalkylenglykolen abgeleitete und mit Alkoxysilylresten, gegebenenfalls über eine Brückengruppe, terminierte Polymere in Frage.

Typischerweise handelt es sich dabei um Verbindungen der Formel XXIII

(RO)₃₋ₙ₅Rₙ₅Si-BG2-A-(BG2-SiRₙ₅(OR)₃₋ₙ₅)ₙ₁ (XXIII),

worin A und n1 die für Verbindungen der Formel XI definierte Bedeutung besitzen,
BG2 für eine kovalente Bindung oder eine Brückengruppe ausgenommen -O-steht,
n5 eine ganze Zahl von 0 bis 2 bedeutet,
R die für Verbindungen der Formeln XIV oder XV definierten Bedeutungen aufweist, und innerhalb eines Moleküls unterschiedliche Reste R sowie unterschiedliche Indizes n5 im Rahmen der gegebenen Definition unterschiedliche Bedeutungen annehmen können, R insbesondere Alkylreste, vorzugsweise Methyl bedeuten, und BG2 insbesondere eine kovalente Bindung oder eine Brückengruppe -O-CO-NH-R'- ist, worin R' mit dem Siliziumatom verbunden ist und eine Alkylgruppe, vorzugsweise Methyl, Ethyl oder Propyl, bedeutet.

Alkoxysilylreste aufweisende Polyether können auch als Gemische von Polymeren unterschiedlicher Kettenlänge zum Einsatz gelangen. Typische Molekulargewichte (Zahlenmittel) bewegen sich im Bereich von 150 bis 3.000.000, vorzugsweise von 250 bis 100.000 und besonders bevorzugt 250 bis 50.000.

Als Katalysatoren können in diesem härtbaren System die weiter oben beschriebenen Organozinnverbindungen eingesetzt werden, beispielsweise Dibutylzinndilaurat. Es können aber auch organische Säuren, wie Toluolsulfonsäure, oder organische Basen, wie Amine, Guanidine, DBU oder DBN, oder Salze dieser Säuren oder Basen eingesetzt werden.

Weitere bevorzugte Zusammensetzungen enthalten Aziridinoreste enthaltende Polyether.

Härtbare Systeme dieses Typs sind beispielsweise aus US-A-4,353,242 bekannt.

Bevorzugte Zusammensetzungen enthalten vernetzbare Polyether, die Alkoxysilylreste, Aziridinoreste, von einer ethylenisch ungesättigten Carbonsäure abgeleitete Reste oder Alkenylreste als vernetzbare Gruppen oder über ringöffnende Metathesereaktion vernetzbare Gruppen aufweisen, sowie mindestens ein oben definiertes Fluortensid der Gruppen a) bis h) gegebenenfalls in Kombination mit einem weiteren Fluortensid und/oder gegebenenfalls in Kombination mit einem Silizium aufweisendem nichtionischem Tensid.

Besonders bevorzugte Zusammensetzungen dieses Typs enthalten vernetzbare Polyether, die Alkoxysilylreste oder Aziridinoreste als vernetzbare Gruppen aufweisen.

Auch bei diesen Zusammensetzungen kommen als zusätzliche Komponenten vorzugsweise Polyole und/oder Alkenylreste und/oder Alkinylreste enthaltende Polyether und/oder hydroxyl- und/oder alkoxy-terminierte Polyether zum Einsatz.

Vorzugsweise werden in diesen Zusammensetzungen jedoch Gemische von Silizium aufweisenden nichtionischen Tensiden und Fluortensiden der Gruppen a) bis h) gegebenenfalls in Kombination mit den oben erwähnten zusätzlichen Polyol- und/oder Polyetherkomponenten eingesetzt.

Härtbare Systeme dieses Typs kommen üblicherweise in Form einer Mehrkomponenten-Dentalabformmasse enthaltend Komponenten I und J zum Einsatz. Darin enthält
m) Komponente I einen vernetzbaren und Aziridinoreste oder Alkoxysilylreste aufweisenden Polyether oder einen über ringöffnende Metathesereaktion vernetzbare Gruppen enthaltenden Polyether,
n) Komponente J einen Katalysator, und
o) mindestens eine der Komponenten I und/oder J enthält ein Fluortensid der Gruppen a) bis h), ein Silizium aufweisendes nichtionisches Tensid und gegebenenfalls ein weiteres Fluortensid.

Als Aziridinoreste oder Alkoxysilylreste aufweisende Polyether kommen üblicherweise von Polyalkylenglykolen abgeleitete und mit Alkoxysilylresten oder mit Aziridinoresten über eine Brückengruppe terminierte Polymere in Frage.

Typischerweise handelt es sich dabei um Verbindungen der folgenden Formel XXVIII 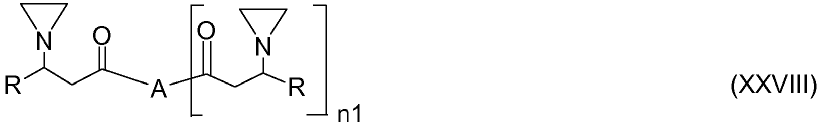
worin A und n1 die für Verbindungen der Formel XI definierte Bedeutung besitzen,
R die für Verbindungen der Formeln XIV oder XV definierten Bedeutungen aufweist, und innerhalb eines Moleküls unterschiedliche Reste R im Rahmen der gegebenen Definition unterschiedliche Bedeutungen annehmen können, und R insbesondere Alkylreste, vorzugsweise Methyl bedeuten.

Aziridinoreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3.000.000, vorzugsweise von 250 bis 100.000 und besonders bevorzugt 250 bis 50.000.

Als Katalysatoren können in diesem härtbaren System Sulfoniumsalze eingesetzt werden.

Weitere bevorzugte Zusammensetzungen enthalten Esterreste einer ethylenisch ungesättigten Carbonsäure enthaltende Polyether.

Härtbare Systeme dieses Typs sind beispielsweise aus EP 0 170 219 A2 bekannt.

Bevorzugt werden Zusammensetzungen die einen vernetzbaren Polyalkylenether, der von Acrylsäure und/oder Methacrylsäure abgeleitete Reste aufweisen, einen durch Hitze oder durch Strahlung aktivierbaren Initiator sowie die das Fluortensid der Gruppen a) bis h) und gegebenenfalls ein weiteres Fluortensid und/oder gegebenenfalls ein Silizium aufweisendes nichtionisches Tensid enthalten.

Diese Zusammensetzungen können als Einkomponenten- oder als Zweikomponenten-Formulierungen eingesetzt werden.

Bevorzugt kommen Einkomponenten-Dentalabformmassen zum Einsatz, die durch UV-Strahlung und/oder Hitze ausgehärtet werden. Neben dem härtbaren Polymersystem sowie dem erfindungsgemäß eingesetzten Tensid sind darin üblicherweise Photoinitiatoren enthalten.

Als vernetzbare Polyether kommen üblicherweise von Polyalkylenglykolen abgeleitete und mit ethylenisch ungesättigten Carbonsäuren terminierte Polymere in Frage.

Typischerweise handelt es sich dabei um Verbindungen der Formeln XXIV und/oder XXVIII

R⁵⁰-A-(CO-R⁵⁰)ₙ₁ (XXIV),

R⁵⁰-CO-A-(CO-R⁵⁰)ₙ₁ (XXVIII),

worin A und n1 die für Verbindungen der Formel XI definierte Bedeutung besitzen,
R⁵⁰ ein ethylenisch ungesättigter Rest, vorzugsweise ein Alkenylrest ist und die Reste R⁵⁰ innerhalb eines Moleküls im Rahmen der gegebenen Bedeutung unterschiedlich sein können.

Ganz besonders bevorzugt ist R⁵⁰ CH₂=CH- oder CH₂=C(CH₃)-, also ein von Acrylsäure oder Methacrylsäure abgeleiteter Rest.

Ethylenisch ungesättigte Carbonsäureesterreste aufweisende Polyether kommen in der Regel als Gemische von Polymeren unterschiedlicher Kettenlänge zum Einsatz. Typische Molekulargewichte (Zahlenmittel) bewegen sich im Bereich von 150 bis 3.000.000, vorzugsweise von 250 bis 100.000 und besonders bevorzugt 250 bis 50.000.

Diese Polyether werden üblicherweise durch elektromagnetische Strahlung, vorzugsweise UV-Strahlung oder sichtbares Licht gehärtet. Als Photoinitiatoren können in diesem härtbaren System z.B. Campherchinon und/oder Amine eingesetzt werden.

In hitzehärtbaren Systemen werden vorzugsweise Peroxidhärter gegebenenfalls kombiniert mit Aminen eingesetzt.

Weitere bevorzugte Zusammensetzungen enthalten über ringöffnende Metathese Polymerisation (ROMP) vernetzbare Gruppen aufweisende Polyether, Polysiloxane und/oder Synthesekautschuke. Härtbare Systeme dieses Typs sind bekannt, z.B. aus EP 1 317 917 A1, US 6,649,146 B2, WO 02/32338 A2 und US 6,455,029.

Diese kommen üblicherweise in Form einer Mehrkomponenten Dentalabformmasse enthaltend Komponenten K und L zum Einsatz. Darin enthält
p) Komponente K Polyether, Polysiloxane und/oder Synthesekautschuke, die über ROMP vernetzbare Gruppen aufweisen,
q) Komponente L einen ROMP Vernetzungskatalysator, und
r) mindestens eine der Komponenten K und/oder L enthält das Fluortensid der Gruppen a) bis h) und gegebenenfalls ein weiteres Fluortensid und/oder gegebenenfalls ein Silizium aufweisendes nichtionisches Tensid.

Als über ROMP vernetzbare Gruppen aufweisende Polyether, Polysiloxane und/oder Synthesekautschuke kommen üblicherweise von Polyalkylenglykolen, Polydialkyl- oder -arylsiloxanen und/oder Polyalkenen oder Polyalkandienen abgeleitete, mit ungesättigten end- und/oder seitenständigen über einen Spacer B gebundenen Resten MT versehene Polymere in Frage.

Bei den Polysiloxanen handelt es sich dabei typischerweise um Verbindungen der folgenden Formel XXV

MT-BG-(SiR₂O)ₘ₅-(SiR(MT)O)ₙ₆-BG-MT (XXV),

worin R unabhängig voneinander Alkyl, Cycloalkyl, Aryl und/oder Aralkyl bedeutet, die gegebenenfalls substituiert sind,
m5 eine ganze Zahl von 10 bis 6.000, vorzugsweise von 20 bis 2.000,
n6 eine ganze Zahl von 0 bis 100, vorzugsweise von 0 bis 10 ist,
BG eine Brückengruppe bedeutet,
MT eine über ROMP vernetzbare Gruppe ist, und
die Reste MT, B2 und R sowie die Indizes m5 und/oder n6 innerhalb eines Moleküls im Rahmen der gegebenen Bedeutung unterschiedlich sein können.

Typische Molekulargewichte (Zahlenmittel) von Verbindungen der Formel XXV bewegen sich im Bereich von 900 bis 500.000, vorzugsweise von 1500 bis 150.000.

Organopolysiloxane mit mindestens 2 ethylenisch ungesättigten Gruppen kommen in der Regel als Gemische von Polymeren unterschiedlicher Kettenlänge zum Einsatz. Typische Molekulargewichte (Zahlenmittel) bewegen sich im Bereich von 900 bis 500.000, vorzugsweise von 1500 bis 150.000.

Besonders bevorzugt werden Organopolysiloxane der Formeln XXV, worin R Methyl ist.

Bei den Polyalkylenglycolen handelt es sich dabei typischerweise um Verbindungen der folgenden Formeln XXVI und/oder XXIX

MT-BG3-(Cₙ₆H₂ₙ₆O)ₘ₆-(Cₙ₆H₂ₙ₆₋₁(BG3-MT)O)ₘ₇-BG3-MT (XXVI),

MT-BG3-A-(BG3-MT))ₙ₁ (XXIX),

worin A und n1 die bei Verbindungen der Formel XI definierte Bedeutung besitzen,
n6 eine ganze Zahl von 4 bis 8, vorzugsweise von 2 bis 4 bedeutet,
m6 eine ganze Zahl von 2 bis 70.000, vorzugsweise von 10 bis 2.500 ist,
m7 eine ganze Zahl von 0 bis 70.000, vorzugsweise von 10 bis 2.500 ist,
die Summe von m6 und m7 3 bis 70.000, vorzugsweise 10 bis 2.500 ist,
BG3 eine Brückengruppe bedeutet,
MT eine über ROMP vernetzbare Gruppe ist, und
die Reste MT und BG3 sowie die Indizes n6, m6 und/oder m7 innerhalb eines Moleküls im Rahmen der gegebenen Bedeutung unterschiedlich sein können.

Bei den Synthesekautschuken handelt es sich dabei typischerweise um Verbindungen der folgenden Formel XXVII 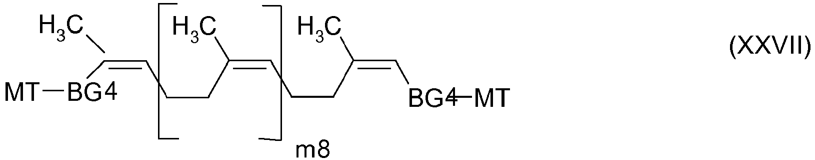
worin BG4 eine Brückengruppe bedeutet,
MT eine über ROMP vernetzbare Gruppe ist, und
m8 eine ganze Zahl von 1.500 bis 30.000, vorzugsweise 20 bis 500, ist.

Neben 1,4 cis und 1,4 trans Polyisopren und deren Mischformen sind Polymere von 1,3-Dienen, wie z.B. 2,3-Dimethyl-1,3-butadien, Polybutadien und Poly-(2-chlor-1,3-butadien) sowie Synthesekautschuke, die durch Copolymerisation zweier oder dreier verschiedener Monomere entstanden. Die wichtigsten synthetischen Kautschuke sind Styrol-Butadien-Kautschuk, Acrylnitril-Butadien-Kautschuk und Isobuten-Isopren-Kautschuk, Ethylen-Propylen-Copolymere (EPM), Ethylen-Propylen-Dien-Terpolymere (EPDM), ferner Elastomere auf der Basis von Polyurethan, Polysulfiden, Chlorsulfonylpolyethylen, wobei der Synthesekautschuk durch Vulkanisation quervernetzt vorliegen kann.

Die ungesättigten ROMP-vernetzbaren Gruppen MT sind z.B. Cycloalkenylgruppen, wie z.B. Cyclobutenyl-, Cyclopentenyl- oder Gruppen der allgemeinen Formel 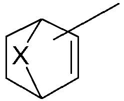 worin X = O, S, NH oder ein gesättigter oder ungesättigter C₁-C₃₀-Kohlenwasserstoffrest ist.

Bei der Brückengruppe BG4 in den oben aufgezählten Formeln handelt es sich vorzugsweise um -O- oder Alkylen, insbesondere um -CH₂-, -CH₂CH₂-, -CH₂CH₂CH₂- oder um -CH(CH₃)-CH₂-.

Als Katalysatoren werden in diesem härtbaren System Salze, Komplexe oder kolloidal vorliegende Formen der Übergangsmetalle der 8. Nebengruppe eingesetzt. Vorzugsweise kommen Ruthenium, Osmium, Wolfram oder Molybdän als Komplexe zum Einsatz. Besonders bevorzugt werden Ruthenium-Carben-Komplexe eingesetzt, wie z.B. der Grubbs-Katalysator.

Die erfindungsgemäßen Zusammensetzungen enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzenden Esterreste einer ethylenisch ungesättigten Carbonsäure enthaltenden Polyether oder der durch ringöffnende Metathesereaktion vernetzenden Polyether, Silicone oder Kautschuke, enthalten vorzugsweise als weitere Komponente einen Alkenylreste enthaltenden Polyether und/oder einen hydroxyl- oder alkoxy-terminierten Polyether. Diese zusätzliche Komponente bewirkt eine weitere Verbesserung der Kontaktwinkeleigenschaften des nicht ausgehärteten plastischen Materials und somit eine weitere Verbesserung der Detailgenauigkeit des entstehenden Abdrucks.

Beispiele für bevorzugte Zusätze dieses Typs sind die weiter oben beschriebenen Verbindungen der Formel XI, XII und XIII.

Die Zusammensetzungen können neben den vernetzbaren Polymeren und dem Fluortensid der Gruppen a) bis h) oder der Tensidmischung weitere Komponenten enthalten, die üblicherweise in solchen Massen eingesetzt werden.

Beispiele für solche weiteren Komponenten sind Füllstoffe, Dabei kann es sich um verstärkende Füllstoffe oder um nicht verstärkende Füllstoffe oder um Gemische davon handeln.

Als verstärkende Füllstoffe eignen sich insbesondere hochdisperse, aktive Füllstoffe mit einer BET-Oberfläche von wenigstens 50 m²/g. Besonders geeignet sind solche mit einer Einzelpartikelgröße im Nanometerbereich, welche als Aggregate und/oder Agglomerate vorliegen können. Bevorzugte verstärkende Füllstoffe sind Substanzen, die ausgewählt sind aus der Gruppe bestehend aus Aluminiumhydroxid, Zinkoxid, Titandioxid, Zirkoniumoxid, Siliziumdioxid sowie gefällter und/oder pyrogener Kieselsäure. Selbstverständlich können die zuvor genannten Verbindungen einzeln oder in beliebiger Kombination miteinander eingesetzt werden, und zwar auch sowohl in hydrophiler als auch in hydrophobierter Form,

Ferner bevorzugt liegt der wenigstens eine verstärkende Füllstoff in Form von Nanopartikeln, als faser- oder blättchenförmiger Füllstoff, beispielsweise als mineralischer, faserförmiger Füllstoff, oder als synthetischer, faserförmiger Füllstoff vor.

Der Anteil an verstärkendem Füllstoff in der erfindungsgemäßen Zusammensetzung beträgt üblicherweise 0 bis 80 Gew.-%, bevorzugt 0,1 bis 50 Gew.-% und besonders bevorzugt 0,1 bis 40 Gew,-%, bezogen auf die gesamte Zusammensetzung.

Als nicht verstärkende Füllstoffe eignen sich prinzipiell dieselben Substanzen wie für die verstärkende Füllstoffe, wobei die nicht verstärkenden jedoch zwingend eine BET-Oberfläche von weniger als 50 m²/g (Schriftenreihe Pigmente Degussa Kieselsäuren, Nummer 12, Seite 5 sowie Nummer 13, Seite 3) aufweisen. Bevorzugte nicht verstärkende Füllstoffe sind Substanzen, die ausgewählt werden aus der Gruppe bestehend aus Erdalkalimetalloxiden, Erdalkalimetallhydroxiden, Erdalkalimetallfluoriden, Erdalkalimetallcarbonaten, Kalziumapatit (Ca₅[(F, Cl, OH, ½CO₃) | (PO₄)₃], insbesondere Kalziumhydroxylapatit (Ca₅[(OH) | (PO₄)₃], Titandioxid, Zirkoniumoxid, Aluminiumhydroxid, Siliziumdioxid, gefällter Kieselsäure und Kalziumcarbonat. Selbstverständlich können die zuvor genannten Verbindungen einzeln oder in beliebiger Kombination miteinander eingesetzt werden, und zwar auch sowohl in hydrophiler als auch in hydrophobierter Form.

Vorzugsweise weisen die eingesetzten nicht verstärkenden Füllstoffe eine mittlere Korngröße von größer als 0,1 µm (Ullmann Encyclopädie der Technischen Chemie, Band 21, Seite 523) auf.

Der Anteil an nicht verstärkendem Füllstoff in der erfindungsgemäßen Zusammensetzung beträgt üblicherweise 0 bis 80 Gew.-%, bevorzugt 0,1 bis 50 Gew.-% und besonders bevorzugt 0,1 bis 40 Gew.-%, bezogen auf die gesamte Zusammensetzung.

Der Gesamtanteil an verstärkenden und nicht verstärkenden Füllstoffen in der erfindungsgemäßen Zusammensetzung beträgt üblicherweise 0 bis 80 Gew.-%, bevorzugt 0,01 bis 80 Gew.-%, besonders bevorzugt 0,05 bis 75 Gew.-%, und ganz besonders bevorzugt 0,1 bis 70 Gew.-%, bezogen auf die gesamte Zusammensetzung.

Die erfindungsgemäßen Zusammensetzungen können darüber hinaus ein oder mehrere der folgenden Zusatzstoffe enthalten: Puffersalze, Wasserfänger, Pastenbildner, weitere Tenside, Wirkstoffe, Weichmacher, optische Abtastung ermöglichende Substanzen, Geschmacks- und/oder Geruchsstoffe, Diagnostik ermöglichende Substanzen, Fluoridisierungsmittel, Bleichsubstanzen, Desensibilisierungsmittel, Haftverbundvermittler, Farbstoffe, Indikatoren, Stabilisatoren (Antioxidantien) sowie antibakterielle Substanzen.

Liegt die erfindungsgemäße Zusammensetzung als Mehrkomponentensystem vor, so wird diese vorzugsweise in geeigneten Primärverpackungen, wie Tuben, Dosen, und besonders bevorzugt in Kartuschen und Schlauchbeuteln, wie sie z.B. in der EP-A-0 723 807, der EP-A-0 541 972, der WO 98/44860 A1, der EP-A-0 492 412, der EP-A-0 492 413 und der EP-A-0 956 908 beschrieben sind, gelagert und auf die spätere Verwendung zugeschnitten proportioniert ist.

Ganz besonders bevorzugt werden Zusammensetzungen enthaltend
a) 25 bis 85 Gew.-% Organopolydialkylsiloxan mit mindestens 2 Alkenylgruppen,
b) 1 bis 70 Gew.-% Organohydrogenpolysiloxan mit einem SiH-Gehalt von 0,1 bis 15,0 mmol/g,
c) 0,0001 bis 2 Gew.-% Hydrosilylierungskatalysator, insbesondere Salze, Komplexe und kolloidal vorliegende Formen der Übergangsmetalle der 8. Nebengruppe,
d) 0 bis 90 Gew.-% nicht verstärkende Füllstoffe mit einer BET-Oberfläche kleiner 50 m²/g,
e) 0,1 bis 50 Gew.-% verstärkende Füllstoffe mit einer BET-Oberfläche größer gleich 50 m²/g,
f) 0 bis 20 Gew.-% Hilfsstoffe und Additive, wie Weichmacher, Farbstoffe, Stabilisatoren, Inhibitoren, Alkyl-verschlossene Fettalkoholethoxylate, usw.
g) 0,01 bis 5,0 Gew.-% nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten und
h) 0,001 bis 5,0 Gew.-% des oben beschriebenen Fluortensids ausgewählt aus den Gruppen a) bis h), wobei das Verhältnis der Tenside g) und h) bevorzugt 100 : 1 bis 1 : 100, besonders bevorzugt 50 : 1 bis 1 : 50, ganz besonders bevorzugt 10 : 1 bis 1 : 10 und insbesondere bevorzugt 5 : 1 bis 1 : 5 ist.

Besonders bevorzugt werden Zweikomponenten-Zusammensetzungen enthaltend die vorstehend definierten Komponenten a) bis h) in den angegebenen Mengen und zusätzlich i) 0,1 bis 25 Gew.-%, bevorzugt 0,1 bis 10,0 Gew.-%, besonders bevorzugt 0,5 bis 5,0 Gew.-%, und ganz besonders bevorzugt 0,5 bis 2,5 Gew.-% verzweigten oder linearen Alkyl-, Hydroxy-, Alkinyl-, und/oder Alkenyl-terminierten Polyalkylenether und/oder deren Mischungen, wobei das Gewichtsverhältnis von Organopolysiloxan a) zum Polyether i) bevorzugt 1 : 1 bis 80 : 1, besonders bevorzugt 1 : 1 bis 60 : 1, ganz besonders bevorzugt 1 : 1 bis 40 : 1 und insbesondere bevorzugt 1 : 1 bis 30 : 1 ist.

Besonders bevorzugt werden Zweikomponenten-Zusammensetzungen enthaltend die vorstehend definierten Komponenten a) bis h) in den angegebenen Mengen und zusätzlich k) 0,1 bis 25 Gew.-%, bevorzugt 0,1 bis 10,0 Gew.-%, besonders bevorzugt 0,5 bis 5,0 Gew.-%, und ganz besonders bevorzugt 0,5 bis 2,5 Gew.-% eines Polyols oder einer Mischung von Polyolen.

Ganz besonders bevorzugt werden Zweikomponenten-Zusammensetzungen enthaltend die vorstehend definierten Komponenten a) bis h) in den angegebenen Mengen und zusätzlich die vorstehend definierten Komponenten i) und k) in den angegebenen Mengen.

Ganz besonders bevorzugt werden Zweikomponenten-Zusammensetzungen bestehend aus Komponente A und B, worin Komponente A enthält
a) 10 bis 80 Gew.-% Organopolydialkylsiloxan mit mindestens 2 Alkenylgruppen,
c) 0,0001 bis 2 Gew.-% Hydrosilylierungskatalysator, insbesondere Salze, Komplexe und kolloidal vorliegende Formen der Übergangsmetalle der 8. Nebengruppe,
d) 0 bis 90 Gew.-% nicht verstärkende Füllstoffe mit einer BET-Oberfläche kleiner 50 m²/kg,
e) 0,1 bis 50 Gew.-% verstärkende Füllstoffe mit einer BET-Oberfläche größer gleich 50 m²/kg,
f) 0 bis 20 Gew.-% Hilfsstoffe und Additive, wie Weichmacher, Farbstoffe, Stabilisatoren und/oder Inhibitoren, und
j) 0,001 bis 5,0 Gew.-% Alkyl-, Aryl-, Aralkyl-verschlossene nichtionische Tenside, bevorzugt Alkyl-verschlossene Fettalkoholethoxylate, Silicontenside, Polyethercarbosilane, Carbosilantenside und Fluortenside, die Alkyl-verschlossen sind und insbesondere Alkyl-verschlossene Fettalkoholethoxylate,
und Komponente B enthält
a) 0,1 bis 70 Gew.-% Organopolydialkylsiloxan mit mindestens 2 Alkenylgruppen,
b) 1,2 bis 80 Gew.-% Organohydrogenpolysiloxan mit einem SiH-Gehalt von 0,1 bis 15,0 mmol/kg,
d) 0 bis 90 Gew.-% nicht verstärkende Füllstoffe mit einer BET-Oberfläche kleiner 50 m²/kg,
e) 0,1 bis 50 Gew.-% verstärkende Füllstoffe mit einer BET-Oberfläche größer gleich 50 m²/kg,
f) 0 bis 20 Gew.-% Hilfsstoffe und Additive, wie Weichmacher, Farbstoffe, Stabilisatoren und/oder Inhibitoren,
g) 0,01 bis 10,0 Gew.-% nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten,
h) 0,001 bis 10,0 Gew.-% der oben beschriebenen Fluortenside ausgewählt aus der Gruppe a) bis h), wobei das Verhältnis der Tenside g) und h) bevorzugt 25 : 1 bis 1 : 25, besonders bevorzugt 20 : 1 bis 1 : 5, ganz besonders bevorzugt 10 : 1 bis 1 : 5 und insbesondere bevorzugt 5 : 1 bis 1 : 3 ist, und
i) 0,5 bis 50 Gew.-% verzweigten oder linearen Alkyl- und/oder Alkinyl- und/oder Alkenyl- und/oder Hydroxy-terminierten Polyalkylenether und/oder deren Mischungen, wobei das Gewichtsverhältnis von Organopolysiloxan a) zum Polyether i) 1 : 50 bis 50 : 1, bevorzugt 10 : 1 bis 1 : 10 und insbesondere bevorzugt 4 : 1 bis 1 : 5 ist.

Ebenfalls ganz besonders bevorzugt werden Zweikomponenten-Zusammensetzungen bestehend aus Komponente A und B, worin Komponente A enthält
a) 10 bis 80 Gew.-% Organopolydialkylsiloxan mit mindestens 2 Alkenylgruppen,
c) 0,0001 bis 2 Gew.-% Hydrosilylierungskatalysator, insbesondere Salze, Komplexe und kolloidal vorliegende Formen der Übergangsmetalle der 8. Nebengruppe,
d) 0 bis 90 Gew.-% nicht verstärkende Füllstoffe mit einer BET-Oberfläche kleiner 50 m²/kg,
e) 0,1 bis 50 Gew.-% verstärkende Füllstoffe mit einer BET-Oberfläche größer gleich 50 m²/kg,
f) 0 bis 20 Gew.-% Hilfsstoffe und Additive, wie Weichmacher, Farbstoffe, Stabilisatoren und/oder Inhibitoren, und
j) 0,001 bis 5,0 Gew.-% Alkyl-, Aryl-, Aralkyl-verschlossene nichtionische Tenside, bevorzugt Alkyl-verschlossene Fettalkoholethoxylate, Silicontenside, Polyethercarbosilane, Carbosilantenside und Fluortenside, die Alkyl-verschlossen sind und insbesondere Alkyl-verschlossene Fettalkoholethoxylate,
und Komponente B enthält,
a) 0,1 bis 70 Gew.-% Organopolydialkylsiloxan mit mindestens 2 Alkenylgruppen,
b) 1,2 bis 80 Gew.-% Organohydrogenpolysiloxan mit einem SiH-Gehalt von 0,1 bis 15,0 mmol/kg,
d) 0 bis 90 Gew.-% nicht verstärkende Füllstoffe mit einer BET-Oberfläche kleiner 50 m²/kg,
e) 0,1 bis 50 Gew.-% verstärkende Füllstoffe mit einer BET-Oberfläche größer gleich 50 m²/kg,
f) 0 bis 20 Gew.-% Hilfsstoffe und Additive, wie Weichmacher, Farbstoffe, Stabilisatoren und/oder Inhibitoren,
g) 0,01 bis 10,0 Gew.-% nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten,
h) 0,001 bis 10,0 Gew.-% der oben beschriebenen Fluortenside ausgewählt aus der Gruppe a) bis h), wobei das Verhältnis der Tenside g) und h) 25 : 1 bis 1 : 25, bevorzugt 20 : 1 bis 1 : 5, besonders bevorzugt 10 : 1 bis 1 : 5 und insbesondere bevorzugt 5 : 1 bis 1 : 3 ist, und
k) 0,5 bis 50 Gew.-% Polyol und/oder Mischungen von Polyolen.

Weitere ganz besonders bevorzugte Zweikomponenten-Zusammensetzungen enthaltend die vorstehend definierten Komponenten a) bis h) in den angegebenen Mengen und zusätzlich die vorstehend definierten Komponenten i) und k) in den angegebenen Mengen.

Die Mengenangaben in der vorstehenden Ausführungsform beziehen sich dabei jeweils auf die Gesamtmasse der Komponente A oder der Komponente B.

Ein weiterer Gegenstand der vorliegenden Erfindung sind Mischungen, welche durch Vermischen einzelner Komponenten von Mehrkomponentenmassen der zuvor beschriebenen härtbaren Zusammensetzung erhältlich sind. Vorzugsweise wird eine Basiskomponente mit einer Katalysatorkomponente in einem Verhältnis von 1 : 2 bis 20 : 1, besonders bevorzugt von 1 : 1 bis 10 : 1 und ganz besonders bevorzugt von 10 : 1, 5 : 1, 4 : 1, 2 : 1 und 1 : 1, vermischt. Diese Mischungen zeichnen sich durch eine ausgezeichnete Benetzbarkeit und ein hervorragendes Anfließverhalten an feuchte Zahn- und Gewebesubstanz aus. Trotz dieser guten hydrophilen Eigenschaften quillt das Material nicht bei Kontakt mit wässrigen Medien wie Wasser, Speichel, Blut, Desinfektionsbad oder wässrigem Gipsbrei. Die gute initiale Benetzbarkeit der Mischungen ist wichtig für die detailgetreue Abformung des Abformmaterials im Patientenmund während der Verarbeitung und dem ersten Kontakt mit feuchter Mund-/Zahnsubstanz und drückt sich durch einen äußerst niedrigen Kontaktwinkel aus, der sich rasch nach dem Vermischen der Komponenten bzw. nach dem Beginn der Härtung ausbildet. Die erfindungsgemäßen Zusammensetzungen zeichnen sich dadurch aus, dass zwischen 30 Sekunden nach Mischbeginn der Komponenten des härtbaren Polymersystems, z.B. der Katalysatorkomponente und der Basiskomponente, oder des Auslösens der Härtung durch Bestrahlung bis zum Abbinden des härtbaren Polymersystems bei einem Wassertropfenalter in den ersten 10 Sekunden, bevorzugt in den ersten 5 Sekunden nach Aufsetzen des Wassertropfens auf die Oberfläche des Dentalabformmaterials, Kontaktwinkel von ≤ 10° erreicht werden. Die Kontaktwinkel werden dabei mit einem Kontaktwinkelmessgerät G40/G23M der Firma Krüss bei 23°C ± 1°C mit der Messmethode "liegender Tropfen" gemessen. Die Messung erfolgte bei 50 % rel. Luftfeuchtigkeit.

Dazu wurden dynamische Kontaktwinkelbestimmungen am liegenden Tropfen mit dem Kontaktwinkelmessgerät Tropfenkonturanalyse-System DSA100 (Fa. Krüss, D-Hamburg), das mit einem vollautomatischen Tropfendosiersystem kombiniert war, bei Raumtemperatur 23°C ± 1°C durchgeführt. Die Messung erfolgte ebenfalls bei 50 % rel. Luftfeuchtigkeit.

Die Benetzung wurde 40 Sekunden, 60 Sekunden, 90 Sekunden und 120 Sekunden nach Mischbeginn des jeweiligen Zweikomponenten-Dentalabformmaterials, d.h. während dessen Verarbeitungszeit, durch Platzieren eines Wassertropfens mit einem Volumen von 2 µl auf die polymerisierende Oberfläche initiiert. Dabei wurde jeweils für die Dauer einer Einzelmessung von 180 Sekunden die dynamische Veränderung der Tropfengeometrie mit einer Auflösung von 10 Bildern pro Sekunde erfasst und mit der Herstellersoftware ausgewertet. Die Kontaktwinkel der Einzelbilder wurden nach der Methode "Circle fit" als gemittelte Kontaktwinkel aus den linken und rechten Kontaktwinkeln der einzelnen Tropfenkonturen ermittelt.

Zudem zeichnet sich auch das ausgehärtete Abformmaterial zum Zeitpunkt des Ausgießens mit Gips (direkt oder 2 Stunden nach dem Aushärten) durch einen Kontaktwinkel von kleiner 10° bei einem Wassertropfenalter in den ersten 10 Sekunden, bevorzugt 5 Sekunden nach Aufsetzen des Wassertropfens auf die Oberfläche des Dentalabformmaterials aus.

Die Erfindung betrifft auch das ausgehärtete Abformmaterial, das sich durch Härten der oben beschriebenen Zusammensetzungen erhalten lässt. Das ausgehärtete Abformmaterial zeichnet sich durch ausgezeichnete mechanische Eigenschaften aus und erfüllt alle Anforderungen nach ISO 4823 an ein elastomeres Dentalabformmaterial.

Die Erfindung betrifft weiterhin die Verwendung der oben beschriebenen Fluortenside ausgewählt aus den Gruppen a) bis h) gegebenenfalls in Kombination mit weiterem Fluortensid und in Kombination mit Silizium aufweisenden nichtionischen Tensid, das mindestens einen (Poly)alkylenoxidrest und eine Molmasse von weniger als 6.000 g/mol aufweist, zur Herstellung von Dentalmassen, insbesondere von Dentalabformmassen.

Die Erfindung betrifft außerdem die Verwendung der oben beschriebenen härtbaren Zusammensetzungen zur Herstellung von Dentalmassen, insbesondere von Dentalabformmassen.

Die folgenden Beispiele erläutern die Erfindung ohne diese zu beschränken.

### A) Additionsvernetzende Silicone

### Herstellungsbeispiel A1: Katalysatormasse eines additionsvernetzenden Zweikomponenten-Silicon-Dentalabformmaterials

In einem Vakuummischer wurden 50,00 Teile eines α,ω-Divinyipoiydimethylsiloxans mit einer Viskosität bei 20°C von 1.000 mPas, 6,00 Teile einer pyrogen hergestellten, hochdispersen, hydrophobierten Kieselsäure mit einer BET-Oberfläche von 170 m²/g, 43,00 Teile Quarzmehl mit einer mittleren Korngröße von 10 µm und 1,00 Teile eines Platin-Katalysators vom Karstedt-Typ mit einem Gehalt an reinem Platin von 1,0 % für 1,5 Stunden homogen gemischt und danach 15 Minuten entgast. Es wurde eine dünnfließende Paste (ISO 4823) erhalten. Die Paste stellt eine mögliche Katalysatorkomponente der erfindungsgemäßen Zweikomponenten-Dentalabformmasse dar.

### Herstellungsbeispiel A2: Grundrezeptur einer Basismasse eines additionsvernetzenden Zweikomponenten-Silicon-Dentalabformmaterials

In einem Vakuummischer wurden 20,00 Teile eines α,ω-Divinylpolydimethylsiloxans mit einer Viskosität bei 20°C von 1000 mPas, 19,00 Teile eines Polymethylhydrogen-siloxans mit einer Viskosität bei 20°C von 200 mPas und einem SiH-Gehalt von 1,8 mmol/g mit 5,00 Teilen einer pyrogen hergestellten, hochdispersen, hydrophobierten Kieselsäure mit einer BET-Oberfläche von 170 m²/g und 46,00 Teilen Quarzmehl mit einer mittleren Korngröße von 10 µm für 1,5 Stunden homogen gemischt und danach 15 Minuten entgast. Die erhaltene Paste stellt eine Grundrezeptur einer Basiskomponente der erfindungsgemäßen Zweikomponenten-Dentalabformmasse dar, auf die in den folgenden Beispielen für die Zugabe der erfindungsgemäßen Fluortenside zurückgegriffen wird.

### Herstellungsbeispiel A3: Basismasse eines additionsvernetzenden Zweikomponenten-Silicon-Dentalabformmaterials mit einem erfindungsgemäßen Fluortensid

94,90 Teile der Grundrezeptur aus Herstellungsbeispiel A2 wurden in einem Vakuummischer mit 0,75 Teilen eines Perfluorpolyethertensids (Fluorolink E10 H der Firma Solvay Solexis) mit einer Oberflächenspannung von 23 dyn/cm bei 20°C und einer Molmasse von ca. 1.500 g/mol, 2,25 Teilen eines Polyethylenoxidmodifizierten Polydimethylsiloxans (PEG-8-Methicone) mit einer Oberflächenspannung von 20,7 dyn/cm (in entionisiertem Wasser, bei 25°C, bei einer Konzentration von 1 %) und einer Molmasse von ca. 620 g/mol, und 1,00 Teilen eines α,ω-Diallylpoly-ethylenglycols mit einer mittleren Molmasse von 2.000 g/mol und 1,10 Teilen Glycerin für 15 Minuten homogen gemischt und danach 15 Minuten entgast. Es wurde eine dünnfließende Paste (ISO 4823) erhalten. Die Paste stellt eine mögliche Basiskomponente der erfindungsgemäßen Zweikomponenten-Dentalabformmasse dar.

### Beispiel A1: Additionsvernetzende Zweikomponenten-Silicon-Dentalabformmasse mit einem erfindungsgemäßen Fluortensid

50 Teile der in Herstellungsbeispiel A1 beschriebenen Katalysatorkomponente und 50 Teile der in Herstellungsbeispiel A3 beschriebenen Basiskomponente wurden aus einer Kartusche (Fa. Mixpac) ausgedrückt und über einen statischen Mischer (Fa. Mixpac) homogen gemischt.

Ergebnis: Es wurde eine dünn fließende Dentalabformmasse (ISO 4823) erhalten, deren Kontaktwinkel zu verschiedenen Zeitpunkten innerhalb der Verarbeitungszeit untersucht wurde (siehe Tabellen 1A und 1B). Es zeigte sich, dass in jeder Phase während der Verarbeitungszeit der Abformmasse ein aufgebrachter Wassertropfen innerhalb von 1 bis 10 Sekunden vollständig auf der Oberfläche der Abformasse spreitet und diese benetzt. Ein solches Material ist in hervorragender Weise geeignet, im Patientenmund trotz Einwirkung von feuchter Zahnsubstanz und Speichel an den abzuformenden Bereichen anzufließen und diese präzise in der Abformung abzubilden. Im Vergleich zu handelsüblichen, nicht mit dem erfindungsgemäßen Fluortensid versehenen additionsvernetzenden Silicon-Dentalabformmaterial (Vergleichsbeispiel AV1), wird der Unterschied im Verhalten eines aufgebrachten Wassertropfens auf der Abformmaterialoberfläche deutlich. Während bei Zusatz des erfindungsgemäßen Fluortensids in jeder Phase während der Verarbeitungszeit der Abformmasse ein aufgebrachter Wassertropfen innerhalb 1 bis 10 Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet, im Patientenmund trotz Einwirkung von feuchter Zahnsubstanz, Speichel und Blut an die abzuformenden Bereiche anzufließen und diese präzise in der Abformung abzubilden.

### Vergleichsbeispiel AV1: Additionsvernetzende Zweikomponenten-Silicon-Dentalabformmasse mit 1,0 % Fluortensid gemäß US-A-4,657,959 Tabelle IV, run 11

Ein additionsvernetzendes Silicon-Dentalabformmaterial gemäß Beispiel Tabelle IV, run 11, der US-A-4,657,959 wurde aus einer Kartusche (Fa. Mixpac) ausgedrückt und über einen statischen Mischer (Fa. Mixpac) homogen gemischt.

Ergebnis: Man erhielt eine dünn fließende Dentalabformmasse (ISO 4823), deren Kontaktwinkel zu verschiedenen Zeitpunkten innerhalb der Verarbeitungszeit untersucht wurde (siehe Tabellen 1 und 2). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wurde. Die aufgebrachten Wassertropfen spreiten nicht auf der Oberfläche der Abformmasse und benetzen diese nicht ausreichend. Die Gleichgewichtskontaktwinkel zwischen 40 Sekunden und 120 Sekunden Materialalter (nach Mischbeginn der Katalysator- und der Basiskomponente) lagen 10 Sekunden nach Aufbringen des Wassertropfens zwischen 38° und 44°. Dieses Vergleichsbeispiel zeigt, dass nicht jede beliebige Kombination von Tensiden zu einem synergistischen Effekt führt. Die Gleichgewichtskontaktwinkel zwischen 40 Sekunden und 120 Sekunden Materialalter (nach Mischbeginn der Katalysator- und der Basiskomponente) lagen 10 Sekunden nach Aufbringen des Wassertropfens zwischen 79° und 63°. Dieses Vergleichsbeispiel zeigt, dass bei Verwendung der Messmethode der vorliegenden Erfindung die Dentalabformmaterialien nach US-A-4,657,959 keine Kontaktwinkel < 10° und kein Spreiten von Wasser auf der Oberfläche erreicht wird.

### Vergleichsbeispiel AV2: Additionsvernetzende Zweikomponenten-Silicon-Dentalabformmasse mit einer nicht synergistisch wirkenden Tensidmischung aus dem besten Silicontensid (run 2) und Fluortensid (run 11) aus Beispiel 1 der US-A-4,657,959.

50 Teile der in US-A-4,657,959 beschriebenen Katalysatorkomponente und 50 Teile der in Herstellungs-Vergleichsbeispiel A9 beschriebenen Basiskomponente wurden aus einer Kartusche (Fa. Mixpac) ausgedrückt und über einen statischen Mischer (Fa. Mixpac) homogen gemischt.

Ergebnis: Es wurde eine dünn fließende Dentalabformmasse (ISO 4823) erhalten, deren Kontaktwinkel zu verschiedenen Zeitpunkten innerhalb der Verarbeitungszeit untersucht wurde (siehe Tabellen 1 und 2). Es zeigte sich, dass in keiner Phase während der Verarbeitungszeit der Abformmasse innerhalb der Messzeit von 30 Sekunden nach Wassertropfenaufgabe ein Kontaktwinkel von < 10° erreicht wurde. Die aufgebrachten Wassertropfen spreiten nicht auf der Oberfläche der Abformmasse und benetzen diese nicht ausreichend. Die Gleichgewichtskontaktwinkel zwischen 40 Sekunden und 120 Sekunden Materialalter (nach Mischbeginn der Katalysator- und der Basiskomponente) lagen 10 Sekunden nach Aufbringen des Wassertropfens zwischen 38° und 44°. Dieses Vergleichsbeispiel zeigt, dass nicht jede beliebige Kombination von Tensiden zu einem synergistischen Effekt führt.

Die Gleichgewichtskontaktwinkel zwischen 40 Sekunden und 120 Sekunden Materialalter (nach Mischbeginn der Katalysator- und der Basiskomponente) lagen 10 Sekunden nach Aufbringen des Wassertropfens zwischen 39° bis 50°. Dieses Vergleichsbeispiel zeigt, dass nicht jede beliebige Kombination von Fluortensiden und Silicontensiden zu einem synergistischen Effekt führt. Überraschenderweise wurde ein synergistischer Effekt nur in Tensidmischungen mit bestimmten Fluortensiden und Silicontensiden erzielt. Die US-A-4,657,959 beschreibt keine Mischungen von verschiedenen Tensiden. Werden, wie in diesem Vergleichsbeispiel, die jeweils besten Tenside aus der experimentellen Reihen der Silicontenside und der Fluortenside der US-A-4,657,959 kombiniert, ist kein Spreiten von Wassertropfen auf der Oberfläche des Silicon-Abformmaterials zu erreichen.

**Tabelle 1: Zeitabhängige Kontaktwinkelmessungen an additionsvernetzenden Siliconen mit erfindungsgemäßen Fluortensiden im Vergleich zum Stand der Technik**

| Beispiele/ Vergleichsbeispiele | Materialalter nach Mischbeginn 40 s ¹⁾ Zeitspanne bis ein Kontaktwinkel von 10° erreicht wird ²⁾ [s] | Materialalter nach Mischbeginn 60 s ¹⁾ Zeitspanne bis ein Kontaktwinkel von 10° erreicht wird ²⁾ [s] | Materialalter nach Mischbeginn 90 s ¹⁾ Zeitspanne bis ein Kontaktwinkel von 10° erreicht wird ²⁾ [s] | Materialalter nach Mischbeginn 120 s ¹⁾ Zeitspanne bis ein Kontaktwinkel von 10° erreicht wird ²⁾ [s] |
|---|---|---|---|---|
| Beispiel A1 | 3 | 3 | 3 | 3 |
| Vergleichsbeispiel AV1 | - ³⁾ | - ³⁾ | - ³⁾ | - ³⁾ |
| Vergleichsbeispiel AV2 | - ³⁾ | - ³⁾ | - ³⁾ | - ³⁾ |

| | | | | |
|---|---|---|---|---|
| ¹⁾ Zeitpunkt des Aufsetzens des Wassertropfens nach Mischbeginn des Abformmaterials ²⁾ Tropfengröße ca. 2 µl, Messzeit Tropfen max. 30 s ³⁾ Innerhalb der Messzeit von 30 s nach Wassertropfenaufgabe wird kein Kontaktwinkel < 10° erreicht. | | | | |

**Tabelle 2: Zeitabhängige Kontaktwinkelmessungen an additionsvernetzenden Siliconen mit erfindungsgemäßen Fluortensiden im Vergleich zum Stand der Technik**

| Beispiele/ Vergleichsbeispiele | Materialalter nach Mischbeginn 40 s ¹⁾ Tropfenalter 10 s ²⁾ Kontaktwinkel [°] | Materialalter nach Mischbeginn 60 s ¹⁾ Tropfenalter 10 s ²⁾ Kontaktwinkel [°] | Materialalter nach Mischbeginn 90 s ¹⁾ Tropfenalter 10 s ²⁾ Kontaktwinkel [°] | Materialalter nach Mischbeginn 120 s ¹⁾ Tropfenalter 10 s ²⁾ Kontaktwinkel [°] |
|---|---|---|---|---|
| Beispiel A1 | < 10 | < 10 | < 10 | < 10 |
| Vergleichsbeispiel AV1 | 79 | 77 | 75 | 63 |
| Vergleichsbeispiel AV2 | 39 | 42 | 45 | 50 |

| | | | | |
|---|---|---|---|---|
| ¹⁾ Zeitpunkt des Aufsetzens des Wassertropfens nach Mischbeginn des Abformmaterials ²⁾ Tropfengröße ca. 2 µl, Messzeit Tropfen max. 30 s | | | | |

### B) Kondensationsvernetzende Silicone

### Herstellungsbeispiel B1: Basismasse eines kondensationsvernetzenden Silicon-Dentalabformmaterials mit einer erfindungsgemäßen synergistisch wirkenden Tensid-Mischung

97 Teile der Basismasse des Handelsprodukts Lastic 90 fine, Kettenbach GmbH + Co. KG, Lot 91631, wurden in einem Vakuummischer mit 1,50 Teilen eines Perfluorpolyethertensids (Fluorolink E10 H der Firma Solvay Solexis) mit einer Oberflächenspannung von 23 dyn/cm bei 20°C und einer Molmasse von ca. 1.500 g/mol, 1,50 Teilen eines mit Polyethylenoxid modifizierten Polydimethylsiloxans (PEG-8-Methicone) mit einer Oberflächenspannung von 20,7 dyn/cm (in entionisiertem Wasser, bei 25°C, bei einer Konzentration von 1 %) und einer Molmasse von ca. 620 g/mol, für 15 Minuten homogen gemischt und entgast. Es wurde eine dünnfließende Paste (ISO 4823) erhalten. Die Paste stellt eine mögliche Basiskomponente der erfindungsgemäßen Zweikomponenten-Dentalabformmasse dar.

### Beispiel B1: Kondensationsvernetzende Zweikomponenten-Silicon-Dentalabformmasse mit einer erfindungsgemäßen synergistisch wirkenden Tensid-Mischung

7,1 Teile einer Katalysatorpaste eines kondensationsvernetzenden Silicon-Dentalabformmaterials (Handelsprodukt Lastic Xtra Pastenhärter, Kettenbach GmbH + Co. KG, Lot 91841) und 92,9 Teile der in Herstellungsbeispiel B1 beschriebenen Basiskomponente wurden auf einem Mischblock mit einem Mischspatel während 30 Sekunden homogen gemischt.

Ergebnis Beispiel B1: Es wurde eine dünnfließende Dentalabformmasse (ISO 4823) erhalten, deren Kontaktwinkel zu verschiedenen Zeitpunkten innerhalb der Verarbeitungszeit untersucht wurde (siehe Tabellen 3 und 4). Es zeigte sich, dass in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt. Ein solches Material ist in hervorragender Weise geeignet, im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen kondensationsvernetzenden Silicon-Dentalabformmaterialien (Vergleichsbeispiel BV1) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet, im Patientenmund trotz Einwirkung von feuchter Zahnsubstanz, Speichel und Blut an die abzuformenden Bereiche anzufließen und diese präzise in der Abformung abzubilden.

### Vergleichsbeispiel BV1: Kondensationsvernetzendes Silicon-Dentalabformmaterial nach dem Stand der Technik (Handelsprodukt)

Ein kondensationsvernetzendes Silicon-Dentalmaterial (Handelsprodukt Lastic 90 fine, Kettenbach GmbH + Co. KG, Lot 91631/91841) wird gemäß Herstellerangaben auf einem Mischblock mit einem Mischspatel homogen gemischt. Ergebnis Vergleichsbeispiel BV1: Die Gleichgewichtskontaktwinkel zwischen 40 und 120 Sekunden Materialalter (nach Mischbeginn der Katalysator- und der Basiskomponente) lagen 10 Sekunden nach Aufbringen des Wassertropfens zwischen 90° und 105°. Dieses Vergleichsbeispiel zeigt, dass kondensationsvernetzende Silicon-Dentalabformmaterialien nach dem Stand der Technik nicht zu dem erfindungsgemäßen Effekt eines spontanen Spreitens eines aufgebrachten Wassertropfens auf der Oberfläche des Dentalabformmaterials führen.

**Tabelle 3: Zeitabhängige Kontaktwinkelmessungen an kondensationsvernetzenden Siliconen mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Zeitspanne bis ein Kontaktwinkel von 10° erreicht wird ²⁾ [s] | Materialalter nach Mischbeginn 60 s ¹⁾ Zeitspanne bis ein Kontaktwinkel von 10° erreicht wird ²⁾ [s] | Materialalter nach Mischbeginn 90 s ¹⁾ Zeitspanne bis ein Kontaktwinkel von 10° erreicht wird ²⁾ [s] | Materialalter nach Mischbeginn 120 s ¹⁾ Zeitspanne bis ein Kontaktwinkel von 10° erreicht wird ²⁾ [s] |
|---|---|---|---|---|
| Beispiel B1 | 0,5 | 0,5 | 0,6 | 0,4 |
| Vergleichsbeispiel BV1 | - ³⁾ | - ³⁾ | - ³⁾ | - ³⁾ |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s ³⁾ Innerhalb der Messzeit von 20 s nach Wassertropfenaufgabe wird kein Kontaktwinkel < 10° erreicht. | | | | |

**Tabelle 4: Zeitabhängige Kontaktwinkelmessungen an kondensationsvernetzenden Siliconen mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Tropfenalter 10 s ²⁾ Kontaktwinkel [°] | Materialalter nach Mischbeginn 60 s ¹⁾ Tropfenalter 10 s ²⁾ Kontaktwinkel [°] | Materialalter nach Mischbeginn 90 s ¹⁾ Tropfenalter 10 s ²⁾ Kontaktwinkel [°] | Materialalter nach Mischbeginn 120 s ¹⁾ Tropfenalter 10 s ²⁾ Kontaktwinkel [°] |
|---|---|---|---|---|
| Beispiel B1 | < 10 | < 10 | < 10 | < 10 |
| Vergleichsbeispiel BV1 | 90 | 94 | 101 | 105 |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s | | | | |

### C) Kondensationsvernetzende Alkoxysilylpolyether-Dentalabformmaterialien

### Herstellungsbeispiel C1: Basismasse eines kondensationsvernetzenden Alkoxysilylpolyether-Dentalabformmaterials mit einer erfindungsgemäßen synergistisch wirkenden Tensid-Mischung

94 Teile einer Basismasse eines kondensationsvernetzenden Alkoxysilylpolyether-Dentalabformmaterials gemäß EP-A-1 226 808 (Handeslprodukt P2-Polyether mono, Heraeus-Kulzer, Lot #290489) wurden in einem Vakuummischer mit 3,00 Teilen eines Perfluorpolyethertensids (Fluorolink E10 H der Firma Solvay Solexis) mit einer Oberflächenspannung von 23 dyn/cm bei 20°C und einer Molmasse von ca. 1.500 g/mol, 3,00 Teilen eines mit Polyethylenoxid modifizierten Polydimethylsiloxans (PEG-8-Methicone) mit einer Oberflächenspannung von 20,7 dyn/cm (in entionisiertem Wasser, bei 25°C, bei einer Konzentration von 1 %) und einer Molmasse von ca. 620 g/mol, für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Beispiel C1: Kondensationsvernetzendes Alkoxysilylpolyether-Dentalabformmaterial mit einer erfindungsgemäßen synergistisch wirkenden Tensid-Mischung

16,7 Teile einer Katalysatorpaste eines kondensationsvernetzenden Alkoxysilyl-Polyether-Dentalabformmaterials gemäß EP-A-1 226 808 (Handelsprodukt P2-Polyether mono, Heraeus-Kulzer, Lot #290489) und 83,3 Teile der in Herstellungsbeispiel C1 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (Heraeus-Kulzer) homogen gemischt.

Ergebnis Beispiel C1: Es wurde eine mittelfließende Dentalabformmasse (ISO 4823) erhalten, deren Kontaktwinkel zu verschiedenen Zeitpunkten innerhalb der Verarbeitungszeit untersucht wurde (siehe Tabellen 5 und 6). Es zeigte sich, dass in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt. Ein solches Material ist in hervorragender Weise geeignet, im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen kondensationsvernetzenden Alkoxysilyl-Dentalabformmaterialien (Vergleichsbeispiel CV1) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet, im Patientenmund trotz Einwirkung von feuchter Zahnsubstanz, Speichel und Blut an die abzuformenden Bereiche anzufließen und diese präzise in der Abformung abzubilden.

### Vergleichsbeispiel CV1: Kondensationsvernetzendes Alkoxysilyl-Polyether-Dentalabformmaterial nach dem Stand der Technik (Handelsprodukt)

Ein kondensationsvernetzendes Alkoxysilyl-Polyether-Dentalabformmaterial gemäß EP-A-1 226 808 (Handelsprodukt P2 Polyether mono, Heraeus Kulzer, Lot #290489) wird gemäß Herstellerangaben mit Hilfe eines elektrischen Austragsgeräts (Plug + Press, Kettenbach GmbH + Co. KG) aus Schlauchbeuteln ausgetragen und über einen dynamischen Mischer (Heraeus-Kulzer) homogen gemischt.

Ergebnis Vergleichsbeispiel CV1: Die Gleichgewichtskontaktwinkel zwischen 40 und 120 Sekunden Materialalter (nach Mischbeginn der Katalysator- und der Basiskomponente) lagen 10 Sekunden nach Aufbringen des Wassertropfens zwischen 62° und 66°. Dieses Vergleichsbeispiel zeigt, dass kondensationsvernetzende Alkoxysilyl-Polyether-Dentalabformmaterialien nach dem Stand der Technik nicht zu dem erfindungsgemäßen Effekt eines spontanen Spreitens eines aufgebrachten Wassertropfens auf der Oberfläche des Dentalabformmaterials führen.

**Tabelle 5: Zeitabhängige Kontaktwinkelmessungen an kondensationsvernetzenden Alkoxysilylpolyether mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Zeitspanne bis ein Kontaktwinkel von 10° erreicht wird ²⁾ [s] | Materialalter nach Mischbeginn 60 s ¹⁾ Zeitspanne bis ein Kontaktwinkel von 10° erreicht wird ²⁾ [s] | Materialalter nach Mischbeginn 90 s ¹⁾ Zeitspanne bis ein Kontaktwinkel von 10° erreicht wird ²⁾ [s] | Materialalter nach Mischbeginn 120 s ¹⁾ Zeitspanne bis ein Kontaktwinkel von 10° erreicht wird ²⁾ [s] |
|---|---|---|---|---|
| Beispiel C1 | 0,25 | 0,25 | 0,25 | 0,25 |
| Vergleichsbeispiel CV1 | - ³⁾ | - ³⁾ | - ³⁾ | - ³⁾ |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s ³⁾ Innerhalb der Messzeit von 20 s nach Wassertropfenaufgabe wird kein Kontaktwinkel < 10° erreicht. | | | | |

**Tabelle 6: Zeitabhängige Kontaktwinkelmessungen an kondensationsvernetzenden Alkoxysilylpolyether mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Tropfenalter 10 s ²⁾ Kontaktwinkel [°] | Materialalter nach Mischbeginn 60 s ¹⁾ Tropfenalter 10 s ²⁾ Kontaktwinkel [°] | Materialalter nach Mischbeginn 90 s ¹⁾ Tropfenalter 10 s ²⁾ Kontaktwinkel [°] | Materialalter nach Mischbeginn 120 s ¹⁾ Tropfenalter 10 s ²⁾ Kontaktwinkel [°] |
|---|---|---|---|---|
| Beispiel C1 | < 10 | < 10 | < 10 | < 10 |
| Vergleichsbeispiel CV1 | 64 | 62 | 66 | 65 |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s | | | | |

### D) Additionsvernetzende Aziridino-Polvether-Dentalabformmaterialien

### Herstellungsbeispiel D1: Basismasse eines additionsvernetzenden Aziridino-Polyether-Dentalabformmaterials mit einer erfindungsgemäßen synergistisch wirkenden Tensid-Mischung

97,6 Teile der Basismasse eines additionsvernetzenden Aziridino-Polyether-Dentalabformmaterials gemäß US-A-4,353,242 (Handelsprodukt Impregum Penta, 3M-Espe, Lot 347351) wurden in einem Vakuummischer mit 0,45 Teilen eines Perfluorpolyethertensids (Fluorolink E10 H der Firma Solvay Solexis) mit einer Oberflächenspannung von 23 dyn/cm bei 20°C und einer Molmasse von ca. 1.500 g/mol, 1,35 Teilen eines mit Polyethylenoxid modifizierten Polydimethylsiloxans (PEG-8-Methicone) mit einer Oberflächenspannung von 20,7 dyn/cm (in entionisiertem Wasser, bei 25°C, bei einer Konzentration von 1 %) und einer Molmasse von ca. 620 g/mol und 0,6 Teilen Glycerin für 15 Minuten homogen gemischt und entgast. Es wurde eine mittelfließende Paste (ISO 4823) erhalten. Die Paste stellt eine mögliche Basiskomponente der erfindungsgemäßen Zweikomponenten-Dentalabformmasse dar.

### Beispiel D1: Additionsvernetzendes Aziridino-Polyether-Dentalabformmaterial mit einer erfindungsgemäßen, synergistisch wirkenden Tensid-Mischung

16,7 Teile einer Katalysatorpaste eines additionsvernetzenden Aziridino-Polyether-Dentalabformmaterials gemäß US-A-4,353,242 (Handelsprodukt Impregum Penta, 3M-Espe, Lot 346743) und 83,3 Teile der in Herstellungsbeispiel D1 beschriebenen Basiskomponente wurden mit Hilfe eines elektrischen Austragsgeräts (Plug + Press-Dispenser, Kettenbach GmbH + Co. KG) jeweils aus Schlauchbeuteln ausgetragen und über einen dynamischen Mischer (3M-Espe) homogen gemischt.

Ergebnis Beispiel D1: Es wurde eine mittelfließende Dentalabformmasse (ISO 4823) erhalten, deren Kontaktwinkel zu verschiedenen Zeitpunkten innerhalb der Verarbeitungszeit untersucht wurde (siehe Tabellen 7 und 8). Es zeigte sich, dass in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt. Ein solches Material ist in hervorragender Weise geeignet, im Patientenmund trotz Einwirkung von feuchter Zahnsubstanz und Speichel an den abzuformenden Bereichen anzufließen und diese präzise in der Abformung abzubilden. Im Vergleich zu handelsüblichen nicht mit der erfindungsgemäßen synergistischen Tensidmischung versehenen additionsvernetzenden Aziridinopolyether-Dentalabformmaterialien (Vergleichsbeispiel DV1) wird der Unterschied im Verhalten eines aufgebrachten Wassertropfens auf der Abformmaterialoberfläche deutlich. Während bei Zusatz der synergistischen Tensidmischung in jeder Phase während der Verarbeitungszeit der Abformmasse ein aufgebrachter Wassertropfen innerhalb von ≤ 10 Sekunden vollständig auf der Oberfläche der Abformmasse spreitet und diese benetzt, spreiten bei der nicht modifizierten Handelsware die aufgebrachten Wassertropfen nicht auf der Oberfläche der Abformmasse und benetzen diese nicht ausreichend. Das erfindungsgemäße Material ist im Gegensatz zu marktüblichen Dentalabformmaterialien in hervorragender Weise geeignet, im Patientenmund trotz Einwirkung von feuchter Zahnsubstanz, Speichel und Blut an die abzuformenden Bereiche anzufließen und diese präzise in der Abformung abzubilden.

### Vergleichsbeispiel DV1: Additionsvernetzendes Aziridino-Polyether-Dentalabformmaterial nach dem Stand der Technik (Handelsprodukt)

Ein additionsvernetzendes Aziridino-Polyether-Dentalabformmaterial gemäß US-A-4,353,242 (Handelsprodukt Impregum penta, 3M-Espe, Lot 347351/346743) wird gemäß Herstellerangaben mit Hilfe eines elektrischen Austragsgeräts (Plug + Press-Dispenser, Kettenbach GmbH + Co. KG) homogen gemischt.

Ergebnis Vergleichsbeispiel DV1: Die Gleichgewichtskontaktwinkel zwischen 40 und 120 Sekunden Materialalter (nach Mischbeginn der Katalysator- und der Basiskomponente) lagen 10 Sekunden nach Aufbringen des Wassertropfens zwischen 53° und 58°. Dieses Vergleichsbeispiel zeigt, dass additionsvernetzende Aziridino-Polyether-Dentalabformmaterialien nach dem Stand der Technik nicht zu dem erfindungsgemäßen Effekt eines spontanen Spreitens eines aufgebrachten Wassertropfens auf der Oberfläche des Dentalabformmaterials führen.

**Tabelle 7: Zeitabhängige Kontaktwinkelmessungen an additionsvernetzende Aziridino-Polyether mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Zeitspanne bis ein Kontaktwinkel von 10° erreicht wird ²⁾ [s] | Materialalter nach Mischbeginn 60 s ¹⁾ Zeitspanne bis ein Kontaktwinkel von 10° erreicht wird ²⁾ [s] | Materialalter nach Mischbeginn 90 s ¹⁾ Zeitspanne bis ein Kontaktwinkel von 10° erreicht wird ²⁾ [s] | Materialalter nach Mischbeginn 120 s ¹⁾ Zeitspanne bis ein Kontaktwinkel von 10° erreicht wird ²⁾ [s] |
|---|---|---|---|---|
| Beispiel D1 | 12,5 | 10 | 10 | 10 |
| Vergleichsbeispiel CV1 | - ³⁾ | - ³⁾ | - ³⁾ | - ³⁾ |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s ³⁾ Innerhalb der Messzeit von 20 s nach Wassertropfenaufgabe wird kein Kontaktwinkel < 10° erreicht. | | | | |

**Tabelle 8: Zeitabhängige Kontaktwinkelmessungen an additionsvernetzende Aziridino-Polyether mit einer erfindungsgemäßen synergistisch wirkenden Tensid-Mischung im Vergleich zum Stand der Technik**

| Beispiele/ Vergleichsbeispiele | Materialalter nach Mischbeginn 40 s ¹⁾ Tropfenalter 10 s ²⁾ Kontaktwinkel [°] | Materialalter nach Mischbeginn 60 s ¹⁾ Tropfenalter 10 s ²⁾ Kontaktwinkel [°] | Materialalter nach Mischbeginn 90 s ¹⁾ Tropfenalter 10 s ²⁾ Kontaktwinkel [°] | Materialalter nach Mischbeginn 120 s ¹⁾ Tropfenalter 10 s ²⁾ Kontaktwinkel [°] |
|---|---|---|---|---|
| Beispiel D1 | < 10 | < 10 | < 10 | < 10 |
| Vergleichsbeispiel DV1 | 53 | 53 | 56 | 58 |

| | | | | |
|---|---|---|---|---|
| ¹⁾ Zeitpunkt des Aufsetzens des Wassertropfens nach Mischbeginn des Abformmaterials ²⁾ Tropfengröße ca. 8 µl, Messzeit Tropfen max. 20 s | | | | |

Die erfindungsgemäßen Beispiele und die Vergleichsbeispiels aus dem Stand der Technik zeigen, dass nicht jede beliebige Kombination von Fluortensiden und Silicontensiden zu einem synergistischen Effekt im Sinne der vorliegenden Erfindung führt. Selbst wenn die jeweils besten Tenside aus der experimentellen Reihe der Silicontenside und der Fluortenside aus dem Stand der Technik kombiniert werden, ist kein Spreiten von Wassertropfen auf der Oberfläche des Silicon-Abformmaterials zu erreichen.

Im Gegensatz dazu wurde überraschenderweise ein synergistischer Effekt nur in Tensidmischungen mit bestimmten Fluortensiden und Silicontensiden gemäß der vorliegenden Erfindung erzielt.

## Patentansprüche

1. Zusammensetzung enthaltend härtbare Polymere ausgewählt aus der Gruppe der durch Additionsreaktion vernetzenden Organopolysiloxane, der durch Kondensationsreaktion vernetzenden Organopolysiloxane, der durch Kondensationsreaktion vernetzenden Alkoxysilylreste enthaltenden Polyether, der durch Additionsreaktion vernetzenden Aziridinoreste enthaltenden Polyether, der durch Additionsreaktion vernetzenden Alkenylreste enthaltenden Polyether, der durch radikalische Polymerisationsreaktion vernetzende Esterreste einer ethylenisch ungesättigten Carbonsäure enthaltenden Polyether oder der durch ringöffnende Metathesereaktion vernetzenden Polyether, Silicone oder Kautschuke, und
enthaltend ferner mindestens ein nichtionisches Fluortensid ausgewählt aus der Gruppe
a) der Fluortenside, die mindestens 2 teil- oder perfluorierte Alkylenoxid- oder Polyalkylenoxideinheiten aufweisen,
sowie enthaltend mindestens ein Silizium enthaltende Gruppen aufweisendes nichtionisches Tensid mit einer Molmasse von weniger als 6000 g/mol, **wobei** das Fluortensid der Gruppe a) ein Blockcopolymer enthaltend Blöcke der Formel Ic ist
B-[O-R_{H}]ₑ-A-[O-R_{F}]ₐ-A'-[O-R'_{H}]_{d}-B' (Ic),
worin
R_{F} ein Rest der Formel -CₘFₙHₒ- ist, wobei innerhalb eines Polyetherrestes die Indizes m, n und o im Rahmen der gegebenen Definitionen unterschiedlich sein können,
m eine ganze Zahl von 2 bis 12 ist,
n eine ganze Zahl von 1 bis 24 ist,
o eine ganze Zahl von 0 bis 23 ist,
wobei die Summe von n und o dem Wert 2m entspricht,
R_{H} ein Rest der Formel -CₚH₂ₚ- ist, wobei innerhalb eines Polyetherrestes der Index p im Rahmen der gegebenen Definition unterschiedlich sein kann,
R'_{H} ein Rest der Formel -C_{q}H_{2q}- ist, wobei innerhalb eines Polyetherrestes der Index q im Rahmen der gegebenen Definition unterschiedlich sein kann,
p eine ganze Zahl von 2 bis 12, vorzugsweise von 2 bis 4, bedeutet,
q eine ganze Zahl von 2 bis 12, vorzugsweise von 2 bis 4, bedeutet,
A und A' unabhängig voneinander eine kovalente Bindung oder eine zweiwertige Brückengruppe, die mit den Blöcken [O-R_{F}], [O-R_{H}] und [O-R'_{H}] über C-C- und/oder C-O-Bindungen verknüpft ist, bedeuten,
B und B' unabhängig voneinander Wasserstoff, ein teil- oder perfluorierter Alkylrest mit 1 bis 6 Kohlenstoffatomen oder ein Alkylrest mit 1 bis 6 Kohlenstoffatomen ist,
a eine ganze Zahl von 2 bis 100 ist, vorzugsweise 2 bis 50,
d eine ganze Zahl von 1 bis 100 ist, vorzugsweise 2 bis 50, und
e eine ganze Zahl von 1 bis 100 ist, vorzugsweise 2 bis 50
oder dass
das Fluortensid der Gruppe a) eine Verbindung der Formel Ica ist
HO-(CH₂CH₂O)ₙₐ-CH₂CF₂O-(CF₂CF₂O)pₐ-(CF₂O)qₐ-CF₂CH₂-(OCH₂CH₂)ₙₐ-OH (Ica),
worin na eine ganze Zahl von 1 bis 20 ist,
pa eine ganze Zahl von 0 bis 12 und
qa eine ganze Zahl von 0 bis 20 bedeutet,
mit der Maßgabe, dass die Summe von pa und qa mindestens 1 sein muss,
und neben dem Fluortensid aus der Gruppe a) andere ionische und/oder nichtionische und/oder amphotere Fluortenside enthalten sind.

2. Zusammensetzung nach einem der vorhergehenden Ansprüche, **dadurch gekennzeichnet, dass**
das Silizium aufweisende nichtionische Tensid enthaltend mindestens einen (Poly)alkylenoxidrest eine Molmasse von weniger als 4000 g/mol, insbesondere von 350 bis 2000 g/mol, besitzt,
und/oder das Silizium aufweisende nichtionische Tensid ein Organosiloxantensid der Formel II und/oder der Formel III ist oder ein Organocarbonsilantensid der Formel IV, V und/oder der Formel VI ist 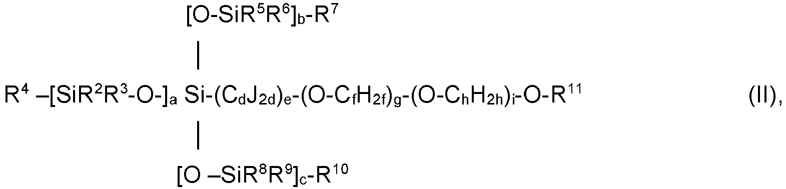 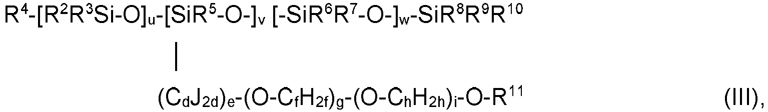 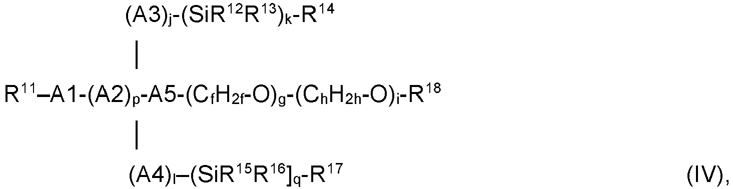 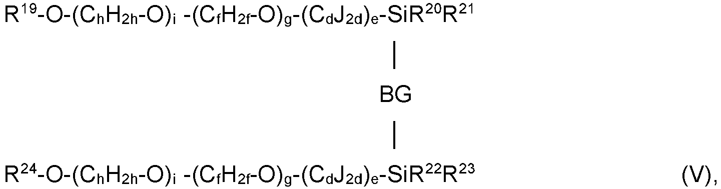
R¹⁹-O-(CₕH₂ₕ-O)ᵢ-(C_{f}H_{2f}-O)_{g}-(C_{d}J_{2d})ₑ-SiR²⁰R²¹R²² (VI),
worin R², R³, R⁴, R⁵, R⁶, R⁷, R⁸, R⁹ und R¹⁰ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
a, b, c und w unabhängig voneinander ganze Zahlen von 0 bis 100 bedeuten, vorzugsweise 0 bis 75, insbesondere 0 bis 35 und ganz besonders bevorzugt 0 bis 15,
v eine ganze Zahl von 1 bis 100 bedeutet, vorzugsweise 1 bis 15 und ganz besonders bevorzugt 1 bis 6,
wobei die Summe von a, b und c zwischen 1 und 300 beträgt, vorzugsweise 1 bis 50, insbesondere 1 bis 10 und ganz besonders bevorzugt 1 bis 3,
und die Summe von v und w zwischen 1 und 200 beträgt, vorzugsweise 2 bis 90, u 0 oder 1 ist,
d eine ganze Zahl von 1 bis 10 ist, vorzugsweise 1 bis 6 und insbesondere 1 bis 3,
J Wasserstoff oder Fluor bedeutet, vorzugsweise Wasserstoff,
e 0 oder 1 ist,
f und h unabhängig voneinander ganze Zahlen von 2 bis 6 bedeuten,
g und i unabhängig voneinander ganze Zahlen von 0 bis 30 sind, vorzugsweise 0 bis 15, wobei die Summe von g und i 1 bis 60 bedeutet, vorzugsweise 2 bis 30, insbesondere 2 bis 15,
R¹¹ Wasserstoff, Alkyl, Alkenyl oder Aryl ist, das gegebenenfalls teilweise oder vollständig fluoriert ist, vorzugsweise Wasserstoff oder Methyl,
R¹², R¹³, R¹⁴, R¹⁵, R¹⁶ und R¹⁷ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
k und q unabhängig voneinander 0 oder 1 bedeuten,
A1 Kohlenstoff oder Silizium bedeutet,
A2, A3 und A4 unabhängig voneinander eine Gruppe C_{d}J_{2d} ist, worin J und d die oben definierten Bedeutungen aufweisen,
j, p und I unabhängig voneinander 0 oder 1 sind,
A5 eine zweiwertige Brückengruppe, insbesondere -O-, -CO-O- oder -CO- bedeutet,
R¹⁸ Wasserstoff, Alkyl, Alkenyl oder Aryl ist, das gegebenenfalls teilweise oder vollständig fluoriert ist, vorzugsweise Wasserstoff oder Methyl,
R²⁰, R²¹, R²² und R²³ unabhängig voneinander Wasserstoff, Alkyl, Alkyloxy, Alkenyl, Alkenyloxy, Alkinyl, Alkinyloxy, Aryl, Aryloxy, Aralkyl, Aralkyloxy, Alkylaryl und/oder Alkylaryloxy bedeuten, die gegebenenfalls teilweise oder vollständig fluoriert sind, vorzugsweise Alkyl oder Alkenyl und insbesondere C₁-C₆-Alkyl,
BG eine zweiwertige Brückengruppe ist, und
R¹⁹ und R²⁴ unabhängig voneinander Wasserstoff, Alkyl, Alkenyl oder Aryl sind, die gegebenenfalls teilweise oder vollständig fluoriert sind, vorzugsweise Wasserstoff oder Methyl,
mit der Massgabe, dass von den Resten R², R³ und R⁴ und/oder den Resten R⁵, R⁶ und R⁷ und/oder den Resten R⁸, R⁹ und R¹⁰ und/oder den Resten R¹⁵, R¹⁶ und R¹⁷ und/oder den Resten R²⁰ und R²¹ und/oder den Resten R²² und R²³ und/oder den Resten R²⁰, R²¹ und R²² nur einer Wasserstoff sein kann,
wobei f und h innerhalb eines Moleküls im Rahmen der gegebenen Definition unterschiedliche Werte annehmen können,
oder das Silizium aufweisende nichtionische Tensid enthaltend mindestens einen (Poly)alkylenoxidrest eine Verbindung der Formel VII, VIII, IX oder X ist 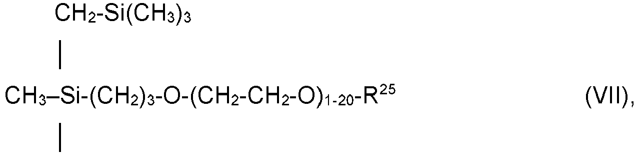 CH₂-Si(CH₃)₃ 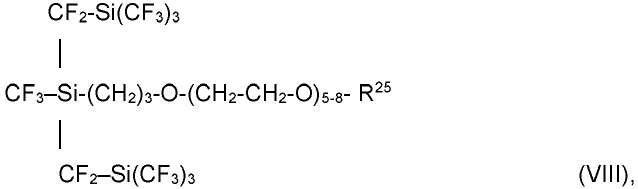 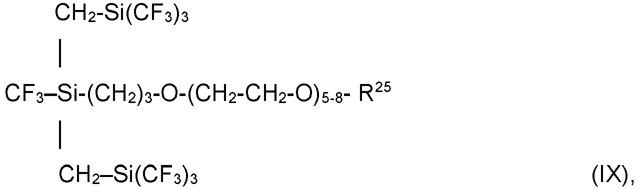 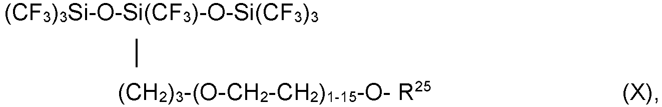 worin R²⁵ Wasserstoff, Methyl, Ethyl, Propyl oder Butyl ist, vorzugsweise Wasserstoff oder Methyl.

3. Zusammensetzung nach mindestens einem der vorhergehenden Ansprüche, **dadurch gekennzeichnet, dass**
diese neben dem mindestens einem Fluortensid der Gruppe a) als weitere Komponente einen Alkenylreste und/oder Alkinylreste enthaltenden Polyether und/oder einen hydroxyl- und/oder aryloxy- und/oder arylalkyloxy- und/oder alkoxy-terminierten Polyether enthält, wobei bevorzugt der Alkenylreste enthaltende Polyether eine Verbindung der Formel XII und der hydroxyl- und/oder alkoxy-terminierte Polyether eine Verbindung der Formel XIII ist
CH₂=CH-CH₂-O-(Cn₂H₂ₙ₂-O)ₘ₉-CH₂-CH=CH₂ (XII),
R³¹-O-(Cₙ₂H₂ₙ₂-O)ₘ₉-R³² (XIII),
worin n2 eine ganze Zahl von 2 bis 8, vorzugsweise von 2 bis 4 bedeutet,
m9 eine ganze Zahl von 3 bis 70000, vorzugsweise von 10 bis 2500 ist,
R³¹ und R³² unabhängig voneinander Wasserstoff oder C₁-C₆-Alkyl, insbesondere Wasserstoff und/oder Methyl, Ethyl oder Propyl bedeuten, und wobei R³¹, R³², n2 und m9 innerhalb eines Moleküls im Rahmen der gegebenen Definitionen unterschiedlich sein können,
und/oder diese nebem dem mindestens einem Fluortensid der Gruppe a) als weitere Komponente ein Polyol enthält, bevorzugt entweder ausgewählt aus der Gruppe der Kohlehydrate, der Polyvinylalkohole, der aliphatischen Di-, Tri-, Tetra-, Penta- und/oder Hexanole und Mischungen von zwei oder mehreren dieser Polyole, oder ausgewählt wird aus der Gruppe der Polyvinylalkohole, der Polysaccharide, Trimethylolpropan, Pentaerythritol, Dipentaerythritol, Glycerin, Allyloxy-1,2-propandiol, 2-Methyl-2,4-pentandiol, Trimethylolpropanallylether, Decandiol, Nonandiol, Octandiol, Heptandiol, Hexandiol, Pentandiol, Butandiol, Propandiol, Ethandiol, Fructose, Glucose und Mischungen von zwei oder mehreren dieser Polyole, insbesondere Glycerin.

4. Zusammensetzung nach mindestens einem der Ansprüche 1 bis 3, **dadurch gekennzeichnet, dass**
das Gewichtsverhältnis von Fluortensiden der Gruppe a) zu anderen Fluortensiden 100 : 1 bis 1 : 100, bevorzugt 50 : 1 bis 1 : 50, besonders bevorzugt 10 : 1 bis 1 : 10 und insbesondere bevorzugt 4 : 1 bis 1 : 5 beträgt,
und/oder das Gewichtsverhältnis von Silizium aufweisendem Tensid zu Fluortensid ausgewählt aus Gruppen a) 100 : 1 bis 1 : 100, bevorzugt 50 : 1 bis 1 : 50, besonders bevorzugt 10 : 1 bis 1 : 10 und insbesondere bevorzugt 5 : 1 bis 1 : 5 beträgt.

5. Zusammensetzung nach mindestens einem der vorhergehenden Ansprüche, **dadurch gekennzeichnet, dass**
diese eine durch Additionsreaktion vernetzende Organopolysiloxan-Mehrkomponenten Dentalabformmasse enthaltend Komponenten A und B ist, worin
a) Komponente A ein Organopolysiloxan mit mindestens zwei ethylenisch ungesättigten Gruppen und einen Hydrosilylierungskatalysator,
b) Komponente B ein Organohydrogenpolysiloxan, und
c) mindestens eine der Komponenten A und/oder B das Fluortensid ausgewählt aus einer der Gruppe a) in Kombination mit einem Silizium enthaltende Gruppen aufweisenden nichtionischen Tensid enthält,
und/oder diese eine durch Kondensationsreaktion vernetzende Organopolysiloxan-Mehrkomponenten Dentalabformmasse enthaltend Komponenten C und D ist, worin
i) Komponente C ein Organopolysiloxan mit mindestens zwei Hydroxylgrup pen,
j) Komponente D ein Kieselsäureester, Polykieselsäureester und/oder ein Organopolysiloxan mit mindestens zwei Alkoxygruppen sowie einen Kon densationskatalysator, und
k) mindestens eine der Komponenten C und/oder D das Fluortensid ausge wählt aus einer der Gruppe a) in Kombination mit einem Silizium enthaltende Gruppen aufweisenden nichtionischenTensid enthält,
und/oder diese eine durch Additionsreaktion vernetzende Alkenylreste enthaltende Polyether Mehrkomponenten Dentalabformmasse enthaltend Komponenten E und F ist, worin
l) Komponente E einen Vernetzungskatalysator,
m) Komponente F einen vernetzbaren und Alkenylreste aufweisenden Polyether sowie ein Organohydrogenpolysiloxan und/oder SiH-Polyether, und
n) mindestens eine der Komponenten E und/oder F das Fluortensid ausgewählt aus einer der Gruppe a) in Kombination mit einem Silizium enthaltende Gruppen aufweisenden nichtionischen Tensid enthält,
und/oder diese eine Alkoxysilylreste enthaltende Polyether Mehrkomponenten Dentalabformmasse enthaltend Komponenten G und H ist, worin
o) Komponente G einen vernetzbaren und Alkoxysilylreste aufweisenden Polyether,
p) Komponente H Wasser, und
q) mindestens eine der Komponenten G und/oder H einen Katalysator sowie das Fluortensid ausgewählt aus einer der Gruppe a) in Kombination mit einem Silizium enthaltende Gruppen aufweisenden nichtionischen Tensid enthält,
und/oder diese eine Mehrkomponenten-Dentalabformmasse enthaltend Komponenten I und J ist, worin
r) Komponente I einen vernetzbaren und Alkoxysilylreste oder Aziridinoreste aufweisenden Polyether oder einen über ringöffnende Metathesereaktion vernetzbare Gruppen enthaltenden Polyether,
s) Komponente J einen Katalysator, und
t) mindestens eine der Komponenten I und/oder J das Fluortensid ausgewählt aus einer der Gruppe a) in Kombination mit einem Silizium enthal- tende Gruppen aufweisenden nichtionischen Tensid enthält,
und/oder diese einen vernetzbaren Polyalkylenether, der von Acrylsäure und/oder Methacrylsäure abgeleitete Reste aufweist, einen chemisch oder durch Strahlung aktivierbaren Initiator sowie das Fluortensid ausgewählt aus der Gruppe a) in Kombination mit einem Silizium enthaltende Gruppen aufweisenden nichtionischen Tensid enthält,
und/oder diese eine über ringöffnende Metathese Polymerisation (ROMP) vernetzbare Gruppen aufweisende Polyether, Polysiloxane und/oder Synthesekautschuke enthaltende Polyether Mehrkomponenten- Dentalabform-masse enthaltend Komponenten K und L ist, worin
u) Komponente K Polyether, Polysiloxane und/oder Synthesekautschuke, die über ROMP vernetzbare Gruppen aufweisen,
v) Komponente L einen ROMP Vernetzungskatalysator, und
w) mindestens eine der Komponenten K und/oder L das Fluortensid ausgewählt aus der Gruppe a) in Kombination mit einem Silizium enthaltende Gruppen aufweisenden nichtionischenTensid enthält.

6. Zusammensetzung nach mindestens einem der vorhergehenden Ansprüche, **dadurch gekennzeichnet, dass** diese Füllstoffe enthält, vorzugsweise in einem Gesamtanteil von 0,01 bis 80 Gew.-%, besonders bevorzugt 0,05 bis 75 Gew.-%, und ganz besonders bevorzugt 0,1 bis 70 Gew.-%, bezogen auf die gesamte Zusammensetzung, und/oder diese ein oder mehrere der folgenden Zusatzstoffe enthält: Puffersalze, Wasserfänger, Pastenbildner, weitere Tenside, Wirkstoffe, Weich-macher, optische Abtastung ermöglichende Substanzen, Geschmacks- und/oder Geruchsstoffe, Diagnostik ermöglichende Substanzen, Fluoridisierungsmittel, Bleichsubstanzen, Desensibilisierungsmittel, Haftverbundvermittler, Farbstoffe, Indikatoren, Stabilisatoren (Antioxidantien) sowie antibakterielle Substanzen.

7. Zusammensetzung nach Anspruch 1, **dadurch gekennzeichnet, dass** diese
a) 10 bis 85 Gew.% Organopolydialkylsiloxan mit mindestens zwei Alkenylgruppen,
b) 1 bis 70 Gew.% Organohydrogenpolysiloxan mit einem SiH-Gehalt von 0,1 bis 15,0 mmol/g,
c) 0,0001 bis 2 Gew.% Hydrosilylierungskatalysator, insbesondere Salze, Komplexe und kolloidal vorliegende Formen der Übergangsmetalle der 8. Neben gruppe,
d) 0 bis 90 Gew.% nicht verstärkende Füllstoffe mit einer BET-Oberfläche kleiner 50 m²/g,
e) 0,1 bis 50 Gew.% verstärkende Füllstoffe mit einer BET-Oberfläche größer gleich 50 m²/g,
f) 0 bis 20 Gew.% Hilfsstoffe und Additive, wie Weichmacher, Farbstoffe, Stabilisatoren, Inhibitoren, Alkyl-verschlossene Fettalkoholethoxylate,
g) 0,01 bis 10,0 Gew.% eines mindestens einen (Poly)alkylenoxidrest sowie eine Silizium enthaltende Gruppe aufweisenden nichtionischen Tensids mit einer Molmasse von weniger als 6000 g/mol, das ein nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten ist, und
h) 0,001 bis 10,0 Gew.% des Fluortensids ausgewählt aus einer der Gruppe a) nach Anspruch 1,
enthält,
und wobei diese optional zusätzlich i) 0,1 bis 25 Gew.% verzweigten oder linearen Alkyl-, Hydroxy-, Alkinyl- und/oder Alkenyl-endgestoppten Polyalkylenether und/oder deren Mischungen enthält, wobei das Gewichtsverhältnis von Organopolysiloxan a) zum Polyether i) bevorzugt 1 : 1 bis 80 : 1, besonders bevorzugt 1 : 1 bis 60 : 1, ganz besonders bevorzugt 1 : 1 bis 40 : 1 und insbesondere bevorzugt 1 : 1 bis 30 : 1 ist.

8. Zusammensetzung nach Anspruch 7, **dadurch gekennzeichnet, dass** diese eine Zweikomponenten-Dentalabformmasse bestehend aus Komponente A und B ist,
worin Komponente A
a) 10 bis 80 Gew.% Organopolydialkylsiloxan mit mindestens zwei Alkenylgruppen,
c) 0,0001 bis 2 Gew.% Hydrosilylierungskatalysator, insbesondere Salze, Komplexe und kolloidal vorliegende Formen der Übergangsmetalle der 8. Nebengruppe,
d) 0 bis 90 Gew.% nicht verstärkende Füllstoffe mit einer BET-Oberfläche kleiner 50 m²/kg,
e) 0,1 bis 50 Gew.% verstärkende Füllstoffe mit einer BET-Oberfläche größer gleich 50 m²/kg,
f) 0 bis 20 Gew.% Hilfsstoffe und Additive, wie Weichmacher, Farbstoffe, Stabilisatoren und/oder Inhibitoren, und
j) 0,001 bis 5,0 Gew.% Alkyl-, Aryl-, Aralkyl-verschlossene nichtionische Tenside, bevorzugt Alkyl-verschlossene Fettalkoholethoxylate, Silicontenside, Polyethercarbosilane, Carbosilantenside und Fluortenside, die Alkyl-verschlossen sind und insbesondere Alkyl-verschlossene Fettalkoholethoxylate,
und Komponente B enthält,
a) 0,1 bis 70 Gew.% Organopolydialkylsiloxan mit mindestens zwei Alkenylgruppen,
b) 1,2 bis 80 Gew.% Organohydrogenpolysiloxan mit einem SiH-Gehalt von 0,1 bis 15,0 mmol/kg,
d) 0 bis 90 Gew.% nicht verstärkende Füllstoffe mit einer BET-Oberfläche kleiner 50 m²/kg,
e) 0,1 bis 50 Gew.% verstärkende Füllstoffe mit einer BET-Oberfläche größer gleich 50 m²/kg,
f) 0 bis 20 Gew.% Hilfsstoffe und Additive, wie Weichmacher, Farbstoffe, Stabilisatoren und/oder Inhibitoren,
g) 0,01 bis 10,0 Gew.% eines mindestens einen (Poly)alkylenoxidrest sowie eine Silizium enthaltende Gruppe aufweisenden nichtionischen Tensids mit einer Molmasse von weniger als 6000 g/mol, das ein nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten ist,
h) 0,001 bis 10,0 Gew % des mindestens einen Fluortensids ausgewählt aus einer der Gruppe a) nach Anspruch 1 enthält, wobei das Verhältnis der Tenside g) und h) 25 : 1 bis 1: 25, bevorzugt 20 : 1 bis 1 : 5, besonders bevorzugt 10 : 1 bis 1 : 5 und insbesondere bevorzugt 5 : 1 bis 1 : 3 ist, und
i) 0,5 bis 50 Gew.% verzweigten oder linearen Alkyl- und/oder Alkinyl- und/oder Alkenyl- und/oder Hydroxyl-endgestoppten Polyalkylenether und/oder deren Mischungen, wobei das Gewichtsverhältnis von Organopolysiloxan a) zum Polyether i) bevorzugt 1 : 50 bis 50 : 1, besonders bevorzugt 10 : 1 bis 1 : 10 und insbesondere bevorzugt 4 : 1 bis 1 : 5 ist.

9. Zusammensetzung nach Anspruch 8, **dadurch gekennzeichnet, dass** diese eine Zweikomponenten-Dentalabformmasse bestehend aus Komponente A und B ist, worin Komponente A enthält
b) 10 bis 80 Gew.% Organopolydialkylsiloxan mit mindestens zwei Alkenylgruppen,
c) 0,01 bis 2 Gew.% Hydrosilylierungskatalysator, insbesondere Salze, Komplexe und kolloidal vorliegende Formen der Übergangsmetalle der 8. Nebengruppe,
d) 0 bis 90 Gew.% nicht verstärkende Füllstoffe mit einer BET-Oberfläche kleiner 50 m²/kg,
e) 0,1 bis 50 Gew.% verstärkende Füllstoffe mit einer BET-Oberfläche größer gleich 50 m²/kg,
f) 0 bis 20 Gew.% Hilfsstoffe und Additive, wie Weichmacher, Farbstoffe, Stabilisatoren und/oder Inhibitoren, und
j) 0,001 bis 5,0 Gew.% Alkyl-, Aryl-, Aralkyl-verschlossene nichtionische Tenside, bevorzugt Alkyl-verschlossene Fettalkoholethoxylate, Silicontenside, Polyethercarbosilane, Carbosilantenside und Fluortenside, die Alkyl-verschlossen sind und insbesondere Alkyl-verschlossene Fettalkoholethoxylate,
und Komponente B enthält
c) 0,1 bis 70 Gew.% Organopolydialkylsiloxan mit mindestens zwei Alkenylgruppen,
d) 1,2 bis 80 Gew.% Organohydrogenpolysiloxan mit einem SiH-Gehalt von 0,1 bis 15,0 mmol/kg,
e) 0 bis 90 Gew.% nicht verstärkende Füllstoffe mit einer BET-Oberfläche kleiner 50 m²/kg,
f) 0,1 bis 50 Gew.% verstärkende Füllstoffe mit einer BET-Oberfläche größer gleich 50 m²/kg,
g) 0 bis 20 Gew.% Hilfsstoffe und Additive, wie Weichmacher, Farbstoffe, Stabilisatoren und/oder Inhibitoren,
h) 0,01 bis 10,0 Gew.% nichtionisches Polyethersiloxantensid mit 3 bis 7 Silziumatomen in der Siloxanteilstruktur und/oder nichtionisches Polyethercarbosilantensid mit 1 bis 7 Silziumatomen in der Carbosilanteilstruktur und einem Alkylenoxidanteil von 1 bis 20 Einheiten,
j) 0,001 bis 10,0 Gew % des Fluortensids ausgewählt aus einer der Gruppe a) nach Anspruch 1, und
k) 0,5 bis 50 Gew.% Polyol und/oder Mischungen von Polyolen, wobei das Gewichtsverhältnis von Organopolysiloxan a) zum Polyol k) bevorzugt 1 : 50 bis 50 : 1, besonders bevorzugt 10 : 1 bis 1 : 10 und insbesondere bevorzugt 4 : 1 bis 1 : 5 ist.

10. Zusammensetzung nach mindestens einem der vorhergehenden Ansprüche, **dadurch gekennzeichnet, dass**
diese 40 Sekunden nach Mischbeginn einen gemäß der Beschreibung gemessenen niedrigen initialen Wassertropfen-Kontaktwinkel von < 10° aufweist, gemessen bei einem Tropfenalter von 10 Sekunden,
insbesondere der Wassertropfen-Kontaktwinkel 40 Sekunden nach Mischbeginn folgende Werte annimmt: nach einem Tropfenalter von 0,25 Sekunden einen Wassertropfen-Kontaktwinkel von <75°, vorzugsweise von <40°; nach einem Tropfenalter von 0,5 Sekunden einen Wassertropfen-Kontaktwinkel von <55°, vorzugsweise <30°; nach einem Tropfenalter von 1 Sekunde einen Wassertropfen-Kontaktwinkel von <35°, vorzugsweise < 25°; nach einem Tropfenalter von 2 Sekunden: einen Wassertropfen-Kontaktwinkel von <20°; und nach einem Tropfenalter von 3 Sekunden einen Wassertropfen-Kontaktwinkel von <10° .

11. Mischungen erhältlich durch Vermischen der Zusammensetzungen nach mindestens einem der vorhergehenden Ansprüche.

12. Ausgehärtetes Abformmaterial erhältlich durch Aushärten der Zusammensetzung nach mindestens einem der Ansprüche 1 bis 10.

13. Verwendung eines Fluortensids ausgewählt aus der Gruppe a) mit mindestens einem Silizium aufweisenden nichtionischen Tensid, das mindestens einen (Poly)alkylenoxidrest und eine Molmasse von weniger als 6.000 g/mol aufweist, gemäß Anspruch 1, gegebenenfalls in Kombination mit mindestens einem weiteren Fluortensid zur Herstellung von Dentalmassen, insbesondere von Dentalabformmassen.

14. Verwendung einer Zusammensetzung gemäß einem der Ansprüche 1 bis 10 zur Herstellung von Dentalmassen, insbesondere von Dentalabformmassen.

## Claims

1. A composition containing curable polymers selected from the group of organopolysiloxanes that crosslink by means of an addition reaction, organopolysiloxanes that crosslink by means of a condensation reaction, polyethers that contain alkoxysilyl groups and crosslink by means of a condensation reaction, polyethers that contain aziridino groups and crosslink by means of an addition reaction, polyethers that contain alkenyl groups and crosslink by means of an addition reaction, polyethers that contain ester groups of an ethylenically unsaturated carboxylic acid and crosslink by means of a radical polymerization reaction, or polyethers, silicones or rubbers that crosslink by means of a ring-opening metathesis reaction
and also contain at least one nonionic fluorosurfactant, selected from the group of
a) fluorosurfactants, which have at least 2 partially fluorinated or perfluorinated alkylene oxide or (poly)alkylene oxide units,
as well as also containing at least one nonionic surfactant having silicon-containing groups with a molecular weight of less than 6000 g/mol, wherein the fluorosurfactant of group a) is a block copolymer containing blocks of formula Ic
B-[O-R_{H}]ₑ-A-[O-R_{F}]ₐ-A'-[O-R'_{H}]_{d}-B' (Ic),
where
R_{F} denotes a group of formula -CₘFₙHₒ-, where the indices m, n and o may be different within one polyether group within the scope of the given definitions,
m denotes integers from 2 to 12,
n denotes integers from 1 to 24,
o denotes integers from 0 to 23,
where the sum of n and o corresponds to the value of 2m,
R_{H} is a group of the formula -CₚH₂ₚ-, where the index p may be different in one polyether group within the scope of the given definition,
R'_{H} is a group of formula -C_{q}H_{2q}-, where the index q may be different within the scope of the given definition in one polyether group,
p is an integer from 2 to 12, preferably from 2 to 4,
q is an integer from 2 to 12, preferably from 2 to 4,
A and A', independently of one another, denote a covalent bond or divalent bridge group, which is linked to the blocks [O-R_{F}], [O-R_{H}] and [O-R'_{H}] via C-C and/or C-O bonds,
B and B', independently of one another, denote hydrogen, a partially fluorinated or perfluorinated alkyl group with 1 to 6 carbon atoms or an alkyl group with 1 to 6 carbon atoms, a is an integer from 1 to 100, preferably 2 to 50,
a is an integer from 1 to 100, preferably 2 to 50,
d is an integer from 1 to 100, preferably 2 to 50 and
e is an integer from 1 to 100, preferably 2 to 50,
or that
the fluorosurfactant of group a) is a compound of formula Ica
HO-(CH₂CH₂O)ₙₐ-CH₂CF₂O-(CF₂CF₂O)ₚₐ-(CF₂O)_{qa}-CF₂CH₂-(OCH₂CH₂)ₙₐ-OH (Ica),
where na is an integer from 1 to 20,
pa is an integer from 0 to 12, and
qa is an integer from 0 to 20,
with the provision that the sum of pa and qa must be at least 1,
and that in addition to containing the fluorosurfactant from group a), other ionic and/or nonionic and/or amphoteric fluorosurfactants are contained.

2. The composition according to at least one of the preceding claims, **characterized in that** the silicon-containing nonionic surfactant containing at least one (poly)alkylene oxide group has a molecular weight of less than 4000 g/mol, in particular 350 to 2000 g/mol
and/or the silicon-containing nonionic surfactant is an organosiloxane surfactant of formula II and/or formula III or an organocarbosilane surfactant of formulas IV, V and/or VI 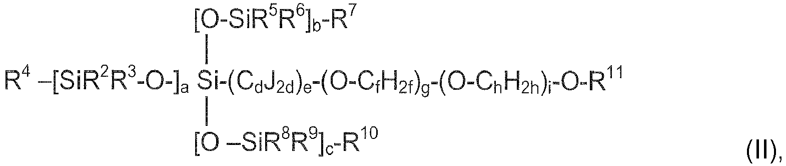 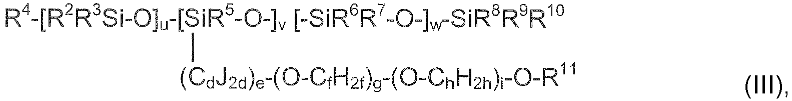 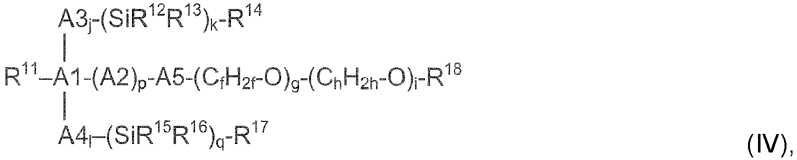 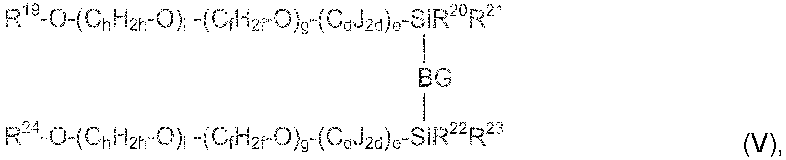
R¹⁹-O-(CₕH₂ₕ-O)ᵢ-(C_{f}H_{2f}-O)_{g}-(C_{d}J_{2d})ₑ-SiR²⁰R²¹R²² (VI),
where R², R³, R⁴, R⁵, R⁶, R⁷, R⁸, R⁹ and R¹⁰, independently of one another, denote hydrogen, alkyl, alkyloxy, alkenyl, alkenyloxy, alkynyl, alkynyloxy, aryl, aryloxy, aralkyl, aralkyloxy, alkylaryl and/or alkylaryloxy, which are optionally either partially or completely fluorinated, preferably alkyl or alkenyl and especially C₁-C₆ alkyl,
a, b, c and w, independently of one another, are integers from 0 to 100, preferably 0 to 75, in particular 0 to 35, and most especially preferably 0 to 15,
v is an integer from 1 to 100, preferably 1 to 15, and most especially preferably 1 to 6,
where the sum of a, b and c is between 1 and 300, preferably 1 to 50, especially 1 to 10, and most especially preferably 1 to 3,
and the sum of v and w is between 1 and 200, preferably 2 to 90,
u is 0 or 1,
d is an integer from 1 to 10, preferably 1 to 6 and especially 1 to 3,
J is hydrogen or fluorine, preferably hydrogen,
e is 0 or 1,
f and h, independently of one another, are integers from 2 to 6,
g and i, independently of one another, are integers from 0 to 30, preferably 0 to 15, where the sum of g and i is 1 to 60, preferably 2 to 30, especially 2 to 15,
R¹¹ is hydrogen, alkyl, alkenyl or aryl, optionally partially or completely fluorinated, preferably hydrogen or methyl,
R¹², R¹³, R¹⁴, R¹⁵, R¹⁶ and R¹⁷, independently of one another, denote hydrogen, alkyl, alkyloxy, alkenyl, alkenyloxy, alkynyl, alkynyloxy, aryl, aryloxy, aralkyl, aralkyloxy, alkylaryl and/or alkylaryloxy, which are optionally partially or completely fluorinated, preferably alkyl or alkenyl and especially C₁-C₆ alkyl,
k and q independently of one another are 0 or 1,
A1 is carbon or silicon,
A2, A3 and A4 independently of one another are a group C_{d}J_{2d}, where J and d have the meanings defined above,
j, p and l, independently of one another, are 0 or 1,
A5 is a divalent bridge group, especially -O-, -CO-O- or -CO-,
R¹⁸ is hydrogen, alkyl, alkenyl or aryl, optionally partially or completely fluorinated, preferably hydrogen or methyl,
R²⁰, R²¹, R²² and R²³, independently of one another, denote hydrogen, alkyl, alkyloxy, alkenyl, alkenyloxy, alkynyl, alkynyloxy, aryl, aryloxy, aralkyl, aralkyloxy, alkylaryl and/or alkylaryloxy, optionally partially or completely fluorinated, preferably alkyl or alkenyl and especially C₁-C₆ alkyl,
BG is a divalent bridge group, and
R¹⁹ and R²⁴ independently of one another denote hydrogen, alkyl, alkenyl or aryl, optionally partially or completely fluorinated, preferably hydrogen or methyl, with the provision that of the groups R², R³ and R⁴ and/or the groups R⁵, R⁶ and R⁷ and/or the groups R⁸, R⁹ and R¹⁰ and/or the groups R¹⁵, R¹⁶ and R¹⁷ and/or the groups R²⁰ and R²¹ and/or the groups R²² and R²³ and/or the groups R²⁰, R²¹ and R²² only one can be hydrogen,
where f and h may assume different values within one molecule within the scope of the given definition,
or the silicon-containing nonionic surfactant containing at least one (poly)alkylene oxide group is a compound of formulas VII, VIII, IX or X 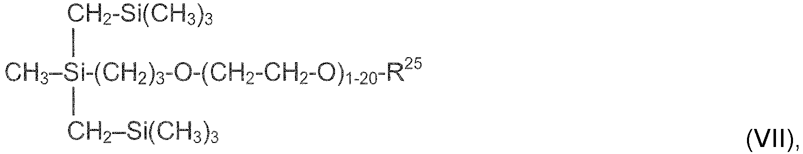 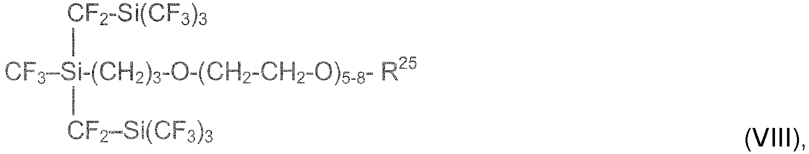 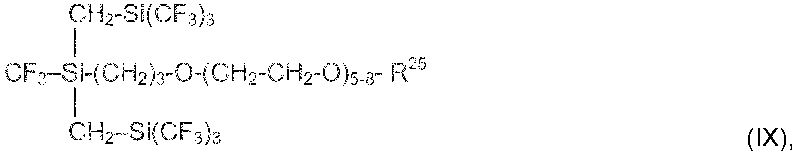 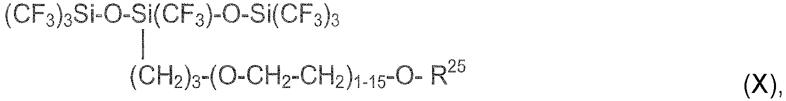 where R²⁵ is hydrogen, methyl, ethyl, propyl or butyl, preferably hydrogen or methyl.

3. The composition according to at least one of the preceding claims, **characterized in that** in addition to containing the at least one fluorosurfactant of group a), it also contains as an additional component a polyether containing alkenyl groups and/or alkynyl groups and/or a hydroxyl- and/or aryloxy- and/or arylalkoxy- and/or alkoxy-terminated polyether, wherein preferably the polyether containing the alkenyl groups is a compound of formula XII, and the hydroxyl-terminated and/or alkoxy-terminated polyether is a compound of formula XIII
CH₂=CH-CH₂-O-(Cₙ₂H₂ₙ₂-O)ₘ₉-CH₂-CH=CH₂ (XII),
R³¹-O-(Cₙ₂H₂ₙ₂-O)ₘ₉-R³² (XIII),
where n2 denotes an integer from 2 to 8, preferably from 2 to 4,
m9 is an integer from 3 to 70,000, preferably from 10 to 2500,
R³¹ and R³², independently of one another, denote hydrogen or C₁-C₆ alkyl, in particular hydrogen and/or methyl, ethyl or propyl, and
where R³¹, R³², n2 and m9 may be different within one molecule within the scope of the given definition,
and/or that, in addition to the at least one fluorosurfactant of groups a), it also contains a polyol as an additional component, wherein preferably the polyol is selected from the group of carbohydrates, polyvinyl alcohols, aliphatic diols, triols, tetraols, pentaols and/or hexanols and mixtures of two or more of these polyols or the polyol is selected from the group of polyvinyl alcohols, polysaccharides, trimethylol propane, pentaerythritol, dipentaerythritol, glycerol, allyloxy-1,2-propanediol, 2-methyl-2,4-pentanediol, trimethylolpropane allyl ether, decanediol, nonanediol, octanediol, heptanediol, hexanediol, pentanediol, butanediol, propanediol, ethanediol, fructose, glucose and mixtures of two or more of these polyols, in particular glycerol.

4. The composition according to any of claims 1 to 3, **characterized in that** the weight ratio of fluorosurfactants of group a) to other fluorosurfactants is 100:1 to 1:100, preferably 50:1 to 1:50, especially preferably 10:1 to 1:10, and in particular preferably 4:1 to 1:5,
and/or the weight ratio of silicon-containing surfactant to fluorosurfactant selected from group a), is 100:1 to 1:100, preferably 50:1 to 1:50, especially preferably 10:1 to 1:10 and in particular preferably 5:1 to 1:5.

5. The composition according to at least one of the preceding claims, **characterized in that** it is an organopolysiloxane multicomponent dental impression compound that crosslinks by means of an addition reaction and contains components A and B, wherein
a) component A contains an organopolysiloxane with at least 2 ethylenically unsaturated groups and a hydrosilylation catalyst,
b) component B contains an organohydrogen polysiloxane, and
c) at least one of components A and/or B contains the fluorosurfactant selected from one of the group a) in combination with a nonionic surfactant having silicon-containing groups,
and/or it is an organopolysiloxane multicomponent dental impression compound that crosslinks by means of a condensation reaction and contains components C and D, wherein
i) component C contains an organopolysiloxane with at least 2 hydroxyl groups,
j) component D contains a silicic acid ester, polysilicic acid ester and/or an organopolysiloxane with at least 2 alkoxy groups and a condensation catalyst, and
k) at least one of components C and/or D contains the fluorosurfactant of group a) in combination with a nonionic surfactant having silicon-containing groups.
and/or it is a polyether multicomponent dental impression compound that crosslinks by means of an addition reaction and contains alkenyl groups and components E and F, wherein
l) component E contains a crosslinking catalyst,
m) component F contains a crosslinkable polyether having alkenyl groups and an organohydrogen polysiloxane and/or SiH polyether, and
n) at least one of components E and/or F contains the fluorosurfactant of groups a) in combination with a nonionic surfactant having silicon-containing groups.
and/or it is a polyether multicomponent dental impression compound containing alkoxysilyl groups and components G and H, wherein
o) component G contains a crosslinkable polyether having alkoxysilyl groups,
p) component H contains water, and
q) at least one of components G and/or H contains a catalyst as well as the fluorosurfactant of group a) in combination with a nonionic surfactant having silicon-containing groups.
and/or it is a multicomponent dental impression compound containing components I and J, wherein
r) component I contains a crosslinkable polyether having alkoxysilyl or aziridino groups groups or a polyether having groups crosslinkable by means of a ring-opening metathesis reaction,
s) component J contains a catalyst, and
t) at least one of components I and/or J contains a fluorosurfactant of group a) in combination with a nonionic surfactant having silicon-containing groups.
and/or it has a crosslinkable polyalkylene ether, which contains groups derived from acrylic acid and/or methacrylic acid, a chemically or radiation activatable initiator, and the fluorosurfactant is selected from one of the group a) in combination with a nonionic surfactant having silicon-containing groups,
and/or it is a polyether multicomponent dental impression compound containing polyethers, polysiloxanes and/or synthetic rubbers having groups that are crosslinkable by means of a ring-opening metathesis polymerization (ROMP) and containing components K and L, wherein
u) component K contains polyethers, polysiloxanes and/or synthetic rubbers having groups crosslinkable via ROMP,
v) component L contains a ROMP-crosslinking catalyst, and
w) at least one of the components K and/or L contains the fluorosurfactant of group a) in combination with a nonionic surfactant having silicon-containing groups.

6. The composition according to at least one of the preceding claims, **characterized in that** it contains fillers, preferably in a total amount of 0.01 to 80 wt%, especially preferably 0.05 to 75 wt% and most especially preferably 0.1 to 70 wt%, based on the total composition and/or it contains one or more of the following additives: buffer salts, water scavengers, paste forming agents, additional surfactants, active ingredients, plasticizers, optical scan-enabling substances, taste and/or odor substances, diagnostics-enabling substances, fluoridation agents, bleach substances, desensitizers, adhesion promoters, dyes, indicators, stabilizers (antioxidants) and antibacterial substances.

7. The composition according to claim 1, **characterized in that** it contains
a) 10 wt% to 85 wt% organopolydialkylsiloxane with at least 2 alkenyl groups,
b) 1 to 70 wt% organohydrogen polysiloxane with an SiH content of 0.1 to 15.0 mmol/g,
c) 0.0001 to 2 wt% hydrosilylation catalyst, especially salts, complexes and colloidal forms of the transition metals of side group 8,
d) 0 to 90 wt% nonreinforcing fillers with a BET surface area of less than 50 m²/g,
e) 0.1 to 50 wt% reinforcing fillers with a BET surface area equal to or greater than 50 m²/g,
f) 0 to 20 wt% auxiliaries and additives, such as plasticizers, dyes, stabilizers, inhibitors, alkyl-capped fatty alcohol ethoxylates,
g) 0.01 to 10.0 wt% of a nonionic surfactant having at least one (poly)alkylene oxide group and a silicon-containing group and having a molecular weight of less than 6000 g/mol, which is a nonionic polyether siloxane surfactant with 3 to 7 silicon atoms in the siloxane substructure and/or nonionic polyether carbosilane surfactant with 1 to 7 silicon atoms in the carbosilane substructure and an alkylene oxide content of 1 to 20 units, and
h) 0.001 to 10.0 wt% of the at least one fluorosurfactant of group a) according to claim 1,
and wherein it optionally additionally contains i) 0.1 to 25 wt% branched or linear alkyl-, hydroxy-, alkynyl- and/or alkenyl-terminated polyalkylene ethers and/or mixtures thereof, where the weight ratio of organopolysiloxanes a) to polyether i) is preferably 1:1 to 80:1, especially preferably 1:1 to 60:1, most especially preferably 1:1 to 40:1 and in particular preferably 1:1 to 30:1.

8. The composition according to claim 7, **characterized in that** it is a two-component dental impression compound consisting of components A and B, where component A contains
a) 10 to 80 wt% organopolydialkylsiloxane with at least 2 alkenyl groups,
c) 0.0001 to 2 wt% hydrosilylation catalyst, in particular salts, complexes and colloidal forms of the transition metals of side group 8,
d) 0 to 90 wt% nonreinforcing fillers with a BET surface area of less than 50 m²/kg,
e) 0.1 to 50 wt% reinforcing fillers with a BET surface area of greater than or equal to 50 m²/kg,
f) 0 to 20 wt% auxiliaries and additives, such as plasticizers, dyes, stabilizers and/or inhibitors, and
j) 0.001 to 5.0 wt% alkyl-, aryl- or aralkyl-capped nonionic surfactants, preferably alkyl-capped fatty alcohol ethoxylates, silicone surfactants, polyether carbosilanes, carbosilane surfactants and fluorosurfactants, which are alkyl-capped, and especially alkyl-capped fatty alcohol ethoxylates,
and component B contains
a) 0.1 to 70 wt% organopolydialkylsiloxane with at least 2 alkenyl groups,
b) 1.2 to 80 wt% organohydrogen polysiloxane with an SiH content of 0.1 to 15.0 mmol/g,
d) 0 to 90 wt% nonreinforcing fillers with a BET surface area of less than 50 m²/kg,
e) 0.1 to 50 wt% reinforcing fillers with a BET surface area greater than or equal to 50 m²/kg,
f) 0 to 20 wt% auxiliaries and additives, such as plasticizers, dyes, stabilizers and/or inhibitors,
g) 0.01 to 10.0 wt% of a nonionic surfactant containing at least one (poly)alkylene oxide group and one silicon-containing group and having a molecular weight of less than 6000 g/mol, which is a nonionic polyether siloxane surfactant with 3 to 7 silicon atoms in the siloxane substructure and/or a nonionic polyether carbosilane surfactant with 1 to 7 silicon atoms in the carbosilane substructure and an alkylene oxide content of 1 to 20 units,
h) 0.001 to 10.0 wt% of the at least one fluorosurfactants of group a) through h) according to claim 1, where the ratio of the surfactants g) and h) is preferably 25:1 to 1:25, especially preferably 20:1 to 1:5, most especially preferably 10:1 to 1:5 and in particular preferably 5:1 to 1:3, and
i) 0.5 to 50 wt% branched or linear alkyl or alkynyl and/or alkenyl and/or hydroxy-terminated (poly)alkylene ethers and/or mixtures thereof, where the weight ratio of organopolysiloxane a) to polyether i) is 1:50 to 50:1, preferably 10:1 to 1:10 and in particular preferably 4:1 to 1:5.

9. The composition according to claim 8, **characterized in that** it is a two-component dental impression compound, consisting of components A and B, where component A contains
a) 10 to 80 wt% organopolydialkylsiloxane with at least 2 alkenyl groups,
c) 0.01 to 2 wt% hydrosilylation catalyst, in particular salts, complexes and colloidal forms of the transition metals of side group 8,
d) 0 to 90 wt% nonreinforcing fillers with a BET surface area of less than 50 m²/kg,
e) 0.1 to 50 wt% reinforcing fillers with a BET surface area greater than or equal to 50 m²/kg,
f) 0 to 20 wt% auxiliaries and additives, such as plasticizers, dyes, stabilizers and/or inhibitors, and
j) 0.001 to 5.0 wt% alkyl-, aryl- or aralkyl-capped nonionic surfactants, preferably alkyl-capped fatty alcohol ethoxylates, silicone surfactants, polyether carbosilanes, carbosilane surfactants and fluorosurfactants, which are alkyl-capped and in particular alkyl-capped fatty alcohol ethoxylates,
and component B contains
a) 0.1 to 70 wt% organopolydialkylsiloxane with at least 2 alkenyl groups,
b) 1.2 to 80 wt% organohydrogen polysiloxane with an SiH content of 0.1 to 15.0 mmol/g,
e) 0 to 90 wt% nonreinforcing fillers with a BET surface area of less than 50 m²/kg,
f) 0.1 to 50 wt% reinforcing fillers with a BET surface area greater than or equal to 50 m²/kg,
g) 0 to 20 wt% auxiliaries and additives, such as plasticizers, dyes, stabilizers and/or inhibitors,
h) 0.01 to 10.0 wt% nonionic polyether siloxane surfactant with 3 to 7 silicon atoms in the siloxane substructure and/or nonionic polyether carbosilane surfactant with 1 to 7 silicon atoms in the carbosilane substructure and an alkylene oxide content of 1 to 20 units,
i) 0.001 to 10.0 wt% of the fluorosurfactant, selected from one of groups a) through h) according to claim 1, where the ratio of surfactants g) and h) is preferably 25:1 to 1:25, especially preferably 20:1 to 1:5, most especially preferably 10:1 to 1:5 and in particular preferably 5:1 to 1:3,
j) 0.001 to 10.0 wt% of the at least one fluorosurfactant, selected from one of group a) according to claim 1, and
k) 0.5 to 50 wt% polyol or mixtures of polyols, where the weight ratio of organopolysiloxane a) to polyol k) is preferably 1:50 to 50:1, especially preferably 10:1 to 1:10 and in particular preferably 4:1 to 1:5.

10. The composition according to at least one of the preceding claims, **characterized in that** has a low initial water droplet contact angle of < 10° measured at a droplet age of 10 seconds, 40 seconds after the start of mixing, particularly that the water droplet contact angle 40 seconds after the start of mixing assumes the following values: after a droplet age of 0.25 second, a water droplet contact angle of < 75°, preferably < 40°; after a droplet age of 0.5 second, a water droplet contact angle of < 55°, preferably < 30°; after a droplet age of 1 second, a water droplet contact angle of < 35°, preferably < 25°; after a droplet age of 2 seconds, a water droplet contact angle of < 20°; and after a droplet age of 3 seconds, a water droplet contact angle of < 10°.

11. The mixtures obtainable by mixing the compositions according to at least one of the preceding claims.

12. A curable impression material obtainable by curing the composition according to at least one of claims 1 to 10.

13. Use of a fluorosurfactant of group a) according to claim 1 with at least one silicon-containing nonionic surfactant having at least one (poly)alkylene oxide group and a molecular weight of less than 6000 g/mol, optionally in combination with at least one additional fluorosurfactant, for production of dental compounds, in particular dental impression compounds.

14. Use of a composition according to any one of claims 1 to 10 for production of dental compounds, in particular dental impression compounds.

## Revendications

1. Composition contenant des polymères durcissables, choisis dans le groupe des organopolysiloxanes subissant une réticulation par réaction d'addition, des organopolysiloxanes subissant une réticulation par réaction de condensation, des polyéthers contenant des radicaux alcoxysilyles subissant une réticulation par condensation, des polyéthers contenant des radicaux aziridino subissant une réticulation par addition, des polyéthers contenant des radicaux alcényle subissant une réticulation par addition, des polyéthers contenant des radicaux ester qui appartiennent à un acide carboxylique éthyléniquement insaturé et qui subissent une réticulation par réaction de polymérisation radicalaire ou des polyéthers, silicones ou caoutchoucs subissant une réticulation par réaction de métathèse impliquant une ouverture de cycle, et
contenant en outre au moins un agent tensioactif fluoré non-ionique choisi dans le groupe
a) des agents tensioactifs fluorés comportant au moins 2 unités d'oxyde d'alkylène ou de poly(oxyde d'alkylène) partiellement fluorées ou perfluorées,
et contenant au moins un agent tensioactif non-ionique qui comporte des groupes contenant du silicium et qui a une masse moléculaire inférieure à 6 000 g/mol, l'agent tensioactif fluoré du groupe a) étant un copolymère en blocs contenant des blocs selon la formule Ic
B[O-R_{H}]ₑ-A-[O-Rd_{F}]ₐ-A'-[O-R'_{H}]_{d}-B' (Ic)
dans laquelle
R_{F} est un radical selon la formule -CₘFₙHₒ-, les indices m, n, o pouvant, au sein d'un radical polyéther, être différents dans le cadre des définitions indiquées,
m est un nombre entier compris entre 2 et 12,
n est un nombre entier compris entre 1 et 24,
o est un nombre entier compris entre 0 et 23,
la somme de n et o correspondant à la valeur 2m,
R_{H} est un radical selon la formule -CₚH₂ₚ-, l'index p pouvant, au sein d'un radical polyéther, être différent dans le cadre de la définition indiquée,
R'_{H} est un radical selon la formule -C_{q}H_{2q}-, l'index q pouvant, au sein d'un radical polyéther, être différent dans le cadre de la définition indiquée,
p signifie un nombre entier compris entre 2 et 12, de préférence entre 2 et 4,
q signifie un nombre entier compris entre 2 et 12, de préférence entre 2 et 4,
A et A' signifient, indépendamment l'un de l'autre, une liaison covalente ou un groupe de liaison divalent qui est relié, au travers de liaisons C-C et/ou de liaison C-O, aux blocs [O-R_{F}], [O-R_{H}] et [O-R'_{H}],
B et B' sont, indépendamment l'un de l'autre, de l'hydrogène, un radical alkyle partiellement fluoré ou perfluoré renfermant 1 à 6 atomes de carbone ou un radical alkyle renfermant 1 à 6 atomes de carbone,
a est un nombre entier compris entre 2 et 100, de préférence entre 2 et 50,
d est un nombre entier compris entre 1 et 100, de préférence entre 2 et 50, et
e est un nombre entier compris entre 1 et 100, de préférence entre 2 et 50
ou bien que
l'agent tensioactif fluoré du groupe a) est un composé selon la formule Ica
HO⁻(CH₂CH₂O)ₙₐ-CH₂CF₂O-(CF₂CF₂O)ₚₐ-(CF₂O)_{qa}-CF₂CH₂-(OCH₂CH₂)ₙₐ-OH (Ica),
dans laquelle na est un nombre entier compris entre 1 et 20,
pa est un nombre entier compris entre 0 et 12, et
qa signifie un nombre entier compris entre 0 et 20,
à condition que la somme de pa et qa soit au moins égale à 1,
et qu'elle contient, outre l'agent tensioactif fluoré issu du groupe a), d'autres agents tensioactifs fluorés ioniques et/ou non-ioniques et/ou amphotères.

2. Composition selon l'une des revendications précédentes, **caractérisée en ce que**
l'agent tensioactif non-ionique qui comporte du silicium et qui contient au moins un radical poly(oxyde d'alkylène) possède une masse moléculaire inférieure à 4 000 g/mol, comprise notamment entre 350 et 2 000 g/mol,
et/ou l'agent tensioactif non-ionique comportant du silicium est un agent tensioactif de type organosiloxane selon la formule II et/ou la formule III, ou bien un agent tensioactif de type silane organocarboné selon la formule IV, V et/ou la formule VI, 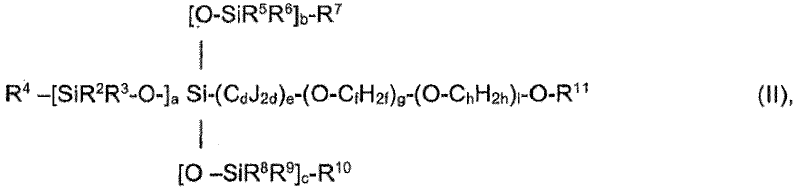 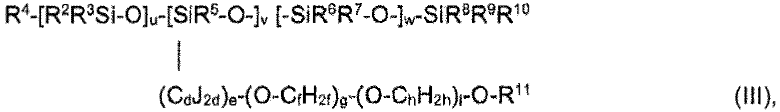 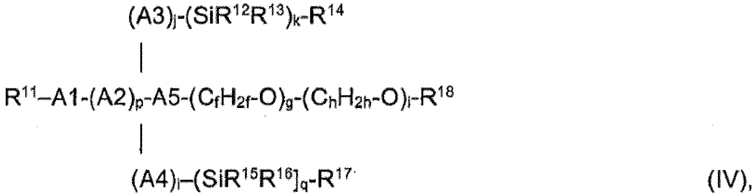 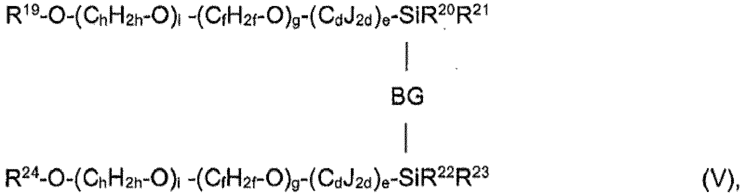
R¹⁹-O-(CₕH₂ₕ-O)ₗ-(C_{f}H_{2f}(-O)₉-(C_{d}J_{2d})ₑ-SiR²⁰R²¹R²² (VI),
dans lesquelles R², R³, R⁴, R⁵, R⁶, R⁷, R⁸, R⁹ et R¹⁰ signifient, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant partiellement ou complètement fluorés, s'agissant préférentiellement d'alkyle ou d'alcényle et notamment d'alkyle en C₁-C₆,
a, b, c et w signifient, indépendamment l'un de l'autre, des nombres entiers compris entre 0 et 100, de préférence entre 0 et 75, notamment entre 0 et 35 et, avec une préférence particulière, entre 0 et 15,
v signifie un nombre entier compris entre 1 et 100, de préférence entre 1 et 15 et, avec une préférence particulière, entre 1 et 6,
la somme de a, b et c étant comprise entre 1 et 300, de préférence entre 1 et 50, notamment entre 1 et 10 et, avec une préférence particulière, entre 1 et 3,
et la somme de v et w étant comprise entre 1 et 200, de préférence entre 2 et 90,
u est 0 ou 1,
d est un nombre entier compris entre 1 et 10, de préférence entre 1 et 6 et notamment entre 1 et 3,
J signifie l'hydrogène ou le fluor, de préférence l'hydrogène,
e est 0 ou 1,
f et h signifient, indépendamment l'un de l'autre, des nombres entiers compris entre 2 et 6,
g et i sont, indépendamment l'un de l'autre, des nombres entiers compris entre 0 et 30, de préférence entre 0 et 15, la somme de g et i signifiant 1 à 60, de préférence 2 à 30, notamment 2 à 15,
R¹¹ est l'hydrogène, alkyle, alcényle ou aryle, lesquels sont le cas échéant partiellement ou complètement fluorés, s'agissant préférentiellement d'hydrogène ou de méthyle,
R¹², R¹³, R¹⁴, R¹⁵, R¹⁶ et R¹⁷ signifient, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant partiellement ou complètement fluorés, s'agissant préférentiellement d'alkyle ou d'alcényle et notamment d'alkyle en C₁-C₆,
k et q signifiant, indépendamment l'un de l'autre, 0 ou 1,
A1 signifie carbone ou silicium,
A2, A3 et A4 sont, indépendamment l'un de l'autre, un groupe C_{d}J_{2d}, dans lequel J et d ont les significations définies ci-dessus,
j, p et 1 sont, indépendamment l'un de l'autre, 0 ou 1,
A5 signifie un groupe de liaison divalent, s'agissant notamment de -O-, -CO-O- ou de -CO-,
R¹⁸ est l'hydrogène, alkyle, alcényle ou aryle, lesquels sont le cas échéant partiellement ou complètement fluorés, s'agissant préférentiellement d'hydrogène ou de méthyle,
R²⁰, R²¹, R²² et R²³ signifient, indépendamment l'un de l'autre, l'hydrogène, alkyle, alkyloxy, alcényle, alcényloxy, alcynyle, alcynyloxy, aryle, aryloxy, aralkyle, aralkyloxy, alkylaryle et/ou alkylaryloxy, lesquels sont le cas échéant complètement ou partiellement fluorés, s'agissant préférentiellement d'alkyle ou d'alcényle et notamment d'alkyle en C₁-C₆,
BG est un groupe de liaison divalent, et
R¹⁹ et R²⁴ sont, indépendamment l'un de l'autre, l'hydrogène, alkyle, alcényle ou aryle, lesquels sont le cas échéant complètement ou partiellement fluorés, s'agissant préférentiellement d'hydrogène ou de méthyle,
à condition que, parmi les radicaux R², R³ et R⁴ et/ou les radicaux R⁵, R⁶ et R⁷ et/ou les radicaux R⁸, R⁹ et R¹⁰ et/ou les radicaux R¹⁵, R¹⁶ et R¹⁷ et/ou les radicaux R²⁰ et R²¹ et/ou les radicaux R²² et R²³ et/ou les radicaux R²⁰, R²¹ et R²², un seul puisse être de l'hydrogène,
f et h pouvant, au sein d'une même de la molécule, adopter différentes valeurs rentrant dans le cadre de la définition indiquée,
ou bien ledit agent tensioactif non-ionique comportant du silicium et contenant au moins un radical poly(oxyde d'alkylène) est un composé selon les formules VII, VIII, IX ou X 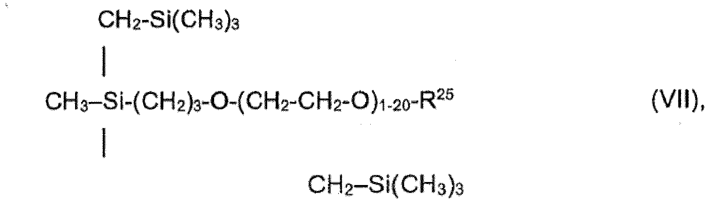 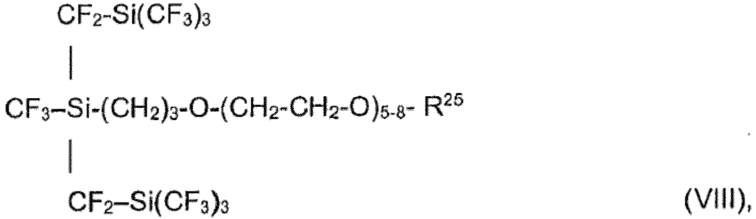 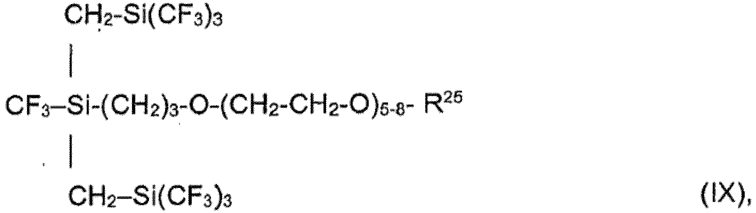 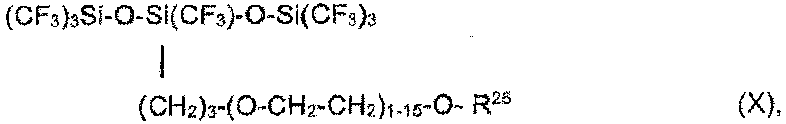 dans lesquelles R²⁵ est l'hydrogène, le méthyle, l'éthyle, le propyle ou le butyle, s'agissant préférentiellement d'hydrogène ou de méthyle.

3. Composition selon au moins une des revendications précédentes, **caractérisée en ce que**
celle-ci contient, outre l'au moins un agent tensioactif fluoré du groupe a), en tant que constituant supplémentaire un polyéther contenant des radicaux d'alcényle et/ou des radicaux d'alcynyle et/ou un polyéther pourvu de unités terminales hydroxyle et/ou aryloxy et/ou arylalkyloxy et/ou alcoxy, le polyéther contenant des radicaux alcényles étant préférentiellement un composé selon la formule XII, et le polyéther pourvu d'unités terminales hydroxyle et/ou alcoxy étant préférentiellement un composé selon la formule XIII
CH₂=CH-CH₂-O-(Cₙ₂H₂ₙ₂-O)ₘ₉-CH₂-CH=CH₂ (XII),
R³¹-O-(Cₙ₂H₂ₙ₂-O)ₘ₉-R³² (XIII),
dans lesquelles n2 est un nombre entier compris entre 2 et 8, de préférence entre 2 et 4,
m9 est un nombre entier compris entre 3 et 70 000, de préférence entre 10 et 2 500,
R³¹ et R³² signifient, indépendamment l'un de l'autre, l'hydrogène ou alkyle en C₁-C₆, notamment l'hydrogène et/ou le méthyle, l'éthyle ou le propyle,
R³¹, R³², n2 et m9 pouvant, au sein d'une même molécule, être différents dans le cadre des définitions indiquées,
et/ou celle-ci contient, outre l'au moins un agent tensioactif fluoré du groupe a), en tant que constituant supplémentaire, un polyol choisi préférentiellement soit dans le groupe des glucides, des alcools polyvinyliques, des di-, tri-, tétra-, penta- et/ou hexanols aliphatiques et des mélanges de deux ou plusieurs desdits polyols, soit dans le groupe des alcools polyvinyliques, des polysaccharides, de triméthylolpropane, de pentaérythritol, de dipentaérythritol, de glycérol, d'allyloxy-1,2-propandiol, de 2-méthyl-2,4-pentandiol, de triméthylolpropanallyléther, de décanediol, de nonanediol, d'octanediol, d'heptanediol, d'hexanediol, de pentanediol, de butanediol, de propanediol, d'éthanediol, de fructose, de glucose et des mélanges deux ou plusieurs desdits polyols, s'agissant notamment de glycérol.

4. Composition selon au moins une des revendications 1 à 3, **caractérisée en ce que**
le rapport de poids entre les agents tensioactifs fluorés du groupe a) et les autres agents tensioactifs fluorés est compris entre 100 : 1 et 1 : 100, de préférence entre 50 : 1 et 1 : 50, avec une préférence particulière entre 10 : 1 et 1 : 10 et, avec une préférence toute particulière, entre 4 : 1 et 1 : 5,
et/ou le rapport de poids entre l'agent tensioactif comportant du silicium et l'agent tensioactif fluoré choisi dans les groupes a) est compris entre 100 : 1 et 1 : 100, de préférence entre 50 : 1 et 1 : 50, avec une préférence particulière entre 10 : 1 et 1 : 10 et, avec une préférence toute particulière, entre 5 : 1 et 1 : 5.

5. Composition selon au moins une des revendications précédentes, **caractérisée en ce que**
celle-ci est un matériau d'empreinte dentaire à plusieurs constituants de type organopolysiloxane subissant une réticulation par addition, lequel contient des constituants A et B, dans lequel
a) le constituant A est un organopolysiloxane ayant au moins deux groupes éthyléniquement insaturés et un catalyseur d'hydrosilylation,
b) le constituant B est un polysiloxane organohydrogéné, et
c) au moins un des constituants A et/ou B contient l'agent tensioactif fluoré choisi dans l'un du groupe a) en combinaison avec un agent tensioactif non-ionique comportant des groupes contenant du silicium,
et/ou celle-ci est un matériau d'empreinte dentaire à plusieurs constituants de type organopolysiloxane subissant une réticulation par réaction de condensation, lequel contient des constituants C et D, dans lequel
i) le constituant C contient un organopolysiloxane ayant au moins deux groupes hydroxyle,
j) le constituant D contient un ester d'acide silicique, un polyester d'acide silicique et/ou un organopolysiloxane ayant au moins deux groupes alcoxy ainsi qu'un catalyseur de condensation, et
k) au moins un des constituants C et/ou D contient l'agent tensioactif fluoré choisi dans l'un du groupe a) en combinaison avec un agent tensioactif non-ionique comportant des groupes contenant du silicium,
et/ou celle-ci est un matériau d'empreinte dentaire à plusieurs constituants de type polyéther qui contenant des radicaux alcényle subissant une réticulation par réaction d'addition, lequel contient des constituants E et F, dans lequel
l) le constituant E contient un catalyseur de réticulation,
m) le constituant F contient un polyéther susceptible d'être réticulé et comportant des radicaux alcényle, ainsi qu'un polysiloxane organohydrogéné et/ou un polyéther de type SiH, et
n) au moins un des constituants E et/ou F contient l'agent tensioactif fluoré choisi dans l'un du groupe a) en combinaison avec un agent tensioactif non-ionique comportant des groupes contenant du silicium,
et/ou celle-ci est un matériau d'empreinte dentaire à plusieurs constituants de type polyéther contenant des radicaux alcoxysilyle, lequel contient les constituants G et H, dans lequel
o) le constituant G contient un polyéther susceptible d'être réticulé et comportant des radicaux alcoxysilyle,
p) le constituant H contient de l'eau, et
q) au moins un des constituants G et/ou H contient un catalyseur ainsi que l'agent tensioactif fluoré choisi dans l'un du groupe a) en combinaison avec un agent tensioactif non-ionique comportant des groupes contenant du silicium,
et/ou celle-ci est un matériau d'empreinte dentaire à plusieurs constituants contenant des constituants I et J, dans lequel
r) le constituant I contient un polyéther susceptible d'être réticulé et comportant des radicaux alcoxysilyle ou des radicaux aziridino ou un polyéther contenant des groupes susceptibles d'être réticulés par réaction de métathèse impliquant une ouverture de cycle,
s) le constituant J contient un catalyseur, et
t) au moins un des constituants I et/ou J contient l'agent tensioactif fluoré choisi dans l'un du groupe a) en combinaison avec un agent tensioactif non-ionique comportant des groupes contenant du silicium,
et/ou celle-ci contient un éther de polyalkylène susceptible d'être réticulé qui comporte des radicaux dérivés d'acide acrylique et/ou d'acide méthacrylique, un initiateur qui peut être activé chimiquement ou par un rayonnement ainsi que l'agent tensioactif fluoré choisi dans l'un du groupe a) en combinaison avec un agent tensioactif non-ionique comportant des groupes contenant du silicium.
et/ou celle-ci est matériau d'empreinte dentaire à plusieurs constituants de type polyéther lequel contient des polyéthers, des polysiloxanes et/ou des caoutchoucs de synthèse comportant des groupes susceptibles d'être réticulés par une polymérisation de type métathèse impliquant une ouverture de cycle (ROMP), et lequel contient les constituants K et L, dans lequel
u) le constituant K contient des polyéthers, des polysiloxanes et/ou des caoutchoucs de synthèse comportant des groupes susceptibles d'être réticulés par ROMP,
v) le constituant L contient un catalyseur de réticulation ROMP, et
w) au moins un des constituants K et/ou L contient l'agent tensioactif fluoré choisi dans le groupe a) en combinaison avec un agent tensioactif non-ionique comportant des groupes contenant du silicium.

6. Composition selon au moins une des revendications précédentes, **caractérisée en ce qu'**elle contient des charges, leur proportion globale étant préférentiellement comprise 0,01 et 80 % en poids, avec une préférence particulière entre 0,05 et 75 % en poids, et avec une préférence toute particulière entre 0,1 et 70 % en poids, par rapport à la composition dans sa totalité, et/ou qu'elle contient un ou plusieurs des additifs suivants : sels à effet tampon, agents d'absorption d'humidité, agents formateurs de pâtes, d'autres agents tensioactifs, principes actifs, plastifiants, substances permettant une lecture optique, arômes et/ou parfums, substances permettant de réaliser des diagnostics, agents de fluoration, agents blanchissants, agents de désensibilisation, agents de pontage promoteurs d'adhésion, colorants, indicateurs, agents stabilisants (antioxydants) ainsi que des substances antibactériennes.

7. Composition selon la revendication 1, **caractérisée en ce qu'**elle contient
a) 10 à 85 % en poids d'organopolydialkylsiloxane pourvu d'au moins deux groupes alcényle,
b) 1 à 70 % en poids d'un polysiloxane organohydrogéné ayant une teneur en SiH comprise entre 0,1 et 15,0 mmol/kg,
c) 0,0001 à 2 % en poids de catalyseur d'hydrosilylation, s'agissant notamment de sels, de complexes et de formes colloïdales de métaux de transition des groupes 8 à 10,
d) 0 à 90 % en poids de charges non-renforçantes ayant une surface BET inférieure à 50 m²/g,
e) 0,1 à 50 % en poids de charges renforçantes ayant une surface BET supérieure ou égale à 50 m²/g,
f) 0 à 20 % en poids d'agents auxiliaires et d'additifs tels que les plastifiants, les colorants, les agents stabilisants, les inhibiteurs, des éthoxylates d'alcools gras fermés par des unités d'alkyle,
g) 0,01 à 10,0 % en poids d'un agent tensioactif non-ionique qui comporte au moins un radical de poly(oxyde d'alkylène) ainsi qu'un groupe qui contient du silicium et qui a une masse moléculaire inférieure à 6 000 g/mol, s'agissant d'un agent tensioactif non-ionique de type polyéthersiloxane ayant 3 à 7 atomes de silicium dans la structure partielle de siloxane et/ou d'un agent tensioactif non-ionique de type polyéthercarbosilane ayant 1 à 7 atomes de silicium dans la structure partielle de carbosilane et une proportion d'oxyde d'alkylène comprise entre 1 et 20 unités, et
h) 0,001 à 10 % en poids de l'agent tensioactif fluoré choisi dans l'un du groupe a) selon la revendication 1,
et celle-ci contenant, en outre, optionnellement i) 0,1 à 25 % en poids d'un éther de polyalkylène ramifié ou linéaire, pourvu d'unités terminales d'alkyle, d'hydroxyle, d'alcynyle et/ou alcényle et/ou leurs mélanges, le rapport de poids entre l'organopolysiloxane a) et le polyéther i) étant de préférence compris entre 1 : 1 et 80 : 1, avec une préférence particulière entre 1 : 1 et 60 : 1, avec une préférence toute particulière entre 1 : 1 et 40 : 1 et, de manière particulièrement préférée, entre 1 : 1 et 30 : 1.

8. Composition selon la revendication 7, **caractérisée en ce qu'**il s'agit d'un matériau d'empreinte dentaire à deux constituants, constitué des constituants A et B,
le constituant A contenant
a) 10 à 80 % en poids d'organopolydialkylsiloxane pourvu d'au moins deux groupes alcényle,
c) 0,0001 à 2 % en poids de catalyseur d'hydrosilylation, s'agissant notamment de sels, de complexes et de formes colloïdales de métaux de transition des groupes 8 à 10,
d) 0 à 90 % en poids de charges non-renforçantes ayant une surface BET inférieure à 50 m²/kg,
e) 0,1 à 50 % en poids de charges renforçantes ayant une surface BET supérieure ou égale à 50 m²/kg,
f) 0 à 20 % en poids d'agents auxiliaires et d'additifs tels que les plastifiants, les colorants, les agents stabilisants et/ou les inhibiteurs, et
j) 0,001 à 5,0 % en poids d'agents tensioactifs non-ioniques fermés par des unités d'alkyle, d'aryle, d'aralkyle, s'agissant préférentiellement d'éthoxylates d'alcools gras fermés par une unité d'alkyle, d'agents tensioactifs de type silicone, de polyéthercarbosilanes, d'agents tensioactifs de type carbosilane et d'agents tensioactifs fluorés qui sont fermés par des unités d'alkyle, et notamment d'éthoxylates d'alcools gras fermés par des unités d'alkyle, et
et le constituant B contenant
a) 0,1 à 70 % en poids d'organopolydialkylsiloxane pourvu d'au moins deux groupes alcényle,
b) 1,2 à 80 % en poids d'un polysiloxane organohydrogéné ayant une teneur en SiH comprise entre 0,1 et 15,0 mmol/kg,
d) 0 à 90 % en poids de charges non-renforçantes ayant une surface BET inférieure à 50 m²/kg,
e) 0,1 à 50 % en poids de charges renforçantes ayant une surface BET supérieure ou égale à 50 m²/kg,
f) 0 à 20 % en poids d'agents auxiliaires et d'additifs tels que les plastifiants, les colorants, les agents stabilisants et/ou les inhibiteurs,
g) 0,01 à 10,0 % en poids d'un agent tensioactif non-ionique qui comporte au moins un radical de poly(oxyde d'alkylène) ainsi qu'un groupe contenant du silicium et qui a une masse moléculaire inférieure à 6 000 g/mol, s'agissant d'un agent tensioactif non-ionique de type polyéthersiloxane ayant 3 à 7 atomes de silicium dans la structure partielle de siloxane et/ou d'un agent tensioactif non-ionique de type polyéthercarbosilane ayant 1 à 7 atomes de silicium dans la structure partielle de carbosilane et une proportion d'oxyde d'alkylène comprise entre 1 et 20 unités,
h) 0,001 à 10 % en poids de l'au moins un agent tensioactif fluoré choisi dans l'un du groupe a) selon la revendication 1, le rapport entre les agents tensioactifs g) et h) étant compris entre 25 : 1 et 1 : 25, de préférence entre 20 : 1 et 1 : 5, avec une préférence particulière entre 10 : 1 et 1 : 5 et, avec une préférence toute particulière, entre 5 : 1 et 1 : 3, et
i) 0,5 à 50 % en poids d'un éther de polyalkylène ramifié ou linéaire, pourvu d'unités terminales d'alkyle, d'hydroxyle, d'alcynyle et/ou alcényle, et/ou de leurs mélanges, le rapport de poids entre l'organopolysiloxane a) et le polyéther i) étant de préférence compris entre 1 : 50 et 50 : 1, avec une préférence particulière entre 10 : 1 et 1 : 10 et, avec une préférence toute particulière, entre 4 : 1 et 1 : 5.

9. Composition selon la revendication 8, **caractérisée en ce qu'**il s'agit d'un matériau d'empreinte dentaire à deux constituants, constitué des constituants A et B,
le constituant A contenant
b) 10 à 80 % en poids d'organopolydialkylsiloxane pourvu d'au moins deux groupes alcényle,
c) 0,01 à 2 % en poids de catalyseur d'hydrosilylation, s'agissant notamment de sels, de complexes et de formes colloïdales de métaux de transition des groupes 8 à 10,
d) 0 à 90 % en poids de charges non-renforçantes ayant une surface BET inférieure à 50 m²/kg,
e) 0,1 à 50 % en poids de charges renforçantes ayant une surface BET supérieure ou égale à 50 m²/kg,
f) 0 à 20 % en poids d'agents auxiliaires et d'additifs tels que les plastifiants, les colorants, les agents stabilisants et/ou les inhibiteurs, et
j) 0,001 à 5,0 % en poids d'agents tensioactifs non-ioniques fermés par des unités d'alkyle, d'aryle, d'aralkyle, s'agissant préférentiellement d'éthoxylates d'alcools gras fermés par une unité d'alkyle, d'agents tensioactifs de type silicone, de polyéthercarbosilanes, d'agents tensioactifs de type carbosilane et d'agents tensioactifs fluorés qui sont fermés par des unités d'alkyle, et notamment d'éthoxylates d'alcools gras fermés par des unités d'alkyle,
et le constituant B contenant
c) 0,1 à 70 % en poids d'organopolydialkylsiloxane pourvu d'au moins deux groupes alcényle,
d) 1,2 à 80 % en poids d'un polysiloxane organohydrogéné ayant une teneur en SiH comprise entre 0,1 et 15,0 mmol/kg,
e) 0 à 90 % en poids de charges non-renforçantes ayant une surface BET inférieure à 50 m²/kg,
f) 0,1 à 50 % en poids de charges renforçantes ayant une surface BET supérieure ou égale à 50 m²/kg,
g) 0 à 20 % en poids d'agents auxiliaires et d'additifs tels que les plastifiants, les colorants, les agents stabilisants et/ou les inhibiteurs,
h) 0,01 à 10,0 % en poids d'agent tensioactif non-ionique de type polyéthersiloxane ayant 3 à 7 atomes de silicium dans la structure partielle de siloxane et/ou d'agent tensioactif non-ionique de type polyéthercarbosilane ayant 1 à 7 atomes de silicium dans la structure partielle de carbosilane et une proportion d'oxyde d'alkylène comprise entre 1 et 20 unités,
j) 0,001 à 10,0 % en poids de l'agent tensioactif fluoré choisi dans l'un du groupe a) selon la revendication 1, et
k) 0,5 à 50 % en poids de polyol et/ou de mélanges de polyols, le rapport de poids entre l'organopolysiloxane a) et le polyol k) étant de préférence compris entre 1 : 50 et 50 : 1, avec une préférence particulière entre 10 : 1 et 1 : 10 et, avec une préférence toute particulière, entre 4 : 1 et 1 : 5.

10. Composition selon au moins une des revendications précédentes, **caractérisée en ce que**
40 secondes après le début du processus de mélange, celle-ci présente un angle de contact avec une goutte d'eau dont la valeur initiale basse est < 10°, la mesure étant effectué selon la description lorsque la pose de ladite goutte remonte à 10 secondes,
ledit angle de contact avec une goutte d'eau correspondant, 40 secondes après le début du processus de mélange, notamment aux valeurs suivantes : 0,25 secondes après la pose d'une goutte d'eau à un angle de contact avec ladite goutte d'eau de < 75°, préférentiellement < 40° ; 0,5 secondes après la pose d'une goutte d'eau à un angle de contact avec ladite goutte d'eau de < 55°, préférentiellement de < 30° ; 1 seconde après la pose d'une goutte d'eau à un angle de contact avec ladite goutte d'eau de < 35°, préférentiellement de < 25° ; 2 secondes après la pose d'une goutte d'eau : à un angle de contact avec ladite goutte d'eau de < 20° ; et 3 secondes après la pose d'une goutte d'eau à un angle de contact avec ladite goutte d'eau de < 10°.

11. Mélanges pouvant être obtenus en mélangeant les compositions selon au moins une des revendications précédentes.

12. Matériau d'empreinte durci, pouvant être obtenu en soumettant la composition selon au moins une des revendications 1 à 10 à un processus de durcissement.

13. Utilisation d'un agent tensioactif fluoré choisi dans le groupe a) en association avec au moins un agent tensioactif non-ionique qui comporte du silicium et qui comporte au moins un radical de poly(oxyde d'alkylène) et dont la masse moléculaire est inférieure à 6 000 g/mol selon la revendication 1, éventuellement en combinaison avec au moins un autre agent tensioactif fluoré, pour préparer des matériaux dentaires, notamment des matériaux d'empreinte dentaires.

14. Utilisation d'une composition selon l'une des revendications 1 à 10, pour préparer des matériaux dentaires, notamment des matériaux d'empreinte dentaires.
